(19) Europäisches Patentamt
European Patent Office
Office européen des brevets

(11) Veröffentlichungsnummer: **0 094 095**
**B1**

(12) # EUROPÄISCHE PATENTSCHRIFT

(45) Veröffentlichungstag der Patentschrift: **19.09.90**

(21) Anmeldenummer: **83104668.5**

(22) Anmeldetag: **11.05.83**

(51) Int. Cl.$^5$: **C 07 D 487/04**, C 07 F 9/547, A 61 K 31/55, A 61 K 31/50, A 61 K 31/415, A 61 K 31/675

(54) **Bicyclische Carbonsäuren und deren Alkyl- und Aralkylester.**

(30) Priorität: **12.05.82 GB 8213850**
**28.02.83 GB 8305505**

(43) Veröffentlichungstag der Anmeldung:
**16.11.83 Patentblatt 83/46**

(45) Bekanntmachung des Hinweises auf die Patenterteilung:
**19.09.90 Patentblatt 90/38**

(84) Benannte Vertragsstaaten:
**AT BE CH DE FR GB IT LI LU NL SE**

(56) Entgegenhaltungen:
**EP-A-0 025 941**
**EP-A-0 042 100**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE AG**
**Postfach 3255**
**CH-4002 Basel (CH)**

(72) Erfinder: **Attwood, Michael Richard**
**1A Benslow Lane**
**Hitchin Herts (GB)**
Erfinder: **Hassall, Cedric Herbert**
**6 Ashcroft Close**
**Harpenden Herts (GB)**
Erfinder: **Lambert, Robert Wilson**
**33 New Road**
**Digswell Welwyn Herts (GB)**
Erfinder: **Lawton, Geoffrey**
**109 Whitehill Road**
**Hitchin Herts (GB)**
Erfinder: **Redshaw, Sally**
**37 Bowmans Avenue**
**Hitchin Herts (GB)**

(74) Vertreter: **Lederer, Franz, Dr. et al**
**Van der Werth, Lederer & Riederer**
**Patentanwälte Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 094 095 B1

**Beschreibung**

Die vorliegende Erfindung betrifft bicyclische Verbindungen, ein Verfahren zu deren Herstellung, Arzneimittel enthaltend diese Verbindungen sowie die Verwendung dieser Verbindungen.

Im speziellen betrifft die Erfindung bicyclische Verbindungen der allgemeinen Formel

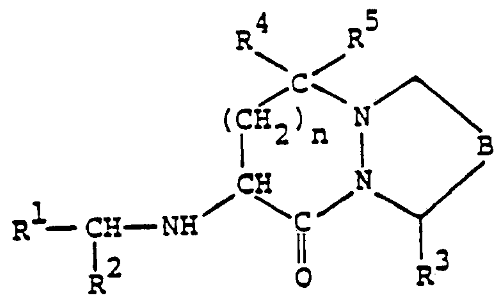

I

worin B Methylen ($-CH_2-$), Aethylen ($-CH_2-CH_2-$) oder Vinylen ($-CH=CH-$), $R^1$ Wasserstoff, Alkyl, Aralkyl, Aminoalkyl, Monoalkylaminoalkyl, Dialkylaminoalkyl, Aralkylcarbonylaminoalkyl, Phthalimidoalkyl, Alkoxycarbonylaminoalkyl, Aryloxycarbonylaminoalkyl, Aralkoxycarbonylaminoalkyl, Alkylaminocarbonylaminoalkyl, Arylaminocarbonylaminoalkyl, Aralkylaminocarbonylaminoalkyl, Alkylsulfonylaminoalkyl oder Arylsulfonylaminoalkyl, $R^2$ Carboxyl, Alkoxycarbonyl oder Aralkoxycarbonyl oder eine Gruppe der Formel

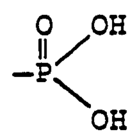

(i)

oder

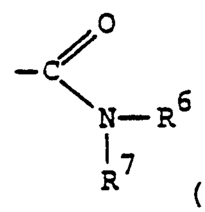

(ii)

$R^3$ Carboxyl, Alkoxycarbonyl oder Aralkoxycarbonyl, $R^4$ und $R^5$ je Wasserstoff oder zusammen eine Oxogruppe, $R^6$ und $R^7$ je Wasserstoff, Alkyl oder Aralkyl und n die Zahl 2 bedeuten, und pharmazeutisch verwendbare Salze davon.

In EP—A—25941 und EP—A—42100 sind Pyrazolopyridazine und Pyridazopyridazine beschrieben, welche ebenfalls eine das Angiotensin umwandelnde Enzym hemmende Wirkung entfalten. Die anmeldegemäßen Pyridazodiazepine unterscheiden sich durch die Anwesenheit einer an den Bicyclus gebundenen Aminogruppe sowie das Vorhandensein eines Siebenerrings anstelle eines Fünfer- bzw. Sechserrings in signifikanter Weise von den vorbekannten Verbindungen.

Die Verbindungen der Formel I enthalten asymmetrische Kohlenstoffatome und können demnach als optisch reine Diastereomere, als diastereomere Racemate oder als diastereomere Mischungen vorliegen. Die vorliegende Erfindung umfasst alle diese Formen. Die erfindungsgemässen Verbindungen weisen vorzugsweise an jedem asymmetrischen Kohlenstoffatom die (S)-Konfiguration auf.

Der in der vorliegenden Beschreibung verwendete Ausdruck "Alkyl" — allein oder in Kombination — bedeutet geradkettige oder verzweigte Alkylreste mit 1—8, vorzugsweise 1—4, Kohlenstoffatomen, wie Methyl, Aethyl, Propyl, Isopropyl, Butyl, tert.-Butyl, Pentyl und Hexyl. Der Arylrest in Aralkyl, Aralkoxycarbonyl, Aryloxycarbonylaminoalkyl, Aralkoxycarbonylaminoalkyl, Arylaminocarbonylaminoalkyl, Aralkylaminocarbonylaminoalkyl oder Arylsulfonylaminoalkyl ist die Phenylgruppe, welche durch Halogen (d.h. Fluor, Chlor, Brom oder Jod), Alkyl, Alkoxy, Trifluormethyl oder Phenyl ein oder mehrfach substituiert sein kann. Beispiele von Arylgruppen sind Phenyl, 4-Chlorphenyl, p-Tolyl, Biphenylyl und dergleichen und Beispiele von Aralkylgruppen sind Benzyl, 4-Chlorbenzyl, 2-Phenyläthyl, 3-Phenylpropyl, 3-(4-Chlorphenyl)propyl, 3-(4-Methoxyphenyl)propyl, 4-Phenylbutyl und dergleichen. Aminomethyl, 2-Aminoäthyl und dergleichen sind Beispiele für Aminoalkylgruppen. Als Beispiele für Monoalkylaminoalkylgruppen können Methylaminomethyl, 2-Methylaminoäthyl, 2-Aethylaminoäthyl und dergleichen und als Beispiele für Dialkylaminoalkylgruppen, 2-Dimethylaminoäthyl, 2-Diäthylaminoäthyl, 3-Dimethylaminopropyl und dergleichen genannt werden. Eine Alkoxygruppe und der Alkoxyrest in einer Alkoxycarbonylgruppe ist geradkettig oder verzweigt und enthält 1—8, vorzugsweise 1—4 Kohlenstoffatome. Spezifische Beispiele für Alkoxycarbonylgruppen sind Methoxycarbonyl und Aethoxycarbonyl.

Eine interessante Untergruppe von Verbindungen der Formel I sind solche, worin $R^1$ Wasserstoff, Alkyl, Aralkyl, Aminoalkyl, Monoalkylaminoalkyl, Dialkylaminoalkyl, Aralkylcarbonylaminoalkyl, Alkoxycarbonylaminoalkyl, Aryloxycarbonylaminoalkyl, Aralkoxycarbonylaminoalkyl, Alkylaminocarbonylaminoalkyl, Arylaminocarbonylaminoalkyl, Aralkylaminocarbonylaminoalkyl, Alkylsulfonylaminoalkyl

oder Arylsulfonylaminoalkyl, $R^2$ Carboxyl oder Alkoxycarbonyl oder eine Gruppe der Formel (i) und $R^3$ Carboxyl oder Alkoxycarbonyl bedeuten.

Eine bevorzugte Klasse von erfindungsgemässen Verbindungen sind solche, worin B Methylen oder Aethylen bedeutet. $R^1$ bedeutet vorzugsweise Alkyl, Aralkyl, Aralkylcarbonylaminoalkyl, Phthalimidoalkyl, Aralkoxycarbonylaminoalkyl oder Aralkylaminocarbonylaminoalkyl. $R^2$ bedeutet vorzugsweise Carboxyl, Alkoxycarbonyl oder Aralkoxycarbonyl oder eine Gruppe der Formel (ii). Vorzugsweise bedeutet $R^3$ eine Carboxylgruppe.

Aus dem obigen folgt, dass Verbindungen der Formel I, worin B Methylen oder Aethylen, $R^1$ Alkyl, Aralkyl, Aralkylcarbonylaminoalkyl, Phthalimidoalkyl, Aralkoxycarbonylaminoalkyl oder Aralkylaminocarbonylaminoalkyl, $R^2$ Carboxyl, Alkoxycarbonyl oder Aralkoxycarbonylamino oder eine Gruppe der Formel (ii) und $R^3$ Carboxyl bedeuten, besonders bevorzugt sind.

Ganz besonders bevorzugte Verbindungen der allgemeinen Formel I sind:

9-(1-Carboxy-3-phenylpropylamino)-octahydro-6,10-dioxo-6H-pyridazo[1,2-a][1,2]diazepin-1-carbonsäure,

9-(1-Aethoxycarbonyl-3-phenylpropylamino)-octahydro-6,10-dioxo-6H-pyridazo[1,2-a][1,2]diazepin-1-carbonsäure,

8-(1-Carboxy-3-phenylpropylamino)-2,3,6,7,8,9-hexahydro-5,9-dioxo-1H,5H-pyrazolo[1,2-a][1,2]diazepin-1-carbonsäure,

9-(1-Benzyloxycarbonyl-3-phenylpropylamino)-octahydro-6,10-dioxo-6H-pyridazo[1,2-a][1,2]diazepin-1-carbonsäure,

9-(1-Carbamoyl-3-phenylpropylamino)-octahydro-6,10-dioxo-6H-pyridazo[1,2-a][1,2]diazepin-1-carbonsäure,

9-(1-Aethylcarbamoyl-3-phenylpropylamino)octahydro-6,10-dioxo-6H-pyridazo[1,2-a][1,2]diazepin-1-carbonsäure,

9-(1-Carboxy-4-phenylbutylamino)-octahydro-6,10-dioxo-6H-pyridazo[1,2-a][1,2]diazepin-1-carbonsäure,

9-(1-Carboxy-2-phenyläthylamino)-octahydro-6,10-dioxo-6H-pyridazo[1,2-a][1,2]diazepin-1-carbonsäure,

9-(1-Carboxy-4-methylpentylamino)-octahydro-6,10-dioxo-6H-pyridazo[1,2-a][1,2]diazepin-1-carbonsäure,

9-(1-Aethoxycarbonyl-4-methylpentylamino)-octahydro-6,10-dioxo-6H-pyridazo[1,2-a][1,2]diazepin-1-carbonsäure,

9-[3-(4-Chlorphenyl)-1-äthoxycarbonylpropylamino]-octahydro-6,10-dioxo-6H-pyridazo[1,2-a][1,2]diazepin-1-carbonsäure,

9-[1-Aethoxycarbonyl-3-(4-methoxyphenyl)propylamino]-octahydro-6,10-dioxo-6H-pyridazo[1,2-a][1,2]diazepin-1-carbonsäure,

9-[3-(4-Biphenylyl)-1-äthoxycarbonylpropylamino]-octahydro-6,10-dioxo-6H-pyridazo[1,2-a][1,2]diazepin-1-carbonsäure,

9-(1-Aethoxycarbonyl-5-phthalimidopentylamino)-octahydro-6,10-dioxo-6H-pyridazo[1,2-a][1,2]diazepin-1-carbonsäure,

8-(1-Aethoxycarbonyl-3-phenylpropylamino)-2,3,6,7,8,9-hexahydro-5,9-dioxo-1H,5H-pyrazolo[1,2-a][1,2]diazepin-1-carbonsäure,

9-(1-Aethoxycarbonyl-3-phenylpropylamino)-octahydro-10-oxo-6H-pyridazo[1,2-a][1,2]diazepin-1-carbonsäure und

9-(5-Benzyloxyformamido-1-äthoxycarbonylpentylamino)-octahydro-6,10-dioxo-6H-pyridazo[1,2-a][1,2]diazepin-1-carbonsäure.

Weitere Beispiele interessanter Verbindungen der Formel I sind:

tert.Butyl 9-(1-äthoxycarbonyl-3-phenylpropylamino)-octahydro-6,10-dioxo-6H-pyridazo[1,2-a][1,2]diazepin-1-carboxylat,

tert.Butyl 9-(1-benzyloxycarbonyl-3-phenylpropylamino)-octahydro-6,10-dioxo-6H-pyridazo[1,2-a][1,2]diazepin-1-carboxylat,

Methyl 9-(1-benzyloxycarbonyl-3-phenylpropylamino)-octahydro-6,10-dioxo-6H-pyridazo[1,2-a][1,2]diazepin-1-carboxylat,

Methyl 9-(1-carboxy-3-phenylpropylamino)-octahydro-6,10-dioxo-6H-pyridazo[1,2-a][1,2]diazepin-1-carboxylat,

Methyl 9-(1-äthoxycarbonyl-3-phenylpropylamino)-octahydro-6,10-dioxo-6H-pyridazo[1,2-a][1,2]diazepin-1-carboxylat,

Aethyl 9-(1-äthoxycarbonyl-3-phenylpropylamino)-octahydro-6,10-dioxo-6H-pyridazo[1,2-a][1,2]diazepin-1-carboxylat,

tert.Butyl 9-(1-carbamoyl-3-phenylpropylamino)-octahydro-6,10-dioxo-6H-pyridazo[1,2-a][1,2]diazepin-1-carboxylat,

tert.Butyl 9-(1-äthylcarbamoyl-3-phenylpropylamino)-octahydro-6,10-dioxo-6H-pyridazo[1,2-a][1,2]diazepin-1-carboxylat,

tert.Butyl 9-(1-äthoxycarbonyl-4-methylpentylamino)-octahydro-6,10-dioxo-6H-pyridazo[1,2-a][1,2]diazepin-1-carboxylat,

tert.Butyl 9-[3-(4-chlorphenyl)-1-äthoxycarbonylpropylamino]-octahydro-6,10-dioxo-6H-pyridazo[1,2-a][1,2]diazepin-1-carboxylat,

tert.Butyl 9-[1-äthoxycarbonyl-3-(4-methoxyphenyl)propylamino]-octahydro-6,10-dioxo-6H-pyridazo[1,2-a][1,2]diazepin-1-carboxylat,

tert.Butyl 9-[3-(4-biphenylyl)-1-äthoxycarbonylpropylamino]-octahydro-6,10-dioxo-6H-pyridazo[1,2-a][1,2]diazepin-1-carboxylat,

tert.Butyl 9-(1-äthoxycarbonyl-5-phthalimidopentylamino)-octahydro-6,10-dioxo-6H-pyridazo[1,2-a][1,2]diazepin-1-carboxylat,

tert.Butyl 8-(1-äthoxycarbonyl-3-phenylpropylamino)-2,3,6,7,8,9-hexahydro-5,9-dioxo-1H,5H-pyrazolo[1,2-a][1,2]diazepin-1-carboxylat,

Benzyl 9-(1-äthoxycarbonyl-3-phenylpropylamino)-octahydro-10-oxo-6H-pyridazo[1,2-a][1,2]diazepin-1-carboxylat und

tert.Butyl 9-(5-benzyloxyformamido-1-äthoxycarbonylpentylamino)-octahydro-6,10-dioxo-6H-pyridazo[1,2-a][1,2]diazepin-1-carboxylat.

Die Verbindungen der Formel I und deren pharmazeutisch verwendbare Salze können erfindungsgemäss dadurch hergestellt werden, dass man

a) eine Verbindung der allgemeinen Formel

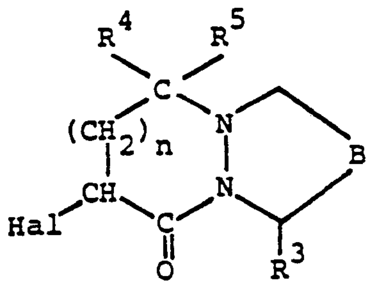

II

worin B, $R^3$, $R^4$, $R^5$ und n die obige Bedeutung besitzen und Hal Halogen bedeutet, mit einer Verbindung der allgemeinen Formel

$$\begin{matrix} R^1\text{—}CH\text{—}NH_2 \\ | \\ R^2 \end{matrix} \qquad \text{III}$$

worin $R^1$ und $R^2$ die obige Bedeutung besitzen, umsetzt, oder

b) eine Verbindung der allgemeinen Formel

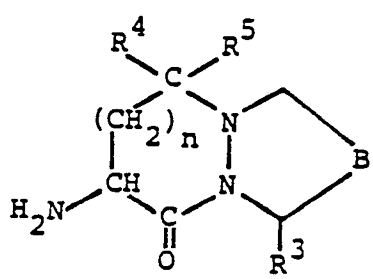

IV

worin B, $R^3$, $R^4$, $R^5$ und n die obige Bedeutung besitzen, mit einer Verbindung der allgemeinen Formel

$$\begin{matrix} R^1\text{—}C{=}O \\ | \\ R^2 \end{matrix} \qquad \text{V}$$

worin $R^1$ und $R^2$ die obige Bedeutung besitzen, reduktive alkyliert, oder

c) zur Herstellung von Verbindungen der Formel I, worin $R^2$ Alkoxycarbonyl oder Aralkoxycarbonyl oder eine Gruppe der Formel (ii) und $R^3$ Alkoxycarbonyl oder Aralkoxycarbonyl bedeuten, eine Verbindung der obigen Formel IV, worin $R^3$ Alkoxycarbonyl oder Aralkoxycarbonyl bedeutet, mit einer Verbindung der allgemeinen Formel

$$\begin{matrix} R^1\text{—}CH\text{—}Q \\ | \\ R^{20} \end{matrix} \qquad \text{VI}$$

worin $R^1$ die obige Bedeutung besitzt, $R^{20}$ Alkoxycarbonyl oder Aralkoxycarbonyl oder eine Gruppe der Formel (ii) und Q eine Abgangsgruppe bedeutet,
umsetzt, oder

d) zur Herstellung von Verbindungen der Formel I, worin $R^2$ eine Gruppe der Formel (ii) und $R^3$ Carboxyl oder tert. Butoxycarbonyl bedeuten, eine Verbindung der Formel I, worin $R^2$ Alkoxycarbonyl und $R^3$ Carboxyl oder tert. Butoxycarbonyl bedeuten, mit einer Verbindung der allgemeinen Formel

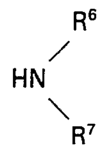

VII

worin $R^6$ und $R^7$ die obige Bedeutung besitzen,
umsetzt, oder

e) zur Herstellung von Verbindungen der Formel I, worin B Aethylen bedeutet, eine Verbindung der Formel I, worin B Vinylen bedeutet, katalytisch hydriert, oder

f) zur Herstellung von Verbindungen der Formel I, worin $R^2$ und/oder $R^3$ Alkoxycarbonyl oder Aralkoxycarbonyl bedeuten, eine Verbindung der Formel I, worin $R^2$ und/oder $R^3$ Carboxyl bedeuten, verestert, oder

g) zur Herstellung von Verbindungen der Formel I, worin $R^2$ und/oder $R^3$ Carboxyl bedeuten, eine Verbindung der Formel I, worin $R^2$ und/oder $R^3$ Alkoxycarbonyl bedeuten, mit einer Säure oder einer Base behandelt, oder

h) zur Herstellung von Verbindungen der Formel I, worin B Methylen oder Aethylen und $R^2$ und/oder $R^3$ Carboxyl bedeuten, eine Verbindung der Formel I, worin $R^2$ und/oder $R^3$ Aralkoxycarbonyl bedeuten, hydriert, oder

i) zur Herstellung von Verbindungen der Formel I, worin $R^1$ Aminoalkyl bedeutet, in einer Verbindung der Formel I, worin $R^1$ Alkoxycarbonylaminoalkyl, Aryloxycarbonylaminoalkyl oder Aralkoxycarbonylaminoalkyl bedeutet, die Alkoxycarbonyl-, Aryloxycarbonyl- oder Aralkoxycarbonylgruppe abspaltet und

j) erwünschtenfalls ein erhaltenes Gemisch von Diastereomeren in die entsprechenden diastereomeren Racemate oder optisch reinen Diastereomeren auftrennt und/oder

k) erwünschtenfalls ein erhaltenes Racemat in die beiden Antipoden auftrennt und

l) erwünschtenfalls eine erhaltene Verbindung der Formel I in ein pharmazeutisch verwendbares Salz überführt.

Die Umsetzung einer Verbindung der Formel II mit einer Verbindung der Formel III gemäss Verfahrensvariante a) erfolgt in an sich bekannter Weise. Zweckmässigerweise wird die Reaktion in einem inerten organischen Lösungsmittel in Gegenwart eines säurebindenden Mittels durchgeführt. Geeignete inerte organische Lösungsmittel sind aromatische Kohlenwasserstoffe, wie Benzol oder Toluol, Dimethylformamid und dergleichen. Das säurebindende Mittel ist vorzugsweise eine anorganische Base, besonders bevorzugt eine tertiäre organische Base, wie Triäthylamin und dergleichen. Wird ein Ueberschuss an einer Verbindung der Formel III eingesetzt, so dient diese gleichzeitig als Säurebindemittel. Die Reaktion wird zweckmässigerweise bei erhöhter Temperatur durchgeführt, beispielsweise bei einer Temperatur zwischen etwa 60° und der Rückflusstemperatur des Reaktionsgemisches.

Die reduktive Alkylierung einer Verbindung der Formel IV mit einer Verbindung der Formel V gemäss Verfahrensvariante b) erfolgt ebenfalls in an sich bekannter Weise. Gemäss einer Methode erfolgt die Umsetzung in Gegenwart von Natriumcyanborhydrid in einem geeigneten Lösungsmittel, wie Wasser, einer Mischung von Wasser und einem Alkanol, z.B. wässrigem Aethanol, und dergleichen. Zweckmässigerweise wird die Reaktion unter neutralen Reaktionsbedingungen und bei ungefähr Raumtemperatur durchgeführt. Gemäss einer anderen Methode wird die Verbindung der Formel IV mit der Verbindung der Formel V umgesetzt und die erhaltene Schiffbase in situ katalytisch hydriert. Bei dieser katalytischen Hydrierung wird eine Vinylengruppe B gleichzeitig zur Aethylengruppe reduziert. Die katalytische Hydrierung erfolgt unter den üblichen Reaktionsbedingungen, beispielsweise unter Verwendung eines Edelmetallkatalysators, wie Palladium auf Kohle oder Raney-Nickel, in einem inerten, organischen Lösungsmittel, wie einem Alkanol, z.B. Aethanol, bei Raumtemperatur und einem Druck von 1—10 Atmosphären.

Die Umsetzung einer Verbindung der Formel IV, worin $R^3$ Alkoxycarbonyl oder Aralkoxycarbonyl bedeutet, mit einer Verbindung der Formel VI gemäss Verfahrensvariante c) erfolgt zweckmässigerweise in Gegenwart eines inerten organischen Lösungsmittels wie Dimethylformamid, Dimethylsulfoxid, Acetonitril und dergleichen bei einer Temperatur zwischen etwa 0° und 100°C und in Gegenwart eines Säurebindemittels, wie einem Alkalimetallcarbonat, z.B. Natriumcarbonat, einer tert. organischen Base, z.B. Triäthylamin, oder einem basischen Ionenaustauscherharz. Die mit Q bezeichnete Abgangsgruppe in einer Verbindung der Formel VI kann beispielsweise ein Halogenatom, wie ein Bromatom, oder eine Sulfonatgruppe der Formel $—O—SO_2Y$ sein, worin Y Methyl, Trifluormethyl, p-Tolyl und dergleichen bedeutet.

Die Reaktion einer Verbindung der Formel I, worin $R^2$ Alkoxycarbonyl und $R^3$ Carboxyl oder tert.

Butoxycarbonyl bedeutet, mit einer Verbindung der Formel VII gemäss Verfahrensvariante d) erfolgt in an sich bekannter Weise, beispielsweise in Wasser oder einem alkoholischen Medium bei einer niedrigen Temperatur, z.B. bei ungefähr 0°C oder in einer verschlossenen Röhre bei erhöhter Temperatur.

Die katalytische Hydrierung einer Verbindung der Formel I, worin B Vinylen bedeutet, gemäss Verfahrensvariante e) erfolgt ebenfalls nach bekannten Methoden. Geeignete Katalysatoren, welche verwendet werden können, sind Edelmetallkatalysatoren, wie Palladium, Platin, Rhutenium oder Rhodium, und Raney-Nickel. Die Edelmetallkatalysatoren können auf einem geeigneten Trägermaterial aufgebracht sein, wie Palladium auf Kohle, Rhodium auf Alluminiumoxid und dergleichen. Die katalytische Hydrierung erfolgt in einem konventionellen inerten organischen Lösungsmittel, wie einem aromatischen Kohlenwasserstoff, z.B. Benzol, Toluol, Xylol etc., einem Alkanol, z.B. Methanol, Aethanol etc. oder einem Aether z.B. Dioxan etc. Die katalytische Hydrierung erfolgt vorzugsweise bei Raumtemperatur und Atmosphärendruck obwohl sie auch bei erhöhter Temperatur und/oder Druck durchgeführt werden kann.

Die Veresterung einer Verbindung der Formel I, worin $R^2$ und/oder $R^3$ Carboxyl bedeuten, gemäss Verfahrensvariante f) erfolgt ebenfalls in an sich bekannter Weise. Beispielsweise wird eine Verbindung der Formel I, worin $R^2$ und/oder $R^3$ Carboxyl bedeuten, mit einem geeigneten Alkanol, z.B. Methanol, Aethanol etc., in Gegenwart einer Säure, z.B. einer Mineralsäure, wie Salzsäure etc. oder mit einem geeigneten Diazoalkan, z.B. Diazomethan oder Phenyldiazomethan, umgesetzt. Gemäss einer alternativen Ausführungsform kann eine Verbindung der Formel I, worin $R^2$ und/oder $R^3$ Carboxyl bedeuten, in bekannter Weise, z.B. durch Umsetzen mit Thionylchlorid, Phosphortrichlorid oder Phosphorpentachlorid, in das entsprechende Säurechlorid überführt werden, welches danach ebenfalls in bekannter Weise mit einem geeigneten Alkanol umgesetzt wird. Eine Verbindung der Formel I, worin $R^2$ und/oder $R^3$ Carboxyl bedeutet, kann auch mit Isobuten in Gegenwart von Schwefelsäure umgesetzt werden, wobei man die entsprechende Verbindung der Formel I erhält, worin $R^2$ und/oder $R^3$ tert. Butoxycarbonyl bedeuten.

Eine Verbindung der Formel I, worin $R^2$ und/oder $R^3$ Alkoxycarbonyl bedeuten, wird gemäss Verfahrensvariante g) durch Umsetzen mit einer Säure oder einer Base in die entsprechende Verbindung der Formel I übergeführt, worin $R^2$ und/oder $R^3$ Carboxyl bedeuten. Diese Ueberführung erfolgt in bekannter Weise, beispielsweise durch Umsetzen mit einem Alkalimetallhydroxid, wie Natriumhydroxid oder Kaliumhydroxid, zweckmässigerweise bei einer Temperatur zwischen Raumtemperatur und dem Siedepunkt des Reaktionsgemisches oder, falls die Alkoxycarbonylgruppe die tert. Butoxycarbonylgruppe ist, durch Umsetzen mit wasserfreier Säure.

Die Hydrogenolyse einer Verbindung der Formel I, worin $R^2$ und/oder $R^3$ Aralkoxycarbonyl bedeuten, zu einer Verbindung der Formel I, worin B Methylen oder Aethylen und $R^2$ und/oder $R^3$ Carboxyl bedeuten, gemäss Verfahrensvariante h) erfolgt ebenfalls in bekannter Weise. Bei dieser Hydrogenolyse wird eine Vinylengruppe B gleichzeitig zur Aethylengruppe reduziert.

Die Abspaltung gemäss Verfahrensvariante i) erfolgt in an sich bekannter Weise, wobei die gewählte spezifische Methode von der Art der abzuspaltenden Gruppe abhängt. Beispielsweise wird eine Aralkoxycarbonylgruppe, z.B. Benzyloxycarbonyl, durch Hydrogenolyse oder Hydrolyse abgespalten.

Die Auftrennung von diastereomeren Mischungen in die diastereomeren Racemate oder optisch reinen Diastereomeren gemäss Verfahrensvariante j) und die Auftrennung von Racematen in die beiden optischen Antipoden gemäss Verfahrensvariante k) erfolgen ebenfalls nach bekannten Methoden.

Gemäss Verfahrensvariante l) können Verbindungen der Formel I durch Umsetzen mit pharmazeutisch verwendbaren Säuren oder Basen, in die pharmazeutisch verwendbaren Salze übergeführt werden. Beispiele von pharmazeutisch verwendbaren Säuren sind anorganische Säuren, wie Hydrohalogensäuren, z.B. Bromwasserstoff oder Chlorwasserstoff, Schwefelsäure, Phosphorsäure und Salpetersäure, und organische Säuren, wie Essigsäure, Maleinsäure, Fumarsäure, Weinsäure, Zitronensäure, Methansulfonsäure und p-Toluolsulfonsäure. Beispiele von pharmazeutisch verwendbaren Basen sind Alkalimetallhydroxide, z.B. Natrium- oder Kaliumhydroxid, Erdalkalimetallhydroxide, z.B. Calcium- oder Magnesiumhydroxid, Ammoniumhydroxid und organische Basen, z.B. Dicyclohexylamin.

Die in Verfahrensvariante a) als Ausgangsstoffe eingesetzten Verbindungen der Formel II, worin B Methylen oder Vinylen, $R^3$ Carboxyl oder Alkoxycarbonyl, $R^4$ und $R^5$ zusammen eine Oxogruppe und n die Zahl 1 bedeuten, gehören einer bekannten Verbindungsklasse an. Spezifische Vertreter, welche bisher nicht beschrieben sind, können wie in den folgenden Beispielen beschrieben, oder in Analogie dazu hergestellt werden. Die übrigen Ausgangsstoffe der Formel II sind noch neu und ebenfalls Gegenstand der vorliegenden Erfindung. Sie können beispielsweise durch Cyclisation einer Verbindung der allgemeinen Formel

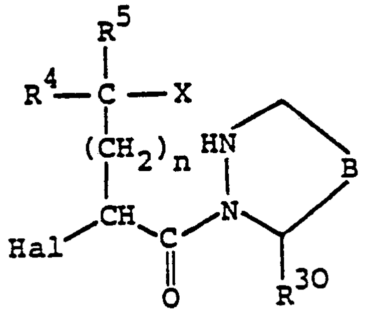

VIII

worin $R^4$, $R^5$, n, B und Hal die obige Bedeutung besitzen und X Halogen und $R^{30}$ Alkoxycarbonyl bedeuten, hergestellt werden. Falls nötig kann eine Alkoxycarbonylgruppe $R^{30}$ im erhaltenen Produkt in Carboxyl oder in eine andere Alkoxycarbonyl- oder Aralkoxycarbonylgruppe überführt werden. Die Cyclisation einer Verbindung der Formel VIII erfolgt nach konventionellen Methoden, beispielsweise in Gegenwart eines geeigneten inerten organischen Lösungsmittels, wie Dimethylformamid oder Tetrahydrofuran, erwünschtenfalls in Gegenwart einer Base, wie einem Alkalimetallcarbonat, z.B. Natriumcarbonat, Kaliumcarbonat etc. oder einer tertiären organischen Base, z.B. Triäthylamin, n-Aethylmorpholin etc., bei einer geeigneten Temperatur zwischen 0° und 80°C. Die Cyclisation wird zweckmässigerweise in situ durchgeführt, d.h. ohne Isolierung der Verbindung der Formel VIII aus dem Reaktionsmedium, in welchem diese hergestellt wird.

Die Ueberführung einer Alkoxycarbonylgruppe $R^{30}$ im erhaltenen Produkt, d.h. in einer Verbindung der Formel II, worin $R^3$ Alkoxycarbonyl bedeutet, in die Carboxylgruppe, kann in analoger Weise durchgeführt werden wie oben für die Verfahrensvariante g) angegeben. Die so erhaltene Verbindung der Formel II, worin $R^3$ Carboxyl bedeutet, kann dann erwünschtenfalls in analoger Weise wie oben für die Verfahrensvariante f) angegeben verestert werden. Alternativ dazu kann eine Verbindung der Formel II, worin $R^3$ Alkoxycarbonyl bedeutet, nach bekannten Methoden zu einer Verbindung der Formel II umgeestert werden, worin $R^3$ eine andere Alkoxycarbonyl- oder Aralkoxycarbonylgruppe bedeutet.

Die Verbindungen der Formel VIII, worin $R^4$ und $R^5$ je Wasserstoff bedeuten, können beispielsweise hergestellt werden, indem man eine Verbindung der allgemeinen Formel

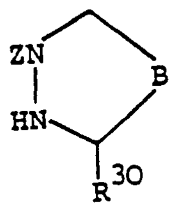

IX

mit einer Verbindung der allgemeinen Formel

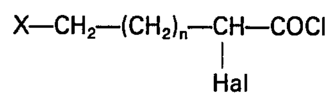

X

umsetzt und in der erhaltenen Verbindung der allgemeinen Formel

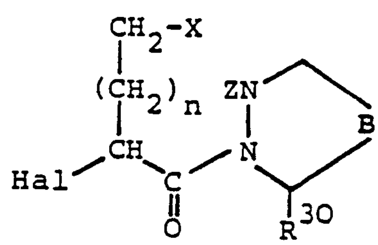

XI

die Benzyloxycarbonylgruppe entfernt, wobei in den obigen Formeln B, $R^{30}$, n X und Hal die obige Bedeutung besitzen und Z Benzyloxycarbonyl bedeutet.

Die Umsetzung einer Verbindung der Formel IX, welche bekannt oder ein Analogon zu einer bekannten Verbindung ist, mit einer Verbindung der Formel X, welche ebenfalls bekannt oder ein Analogon einer bekannten Verbindung ist, erfolgt nach allgemein bekannten Methoden, beispielsweise in Gegenwart eines inerten organischen Lösungsmittels, z.B. eines halogenierten Kohlenwasserstoffs, wie Methylenchlorid, und in Gegenwart eines säurebindenden Mittels, wie eines Alkalimetallcarbonates, z.B. Nariumcarbonat, oder eines Alkalimetallbicarbonates, z.B. Natriumbicarbonat, bei ungefähr Raumtemperatur.

Die Abspaltung der Benzyloxycarbonylgruppe aus einer Verbindung der Formel XI kann beispielsweise durch Umsetzen mit Bromwasserstoff in Eisessig bei ungefähr Raumtemperatur oder mittels Wasserstoff in Gegenwart eines Katalysators nach bekannten Methoden erfolgen.

Die Verbindungen der Formel VIII, worin $R^4$ und $R^5$ zusammen eine Oxogruppe bedeuten, können hergestellt werden, indem man beispielsweise eine Verbindung der obigen Formel IX mit einer Verbindung der allgemeinen Formel

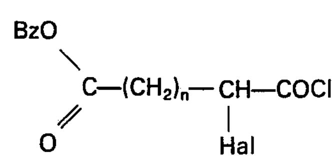

XII

worin n und Hal die obige Bedeutung besitzen und Bz Benzyl bedeutet, umsetzt, die Benzyl- und Benzyloxycarbonylgruppen in der erhaltenen Verbindung der allgemeinen Formel

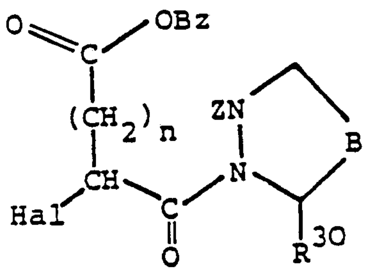

XIII

worin B, $R^{30}$, n, Hal, Bz und Z die obige Bedeutung besitzen, entfernt und die erhaltene Säure der allgemeinen Formel

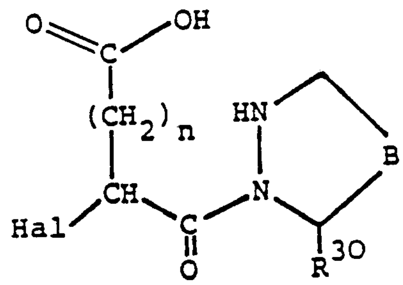

XIV

worin B, $R^{30}$, n und Hal die obige Bedeutung besitzen, in das entsprechende Säurehalogenid überführt.

Die Reaktion einer Verbindung der Formel IX mit einer Verbindung der Formel XII, welche bekannt oder ein Analogon einer bekannten Verbindung ist, kann in analoger Weise durchgeführt werden wie weiter oben für die Umsetzung einer Verbindung der Formel IX mit einer Verbindung der Formel X angegeben.

Die Entfernung der Benzyl- und Benzyloxycarbonylgruppen aus einer Verbindung der Formel XIII kann nach allgemein bekannten Methoden durchgeführt werden, beispielsweise mittels Wasserstoff in Gegenwart eines Katalysators, wie eines Edelmetallkatalysators, z.B. Palladium auf Kohle, oder, falls $R^{30}$ etwas anderes als tert. Butoxycarbonyl bedeutet, mittels Bromwasserstoff in Eisessig.

Die Ueberführung einer Säure der Formel XIV in das entsprechende Säurehalogenid der Formel VIII kann ebenfalls nach bekannten Methoden erfolgen, beispielsweise unter Verwendung eines geeigneten Halogenierungsmittels, wie Thionylchlorid, Phosphorpentachlorid und dergleichen.

Die ebenfalls in Verfahrensvariante a) als Ausgangsstoffe verwendeten Verbindungen der Formel III sind bekannt oder Analoga von bekannten Verbindungen, welche in ähnlicher Weise wie die bekannten hergestellt werden können.

Die in Verfahrensvariante b) als Ausgangsstoffe eingesetzten Verbindungen der Formel IV sind neu und ebenfalls Gegenstand vorliegender Erfindung.

Die Verbindungen der Formel IV können beispielsweise hergestellt werden, indem man eine Verbindung der Formel II mit einem Alkalimetallazid umsetzt und das erhaltene Azid der allgemeinen Formel

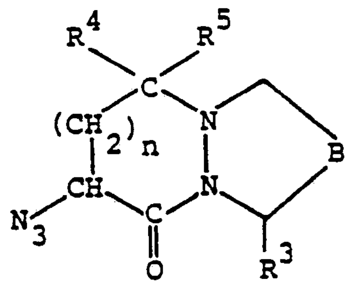

XV

worin B, $R^3$, $R^4$, $R^5$ und n die obige Bedeutung besitzen, reduziert.

Die Umsetzung einer Verbindung der Formel II mit einem Alkalimetallazid, vorzugsweise Natriumazid, erfolgt in bekannter Weise, beispielsweise in Gegenwart eines inerten organischen Lösungsmittels, wie einem Keton, z.B. Aceton, bei erhöhter Temperatur, z.B. bei der Rückflusstemperatur des Reaktionsgemisches.

Konventionelle Methoden werden für die Reduktion eines Azids der Formel XV zu einer Verbindung der Formel IV verwendet. Gemäss einer bevorzugten Ausführungsform wird ein Azid der Formel XV mit Triphenylphosphin in einem geeigneten inerten organischen Lösungsmittel, wie Dioxan, bei ungefähr Raumtemperatur umgesetzt und anschliessend mittels Säure, z.B. Salzsäure, hydrolysiert.

Die Verbindungen der Formel IV können beispielsweise auch durch Cyclisation einer Verbindung der allgemeinen Formel

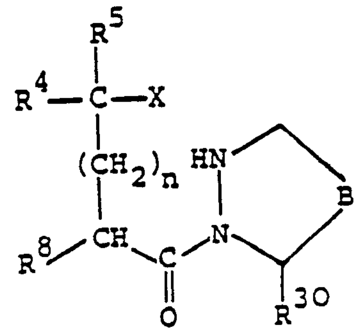

XVI

worin B, $R^{30}$, $R^4$, $R^5$, n und X die obige Bedeutung besitzen und $R^8$ Phthaloylamino bedeutet, und Entfernen der Phthaloylgruppe aus der erhaltenen Verbindung der allgemeinen Formel

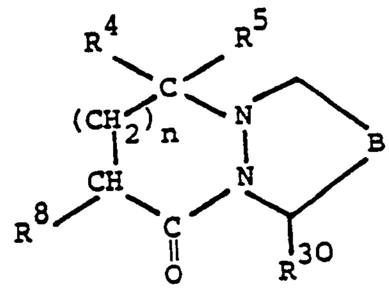

XVII

worin B, $R^{30}$, $R^4$, $R^5$, $R^8$ und n die obige Bedeutung besitzen, erhalten werden.

Die Alkoxycarbonylgruppe $R^{30}$ kann vor oder nach dem Entfernen der Phthaloylgruppe erwünschtenfalls in Carboxyl, eine andere Alkoxycarbonyl- oder Aralkoxycarbonylgruppe überführt werden.

Die Cyclisation einer Verbindung der Formel XVI kann nach allgemein bekannten Methoden durchgeführt werden, beispielsweise im wesentlichen in der selben Weise wie weiter oben im Zusammenhang mit der Cyclisation einer Verbindung der Formel VIII angegeben. Die Verbindungen der Formel XVI werden vorzugsweise in situ cyclisiert.

Das Entfernen der Phthaloylgruppe aus einer Verbindung der Formel XVII erfolgt ebenfalls in an sich bekannter Weise mittels Hydrazin, zweckmässigerweise in einem inerten organischen Lösungsmittel wie einem Alkanol, z.B. Aethanol, bei Raumtemperatur oder einer erhöhten Temperatur, z.B. bei der Rückflusstemperatur des Reaktionsgemisches.

Die allfällige Ueberführung einer Alkoxycarbonylgruppe $R^{30}$ in eine Carboxylgruppe, eine andere Alkoxycarbonyl- oder Aralkoxycarbonylgruppe kann in analoger Weise durchgeführt werden wie weiter oben im Zusammenhang mit der Herstellung der Ausgangsstoffe der Formel II angegeben.

Die Verbindungen der Formel XVI, worin $R^4$ und $R^5$ je Wasserstoff bedeuten, können beispielsweise durch Umsetzen einer Verbindung der obigen Formel IX mit einer Verbindung der allgemeinen Formel

$$X{-}CH_2{-}(CH_2)_n{-}\underset{\substack{|\\ R^8}}{CH}{-}COCl \qquad XVIII$$

worin $R^8$, n und X die obige Bedeutung besitzen, und Abspalten der Benzyloxycarbonylgruppe aus der erhaltenen Verbindung der allgemeinen Formel

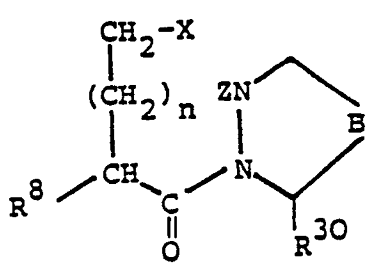

XIX

worin B, $R^{30}$, $R^8$, n, X und Z die obige Bedeutung besitzen, erhalten werden.

Die Umsetzung einer Verbindung der Formel IX mit einer Verbindung der Formel XVIII, welche bekannt oder ein Analogoneiner bekannten Verbindung ist, kann in der selben Weise durchgeführt werden wie weiter oben im Zusammenhang mit der Umsetzung einer Verbindung der Formel IX mit einer Verbindung der Formel X angegeben.

Die Abspaltung der Benzyloxycarbonylgruppe aus einer Verbindung der Formel XIX kann in derselben Weise erfolgen wie weiter oben im Zusammenhang mit dem Abspalten der Benzyloxycarbonylgruppe aus einer Verbindung der Formel XI angegeben.

Die Verbindungen der Formel XVI, worin $R^4$ und $R^5$ zusammen eine Oxogruppe bedeuten, können hergestellt werden, indem man beispielsweise eine Verbindung der obigen Formel IX mit einer Verbindung der allgemeinen Formel

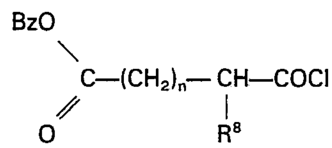

XX

worin $R^8$, n und Bz die obige Bedeutung besitzen, umsetzt, die Benzyl- und Benzyloxycarbonylgruppen aus der erhaltenen Verbindung der allgemeinen Formel

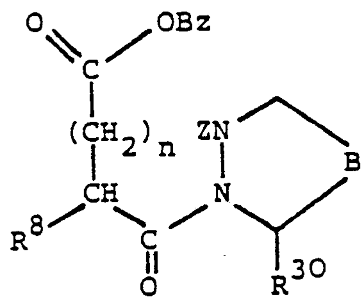

XXI

worin B, $R^8$, $R^{30}$, n, Z und Bz die obige Bedeutung besitzen, abspaltet und die erhaltene Säure der allgemeinen Formel

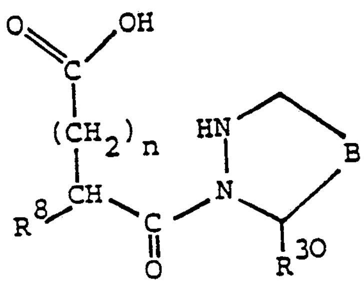

XXII

worin B, $R^8$, $R^{30}$ und n die obige Bedeutung besitzen, in das entsprechende Säurehalogenid überführt.

Die Umsetzung einer Verbindung der Formel IX mit einer Verbindung der Formel XX welche bekannt oder ein Analogon zu einer bekannten Verbindung ist, kann unter denselben Reaktionsbedingungen durchgeführt werden wie weiter oben im Zusammenhang mit der Umsetzung einer Verbindung der Formel IX mit einer Verbindung der Formel X angegeben.

Das Abspalten der Benzyl- und Benzyloxycarbonylgruppen aus einer Verbindung der Formel XXI kann in derselben Weise erfolgen wie weiter oben im Zusammenhang mit dem Abspalten dieser Gruppen aus einer Verbindung der Formel XIII angegeben.

Die Ueberführung einer Säure der Formel XXII in das entsprechende Säurehalogenid der Formel XVI kann in analoger Weise erfolgen wie weiter oben im Zusammenhang mit der Ueberführung einer Säure der Formel XIV in das entsprechende Säurehalogenid angegeben.

Die in den Verfahrensvarianten b) und c) als Ausgangsstoffe eingesetzten Verbindungen der Formeln V und VI sind entweder bekannt oder Analoga bekannter Verbindungen, welche ihrerseits in ähnlicher Weise wie die bekannten Verbindungen hergestellt werden können.

Die in Verfahrensvariante d) als Ausgangsstoffe eingesetzten Verbindungen der Formel VII sind bekannt.

Die Verbindungen der Formel I und deren pharmazeutisch verwendbaren Salze sind wertvolle Antihypertensiva. Sie hemmen das Angiotensin umwandelnde Enzym (ACE), welches für die Umwandlung

von Angiotensin I zu Angiotensin II verantwortlich ist, und sind deshalb nützlich, einen durch Angiotensin bewirkten Bluthochdruck zu vermindern oder zu lindern.

Die Aktivität der vorliegenden Verbindungen, das Angiotensin umwandelnde Enzym in vitro zu hemmen, kann durch den folgenden Test bestimmt werden.

Die verwendete Methode basiert auf einer Methode von Cushman and Cheung (Biochem. Pharmacol., *20*, 1637—1648) unter Berücksichtigung der Modifikationen von Hayakari et al. (Anal. Biochem., *84*, 361—369). Das Substrat (Hippuryl-histidyl-leucin, 2 mM) wird in Abwesenheit oder Gegenwart verschiedener Konzentrationen der Testverbindungen in Kaliumphosphatpuffer (pH 8,3; 100 mM), welches Natriumchlorid (3 mM) enthält, mit Angiotensin umwandelndem Enzym während 24 Minuten bei 37°C (Gesamtwert 500 µl) inkubiert. (Falls die Testsubstanz ein Ester ist, so ist es angezeigt, diesen mittels Schweineleberesterase vor Ausführung des Test zu spalten). Die Reaktion wird durch Zugabe von 3 ml Kaliumphosphatpuffer (pH 8,3; 200 mM) bei 0°C abgebrochen. Man versetzt anschliessend mit 2,4,6-Trichlor-s-triazin (3%) in 1,5 ml Dioxan und schüttelt die erhaltene Mischung, bis der gelbe Chromophor voll entwickelt ist. Die Proben werden anschliessend zentrifugiert, um allenfalls entstandenen Festkörper abzutrennen. Der durch Reaktion von 2,4,6-Trichlor-s-triazin mit freier Hippursäure gebildete gelbe Chromophor wird spektrophotometrisch bei 382 nm ausgemessen. Der $IC_{50}$-Wert ist diejenige Konzentration einer Testverbindung, welche die Spaltung von Hippuryl-histidyl-leucin mittels Angiotensin umwandelndem Enzym unter den oben genannten Bedingungen um 50% reduziert.

Die nachfolgende Tabelle enthält die Resultate, welche mit repräsentativen Vertretern der Verbindungen der Formel I im vorhergehend beschriebenen Text erhalten wurden.

Verbindung A: 9(S)-(1-Carboxy-3-phenylpropylamino)-octahydro-6,10-dioxo-6H-pyridazo[1,2-a][1,2]diazepin-1(S)-carbonsäure; Isomer A (vergleiche Beispiel 1).

Verbindung B: 9(S)-[1(R und S)-Carboxy-4-phenylbutylamino]-octahydro-6,10-dioxo-6H-pyridazo[1,2-a][1,2]diazepin-1(S)-carbonsäure (vergleiche Beispiel 21).

Verbindung C: 9(S)-[1(R und S)-Carboxy-2-phenyläthylamino]-octahydro-6,10-dioxo-6H-pyridazo[1,2-a][1,2]diazepin-1(S)-carbonsäure (vergleiche Beispiel 22).

Verbindung D: 9(S)-[1(R und S)-Carboxy-4-methylpentylamino]-octahydro-6,10-dioxo-6H-pyridazo[1,2-a][1,2]diazepin-1(S)-carbonsäure (vergleiche Beispiel 23).

TABELLE

| Verbindung | $IC_{50}$ |
|---|---|
| A | $4,2 \times 10^{-9}$ M |
| B | $1,1 \times 10^{-8}$ M |
| C | $2,6 \times 10^{-8}$ M |
| D | $1,2 \times 10^{-8}$ M |

Die Verbindungen der Formel I und deren pharmazeutisch verwendbare Salze können in Form pharmazeutischer Präparate, welche diese Verbindungen zusammen mit geeigneten pharmazeutischen Trägermaterialien enthalten, als Arzneimittel verwendet werden. Als Trägermaterialien kommen dabei für die enterale, beispielsweise orale, oder parenterale Verabreichung geeignete organische oder anorganische Trägermaterialien in Frage, beispielsweise Wasser, Gelatine, Gummi arabicum, Lactose, Stärke, Magnesiumstearat, Talk, pflanzliche Oele, Polyalkylenglykole, Vaselin und dergleichen. Die pharmazeutischen Präparate können in fester Form, beispielsweise als Tabletten, Dragées, Suppositorien oder Kapseln oder in flüssiger Form, z.B. als Lösungen, Suspensionen oder Emulsionen verarbeitet werden. Die pharmazeutischen Präparate können üblichen pharmazeutischen Behandlungen, wie Sterilisation, unterworfen werden und/oder Zusatzstoffe enthalten, wie Konservierungsmittel, Stabilisierungsmittel, Netzmittel oder Emulgiermittel, Salze zum Variieren des osmotischen Druckes oder Puffer. Die pharmazeutischen Präparate können auch noch andere therapeutisch wertvolle Verbindungen enthalten.

Die Verbindungen der Formel I und deren pharmazeutisch verwendbare Salze können Erwachsenen in täglichen Dosen von etwa 0,1 bis 100 mg, vorzugsweise etwa 1—50 mg/kg Körpergewicht verabreicht werden. Die tägliche Dosis kann in einer einzigen Dosis oder in Teildosen verabreicht werden. Es sei an dieser Stelle betont, dass die oben genannten Dosierungen als Beispiele angegeben wurden, und dass sie nach oben oder nach unten variiert werden können in Abhängigkeit von Faktoren, wie den spezifischen Eigenschaften der verabreichten Verbindung oder des verabreichten Salzes, der Art der Verabreichung, dem Grad der zu behandelnden Indikation und dem Zustand des Patienten, wie er vom behandelnden Arzt festgestellt wird.

Die nachfolgenden Beispiele veranschaulichen die vorliegende Erfindung. Alle Temperaturen sind in Celsiusgraden angegeben.

Beispiel 1

1,25 g 9(S) - Amino - octahydro - 6,10 - dioxo - 6H - pyridazo[1,2-a][1,2]diazepin - 1 - (S) - carbonsäure-monohydrat und 1,8 g 2-Oxo-4-phenylbuttersäure werden in 6 ml 2N-Natriumhydroxidlösing bei Raumtemperatur gelöst. Man gibt 0,3 g Natriumcyanborhydrid zu und rührt das Gemisch während 50 Minuten. Dann gibt man weitere 0,3 g Natriumcyanborhydrid zu und nach weiteren 85 Minuten nochmals 0,3 g Natriumcyanborhydrid und 0,9 g 2-Oxo-4-phenylbuttersäure. Der pH-Wert wird durch Zugabe von Natriumhydroxidlösung auf 7,5—8 eingestellt, und das Gemisch während 3 Tagen stehengelassen. Man gibt 100 ml Diäthyläther, 20 ml Wasser und 30 g Duolit C 225 Ionenaustauscherharz ($H^+$-Form) zu und mischt das Gemisch während 100 Minuten gut durch. Die ätherische Phase wird dann entfernt und die wässrige Phase, welche das Harz enthält, wird auf eine Säule gegeben, welche mit weiteren 10 g des oben genannten Harzes gefüllt ist. Die Kolonne wird mit 80 ml Wasser gewaschen und dann mit 200 ml Wasser, welches 2% Pyridin enthält, eluiert. Die Eluate werden eingedampft, wobei man 1,1 g rohes 9(S) - [1(R und S) - Carboxy - 3 - phenylpropylamino]octahydro - 6,10 - dioxo - 6H - pyridazo[1,2-a][1,2]diazepin - 1(S) - carbonsäure erhält.

Das Isomerengemisch wird auf Amberlit XAD 2 Polistyrolharzsäulen mit 0,1N wässrigem Ammoniak, welches 5% Methanol enthält, als Eluierungsmittel aufgetrennt. Diastereomer A wird vor Diastereomer B eluiert; Diastereomer A weist auch auf Silicagel-Dünnschichtchromatographieplatten, welche mit Butan-1-ol/Wasser/Essigsäure (4:1:1) eluiert werden, einen höheren Rf-Wert auf. Auf diese Weise werden 0,32 g Diastereomer A und 0,35 g Diastereomer B in Form der Ammoniumsalze erhalten, aus denen das Ammoniak mittels Duolit C 225 Harz entfernt wird, wobei man 2% Pyridin enthaltendes Wasser als Eluierungsmittel verwendet.

Das als Ausgangsmaterial verwendete 9(S) - Aminooctahydro - 6,10 - dioxo - 6H - pyridazo[1,2-a][1,2]diazepin - 1 - (S) - carbonsäuremonohydrat kann wie folgt hergestellt werden:

A) 16 g L-Glutaminsäure-γ-benzylester und 11 g Phthalsäureanhydrid, beide fein verrieben, werden bei der Rückflusstemperatur während 2 Stunden in 20 ml Toluol gerührt. Dann gibt man 50 ml Toluol zu und dampft das Reaktionsgemisch ein. Dann gibt man weitere 80 ml Toluol zu und lässt das Gemisch kristallisieren. Umkristallisation aus Toluol liefert 15 g α(S) - 2 - Benzyloxycarbonyläthyl - 1,3 - dihydro - 1,3 - dioxo - 2 - isoindolessigsäure vom Schmelzpunkt 96—98°; $[\alpha]_D^{20}$ = −50,4° (c = 1% in Methanol).

B) 56 g α(S) - (2 - Benzyloxycarbonyläthyl) - 1,3 - dihydro - 1,3 - dioxo - 2 - isoindolessigsäure in 500 ml trockenem Dimethyläther werden während 2 Stunden mit 40 g Phosphorpentachlorid bei Raumtemperatur gerührt. Man gibt weitere 2 g Phosphorpentachlorid zu, rührt das Gemisch während 30 Minuten, filtriert ungelösten Festkörper ab und dampft das Filtrat ein. Der Rückstand wird zweimal mit Toluol eingedampft und danach in 262 ml Methylenchlorid gelöst und mit Eis/Wasser gekühlt. Unter Rühren gibt man 262 ml Methylenchlorid, welche 44 g tert.-Butyl 1-(benzyloxycarbonyl)hexahydro-3-pyridazincarboxylat enthalten und unmittelbar darauf 420 ml gesättigte wässrige Natriumbicarbonatlösung zu. Das Gemisch wird bei Raumtemperatur über Nacht gerührt, danach mit Aethylacetat verdünnt und partiell soweit eingedampft, bis sich die Emulsion abscheidet. Die organische Phase wird danach mit wässriger Natriumdihydrogenphosphatlösung, wässriger Natriumcarbonatlösung und Kochsalzlösung gewaschen, getrocknet und eingedampft. Der Rückstand wird in 1040 ml Methanol gelöst und bei Atmosphärendruck über 7,4 g 10% Palladium auf Kohle bis zur beendeten Wasserstoffaufnahme hydriert. Der Katalysator wird entfernt und das Filtrat eingedampft, wobei man 61 g rohe gamma(S) - (6 - tert. - Butoxycarbonylhexahydro - 1 - pyridazinyl) - carbonyl - 1,3 - dioxo - 2 - isoindolinbuttersäure (2 Diastereomere) erhält. Kristallisation aus Aethylacetat/Diäthyläther liefert 19 g des (S,S)-Isomeren von Schmelzpunkt 132—134°; $[\alpha]_D^{25}$ = −54,4° (c = 0,5% in Methanol). Chromatographie an Silicagel unter Verwendung von Diäthyläther als Eluierungsmittel liefert das (S,R)-Isomere vom Schmelzpunkt 134—137° (aus Aethylacetat/Diäthyläther); $[\alpha]_D^{25}$ = −6,2° (c = 0,5% in Methanol).

C) 2,2 g gamma(S) - (6(S) - tert. - Butoxycarbonyl - hexahydro - 1 - pyridazinyl] - carbonyl - 1,3 - dioxo - 2 - isoindol in buttersäure in 60 ml trockenem Tetrahydrofuran werden bei 0° mit 1,1 ml N-Aethylmorpholin und 1,1 g Phosphorpentachlorid gerührt. Nach 1 Stunde gibt man weitere 0,2 ml N-Aethylmorpholin und 0,2 g Phosphorpentachlorid zu und nach weiteren 5 Stunden weitere 0,15 ml N-Aethylmorpholin und 0,15 g Phosphorpentachlorid. Man lässt das Reaktionsgemisch über Nacht bei Raumtemperatur stehen, dampft ein, verdünnt den Rückstand mit 150 ml Aethylacetat, wäscht die Lösung mit 1N-Salzsäure- und Natriumchloridlösung und trocknet über Magnesiumsulfat. Eindampfen des Lösungsmittels liefert 2,4 g rohes tert.-Butyl octahydro - 6,10 - dioxo - 9(S) - phthalimido - 6H - pyridazo[1,2-a][1,2]diazepin - 1(S) - carboxylat. Nach Reinigung durch Chromatographie an Silicagel unter Verwendung von Aether als Eluierungsmittel und Kristallisation aus Aethylacetat/Diäthyläther schmilzt das Produkt bei 181—185°, $[\alpha]_D^{20}$ = −80,0° (c = 0,5% in Methanol).

D) 2,4 g tert. - Butyl octahydro - 6,10 - dioxo - 9(S) - phthalimido - 6H - pyridazo[1,2-a][1,2]diazepin - 1(S) - carboxylat werden in 20 ml Trifluoressigsäure gelöst. Nach 80 Minuten wird das Gemisch eingedampft, und der Rückstand zweimal mit Toluol versetzt und wieder eingedampft. Der Rückstand wird mit Aethylacetat verrieben, wobei man 1,7 g Octahydro - 6,10 - dioxo - 9(S) - phthalimido - 6H - pyridazo[1,2-a][1,2]diazepin - 1 - (S) - carbonsäure erhält. Eine Probe wird aus Aceton/Wasser umkristallisiert und schmilzt bei 307—310° (Zers.); $[\alpha]_D^{20}$ = −139° (c = 0,5% in Dimethylformamid).

E) 4,9 g Octahydro - 6,10 - dioxo - 9(S) - phthalimido - 6H - pyridazo[1,2-a][1,2]diazepin - 1(S) - carbonsäure werden während 40 Minuten in 80 ml Aethanol, welches 1,4 ml Hydrazinhydrat enthält,

erhitzt. Das Gemisch wird über Nacht bei Raumtemperatur stehengelassen und danach eingedampft. 100 ml 2N-Essigsäure werden zugegeben, das Gemisch bei Raumtemperatur während 70 Minuten gerührt und dann filtriert. Das Filtrat wird eingedampft und der Rückstand nochmals mit Wasser eingedampft. Der Rückstand wird danach in 60 ml warmem Wasser gelöst, filtriert und das Filtrat eingeengt. Dann verdünnt man mit 30 ml Aethanol und lässt kristallisieren. Auf diese Weise erhält man 2,65 g 9(S) - Amino - octahydro - 6,10 - dioxo - 6H - pyridazo[1,2-a][1,2]diazepin - 1(S) - carbonsäuremonohydrat von Schmelzpunkt 195—200° (Zers.); $[\alpha]_D^{20} = -174,6°$ (c = 0,5% in 2N-Salzsäure).

Beispiel 2

2,3 g 9(S) - Amino - octahydro - 6,10 - dioxo - 6H - pyridazo[1,2-a][1,2]diazepin - 1(S) - carbonsäuremonohydrat werden bei Raumtemperatur in 25 ml Aethanol und 5 ml Wasser mit 1,1 g Aethyl 2-oxo-4-phenylbutyrat und 0,3 g Natriumcyanborhydrid gerührt. Während der nächsten 48 Stunden werden weitere 8 g Aethyl 2-oxo-4-phenylbutyrat und 1,5 g Natriumcyanborhydrid in 5 Portionen zu der gerührten Mischung gegeben, welche danach für 3 Tage stehengelassen wird. Das Gemisch wird eingedampft, der Rückstand mit 120 ml Aethylacetat verdünnt, und die erhaltene Lösung mit zweimal 50 ml Wasser und mit 5 ml gesättigter wässriger Natriumbicarbonatlösung extrahiert. Die wässrigen Extrakte werden mit 30 ml Diäthyläther gewaschen, danach angesäuert (pH 3—4) und mit 100 ml Aethylacetat und 50 ml Aethylacetat extrahiert. Die vereinigten organischen Extrakte werden mit Natriumchloridlösung gewaschen, über Magnesiumsulfat getrocknet und eingedampft, wobei man 3,7 g rohe 9(S) - [1(R und S) - Aethoxycarbonyl - 3 - phenylpropylamino] - octahydro - 6,10 - dioxo - 6H - pyridazo[1,2-a][1,2]diazepin - 1(S) - carbonsäure in Form eines Diastereomerengemisches erhält, welches auf einer Silicageldünnschichtchromatographieplatte mit Diäthyläther, welcher 10% Essigsäure enthält, als Laufmittel aufgetrennt werden. Die Auftrennung der beiden Diastereomeren erfolgt durch Chromatographie auf einer Silicagelsäule unter Verwendung von Diäthyläther, welcher 5—15% Essigsäure enthält, als Eluierungsmittel. Das Diastereomere mit dem niedrigeren Rf-Wert (Isomer B) wird in Toluol gelöst und mit trockenem Chlorwasserstoff behandelt. Der so erhaltene Festkörper wird aus Aethanol/Aethylacetat umkristallisiert, wobei man 9(S) - [1(R oder S) - Aethoxycarbonyl - 3 - phenylpropylamino] - octahydro - 6,10 - dioxo - 6H - pyridazo[1,2-a][1,2]diazepin - 1(S) - carbonsäurehydrochlorid vom Schmelzpunkt 202—207° (Zers.) erhält.

Beispiel 3

In analoger Weise wie im ersten Absatz von Beispiel 1 beschrieben, erhält man aus 1,13 g 8(S) - Amino - 2,3,6,7,8,9 - hexahydro - 5,9 - dioxo - 1H,5H - pyrazolo[1,2-a][1,2]diazepin - 1(S) - carbonsäure und 2,7 g 2 - Oxo - 4 - phenylbuttersäure 350 mg 8(S) - 1 - (Carboxy - 3 - phenylpropylamino) - 2,3,6,7,8,9 - hexahydro - 5,9 - dioxo - 1H,5H - pyrazolo[1,2-a][1,2]diazepin - 1 - (S) - carbonsäure in Form einer lyophylisierten Mischung.

Die als Ausgangsmaterial verwendete 8(S) - Amino - 2,3,6,7,8,9 - hexahydro - 5,9 - dioxo - 1H,5H - pyrazolo[1,2-a][1,2]diazepin - 1(S) - carbonsäure kann wie folgt hergestellt werden:

A) 17,2 g tert.-Butylacrylat werden solange mit einer Lösung von Diazomethan in Diäthyläther versetzt, bis die gelbe Färbung während 2 Minuten nach der Zugabe bestehenbleibt. Man entfernt das Lösungsmittel durch Eindampfen, löst das zurückbleibende Oel in 300 ml Aethanol und hydriert die Lösung über 10% Palladium auf Kohle. Der Katalysator wird abfiltriert und das Filtrat eingedampft. Man löst das zurückbleibende Oel in 260 ml Aethylacetat und gibt eine Lösung von 24 g Natriumbicarbonat in 260 ml Wasser zu. Man kühlt die Mischung unter Rühren auf 0° ab und versetzt diese tropfenweise mit einer Lösung von 18,76 g Benzylchloroformat in 50 ml Aethylacetat. Nach 1 Stunde wird die organische Phase abgetrennt, mit 2N-Salzsäure und Natriumchloridlösung gewaschen, über Magnesiumsulfat getrocknet und eingedampft. Das zurückbleibende Oel wird an Silicagel chromatographiert. Eluieren mit Aethylacetat/n-Hexan (1:1) liefert 10,81 g (25%) 1-Benzyl 3-tert.-butyl 1,3-pyrazolidindicarboxylat als weissen Festkörper vom Schmelzpunkt 55—57° (aus Diäthyläther/n-Hexan).

B) 22,02 g α(S) - (2 - Benzyloxycarbonyläthyl) - 1,3 - dihydro - 1,3 - dioxo - 2 - isoindolessigsäure (hergestellt wie in Beispiel 1 beschrieben) in 210 ml trockenem Diäthyläther werden mit 15,01 g Phosphorpentachlorid während 2 1/2 Stunden gerührt. Das Gemisch wird filtriert und das Filtrat eingedampft. Man nimmt den Rückstand zweimal in Toluol auf und dampft ihn jedesmal wieder ein, löst danach den Rückstand in 110 ml Methylenchlorid und kühlt die Lösung auf 0°. Die Lösung wird dann bei 0° gerührt und mit einer Lösung von 15,3 g 1-Benzyl 3-tert.-butyl 1,3-pyrazolidindicarboxylat in 110 ml Methylenchlorid versetzt. 170 ml gesättigte wässrige Natriumbicarbonatlösung wird zugegeben, und das Gemisch während 1 Stunde bei Raumtemperatur gerührt. Das organische Lösungsmittel wird abgedampft, und der Rückstand mit Aethylacetat extrahiert. Die Extrakte werden mit Natriumchloridlösung gewaschen, über Magnesiumsulfat getrocknet und eingedampft. Das so erhaltene Oel wird an Silicagel chromatographiert. Eluieren mit Aethylacetat/n-Hexan (1:1) liefert 25,62 g (78%) tert.-Butyl 1 - benzyloxycarbonyl - 2 - (5 - benzyloxycarbonyl - 2 - phthalimidobutyryl) - 3 - pyrazolidincarboxylat (2 Diastereomere) in Form eines farblosen Oels.

C) 24,97 g tert.-Butyl 1 - benzyloxycarbonyl - 2 - (5 - benzyloxycarbonyl - 2 - phthalimidobutyryl) - 3 - pyrazolidincarboxylat werden in 250 ml Methanol gelöst und über 2 g 10% Palladium auf Kohle hydriert. Der Katalysator wird abfiltriert, und das Filtrat eingedampft, wobei man 16 g (98%) tert.-Butyl 2 -

(5 - carboxy - 2 - phthalimidobutyryl) - 3 - pyrazolidincarboxylat (2 Diastereomere) in Form eines Gummis erhält.

D) Eine Lösung von 3,45 g tert.-Butyl 2 - (5 - carboxy - 2 - phthalimidobutyryl) - 3 - pyrazolidincarboxylat und 0,92 g N-Aethylmorpholin in 50 ml trockenem Tetrahydrofuran wird unter Rühren auf 0° abgekühlt und mit 1,66 g Phosphorpentachlorid versetzt. Nach 1 Stunde werden weitere 0,92 g N-Aethylmorpholin und 1,66 g Phosphorpentachlorid zugegeben, und nach einer weiteren Stunde, weitere 0,92 g N-Aethylmorpholin. Das Lösungsmittel wird abgedampft, und der Rückstand zwischen Wasser und Aethylacetat verteilt. Die organische Phase wird mit gesättigter wässriger Natriumbicarbonatlösung gewaschen, über Magnesiumsulfat getrocknet und eingedampft. Das zurückbleibende Oel wird an Silicagel chromatographiert. Eluieren mit Diäthyläther liefert zunächst 1,1 g (33%) tert.-Butyl 2,3,6,7,8,9 - hexahydro - 5,9 - dioxo - 8(S) - phthalimido - 1H,5H - pyrazolo[1,2-a][1,2]diazepin - 1(S) - carboxylat in Form eines weissen Schaumes und danach 0,52 g (15%) tert.-Butyl 2,3,6,7,8,9 - hexahydro - 5,9 - dioxo - 8(S) - phthalimido - 1H,5H - pyrazolo[1,2-a][1,2]diazepin - 1(R) - carboxylat in Form eines weissen Festkörpers vom Schmelzpunkt 180—181° (aus Aethylacetat/n-Hexan).

E) In analoger Weise wie in Beispiel 1D) beschrieben, erhält man aus 3,72 g tert.-Butyl 2,3,6,7,8,9 - hexahydro - 5,9 - dioxo - 8(S) - phthalimido - 1H-5H - pyrazolo[1,2-a][1,2]diazepin - 1(S) - carboxylat und 30 ml Trifluoressigsäure 2,09 g (65%) 2,3,6,7,8,9 - Hexahydro - 5,9 - dioxo - 8(S) - phthalimido - 1H,5H - pyrazolo[1,2-a][1,2]diazepin - 1(S) - carbonsäure in Form eines weissen Festkörpers vom Schmelzpunkt 232—233° (aus Acetonitril).

F) In analoger Weise wie in Beispiel 1E) beschrieben, jedoch unter Verwendung von Hydrazinhydrat bei Raumtemperatur anstelle von 70°, erhält man aus 3,57 g 2,3,6,7,8,9 - Hexahydro - 5,9 - dioxo - 8(S) - phthalimido - 1H,5H - pyrazolo[1,2-a][1,2]diazepin - 1(S) - carbonsäure 2,0 g (88%) 8(S) - Amino - 2,3,6,7,8,9 - hexahydro - 5,9 - dioxo - 1H,5H - pyrazolo[1,2-a][1,2]diazepin - 1(S) - carbonsäure in Form eines lyophylisierten Festkörpers.

### Beispiel 4

5,94 g tert.Butyl 9(S) - amino - octahydro - 6,10 - dioxo - 6H - pyridazo[1,2-a][1,2]diazepin - 1(S) - carboxylat, 5,42 g Aethyl 2-brom-4-phenylbutanoat und 2,0 g Triäthylamin werden in 65 ml Acetonitril gelöst, und die Lösung während 17 Stunden unter Rückfluss gekocht. Nach dem Eindampfen wird der Rückstand zwischen Aethylacetat und Wasser verteilt. Die organische Phase wird nacheinander mit 2N wässriger Essigsäure und wässriger Natriumcarbonatlösung gewaschen und danach eingedampft. Nach Säulenchromatographie an Silicagel unter Verwendung von Diäthyläther/n-Hexan als Eluierungsmittel erhält man 3,1 g tert.Butyl 9(S) - [1(R) - äthoxycarbonyl - 3 - phenylpropylamino] - octahydro - 6,10 - dioxo - 6H - pyridazo[1,2-a][1,2]diazepin - 1(S) - carboxylat als Gummi und 3,25 g tert.Butyl 9(S) - [1(S) - äthoxycarbonyl - 3 - phenylpropylamino] - octahydro - 6,10 - dioxo - 6H - pyridazo[1,2-a][1,2]diazepin - 1(S) - carboxylat als weisse Kristalle vom Schmelzpunkt 55—58° nach Behandlung mit Hexan.

Das als Ausgangsmaterial verwendete tert.Butyl 9(S) - amino - octahydro - 6,10 - dioxo - 6H - pyridazo[1,2-a][1,2]diazepin - 1(S) - carboxylat kann wie folgt hergestellt werden:

8,54 g tert.Butyl octahydro - 6,10 - dioxo - 9(S) - phthalimido - 6H - pyridazo[1,2-a][1,2]diazepin - 1(S) - carboxylat werden in 85 ml Aethanol suspendiert und mit 2 ml Hydrazinhydrat versetzt. Das Gemisch wird während 2 1/2 Stunden bei 20° gerührt und danach eingedampft. Der Rückstand wird während 1 Stunde mit 2N wässriger Essigsäure gerührt und danach filtriert. Das Filtrat wird mit festem Natriumcarbonat basisch gestellt und mit Methylenchlorid extrahiert. Die organischen Extrakte werden getrocknet und eingedampft, wobei man 5,9 g tert.Butyl 9(S) - amino - octahydro - 6,10 - dioxo - 6H - pyridazo[1,2-a]][1,2]diazepin - 1(S) - carboxylat als Gummi erhält.

### Beispiel 5

131,8 g tert.Butyl 9(S) - amino - octahydro - 6,10 - dioxo - 6H - pyridazo[1,2-a][1,2]diazepin - 1(S) - carboxylat und 61,6 ml Triäthylamin werden in 2,8 l Acetonitril gelöst. Die Lösung wird mit 150,9 g Aethyl 2(R)-trifluormethansulfonyloxy-4-phenylbutanoat (nach Standardmethoden aus 2(R)-Hydroxy-4-phenylbuttersäure hergestellt) versetzt, und das Gemisch während 90 Minuten bei 20° gerührt. Die Lösung wird eingedampft, und das zurückbleibende Oel zwischen Aethylacetat und Wasser verteilt. Die organische Phase wird getrocknet und eingedampft, und der Rückstand durch eine kurze Kolonne von Silicagel unter Verwendung von Aethylacetat/n-Hexan (3:1) als Eluierungsmittel filtriert. Eindampfen der Eluate liefert 187,5 g tert.Butyl 9(S) - [1(S) - äthoxycarbonyl - 3 - phenylpropylamino] - octahydro - 6,10 - dioxo - 6H - pyridazo[1,2-a][1,2]diazepin - 1(S) - carboxylat.

### Beispiel 6

13,7 g tert.Butyl 9(S) - amino - octahydro - 6,10 - dioxo - 6H - pyridazo[1,2-a][1,2]diazepin - 1(S) - carboxylat und 29 g Aethyl 2-oxo-4-phenylbutanoat werden bei 20° während 17 Stunden in 200 ml Tetrahydrofuran in Gegenwart von 15 g verriebenem 4A Molekularsieb gerührt. Fünf 1,5 g-Portionen Natriumcyanborhydrid werden in stündlichen Intervallen zugegeben. Nach beendeter Zugabe wird für weitere 2 Stunden gerührt. Das Lösungsmittel wird abgedampft, und der Rückstand zwischen Aethylacetat und wässriger Natriumcarbonatlösung verteilt. Die organische Phase wird eingedampft, und der Rückstand an Silicagel chromatographiert, wobei man Diäthyläther/n-Hexan als Eluierungsmittel verwendet. So

werden 4,8 g tert.Butyl 9(S) - [1(R) - äthoxycarbonyl - 3 - phenylpropylamino] - octahydro - 6,10 - dioxo - 6H - pyridazo[1,2-a][1,2]diazepin - 1(S) - carboxylat und 4,1 g tert.Butyl 9(S) - [1(S) - äthoxycarbonyl - 3 - phenylpropylamino] - octahydro - 6,10 - dioxo - 6H - pyridazo[1,2-a][1,2]diazepin - 1(S) - carboxylat erhalten.

Beispiel 7

2,0 g tert.Butyl 9(S) - amino - octahydro - 6,10 - dioxo - 6H - pyridazo[1,2-a][1,2]diazepin - 1(S) - carboxylat und 4,2 g Aethyl 2-oxo-4-phenylbutanoat werden in 50 ml Aethanol, welches 5 ml Essigsäure enthält, gelöst. 4 g zerriebenes Molekularsieb werden zugegeben, und das Gemisch über 10% Palladium auf Kohle bei 20° und 4 Atmosphären während 40 Stunden hydriert. Abfiltrieren und Eindampfen liefert einen öligen Rückstand, der an Silicagel unter Verwendung von Aethylacetat/n-Hexan als Eluierungsmittel chromatographiert wird. Auf diese Weise werden 950 mg tert.Butyl 9(S) - [1(R) - äthoxycarbonyl - 3 - phenylpropylamino] - octahydro - 6,10 - dioxo - 6H - pyridazo[1,2-a][1,2]diazepin - 1(S) - carboxylat und 870 mg tert.Butyl 9(S) - [1(S) - äthoxycarbonyl - 3 - phenylpropylamino] - octahydro - 6,10 - dioxo - 6H - pyridazo[1,2-a][1,2]diazepin - 1(S) - carboxylat erhalten.

Beispiel 8

860 mg tert.Butyl 9(S) - [1(S) - äthoxycarbonyl - 3 - phenylpropylamino] - octahydro - 6,10 - dioxo - 6H - pyridazo[1,2-a][1,2]diazepin - 1(S) - carboxylat werden in 1 ml Essigsäure gelöst und mit 4 ml einer 45%-igen Lösung von Bromwasserstoff in Essigsäure versetzt. Die Lösung wird während einer Stunde bei 20° stehen gelassen und danach eingedampft. Der Rückstand wird mit Diäthyläther gerührt und filtriert, wobei man 840 mg 9(S) - [1(S) - äthoxycarbonyl - 3 - phenylpropylamino] - octahydro - 6,10 - dioxo - 6H - pyridazo[1,2-a][1,2]diazepin - 1(S) - carbonsäure - hydrobromid vom Schmelzpunkt 216—218° (aus Aethanol/Aethylacetat) erhält.

Beispiel 9

In analoger Weise wie in Beispiel 8 beschrieben, erhält man aus 5,4 g tert.Butyl 9(S) - [1(R) - äthoxycarbonyl - 3 - phenylpropylamino] - octahydro - 6,10 - dioxo - 6H - pyridazo[1,2-a][1,2]diazepin - 1(S) - carboxylat 4,41 g 9(S) - [1(R) - äthoxycarbonyl - 3 - phenylpropylamino] - octahydro - 6,10 - dioxo - 6H - pyridazo[1,2-a][1,2]diazepin - 1(S) - carbonsäure-hydrobromid vom Schmelzpunkt 201—203° (aus Aethanol/Aethylacetat).

Beispiel 10

In analoger Weise wie in Beispiel 7 beschrieben, erhält man aus 5,34 g 2-Oxo-4-phenylbuttersäure und 2,97 g tert.Butyl 9(S) - amino - octahydro - 6,10 - dioxo - 6H - pyridazo[1,2-a][1,2]diazepin - 1(S) - carboxylat 1,7 g tert.Butyl 9(S) - [1(R) - carboxy - 3 - phenylpropylamino] - octahydro - 6,10 - dioxo - 6H - pyridazo[1,2-a][1,2]diazepin - 1(S) - carboxylat und 1,75 g tert.Butyl 9(S) - [1(S) - carboxy - 3 - phenylpropylamino] - octahydro - 6,10 - dioxo - 6H - pyridazo[1,2-a][1,2]diazepin - 1(S) - carboxylat, wobei beide Diastereomere nach Chromatographie an Silicagel als Gummi isoliert werden.

Beispiel 11

500 mg tert.Butyl 9(S) - [1(S) - carboxy - 3 - phenylpropylamino] - octahydro - 6,10 - dioxo - 6H - pyridazo[1,2-a][1,2]diazepin - 1(S) - carboxylat werden in Diäthyläther gelöst und bei 20° mit einer Lösung von Phenyldiazomethan in Diäthyläther versetzt. Die Lösung wird mit wässriger Natriumbicarbonatlösung gewaschen, getrocknet und eingedampft. Der Rückstand wird an Silicagel chromatographiert, wobei man Aethylacetat/n-Hexan (1:1) als Eluierungsmittel verwendet. Auf diese Weise erhält man 430 mg tert.Butyl 9(S) - [1(S) - benzyloxycarbonyl - 3 - phenylpropylamino] - octahydro - 6,10 - dioxo - 6H - pyridazo[1,2-a][1,2]diazepin - 1(S) - carboxylat als Gummi.

Beispiel 12

410 mg tert.Butyl 9(S) - [1(S) - benzyloxycarbonyl - 3 - phenylpropylamino] - octahydro - 6,10 - dioxo - 6H - pyridazo[1,2-a][1,2]diazepin - 1(S) - carboxylat werden mit 3 ml Trifluoressigsäure versetzt. Die Lösung wird während 1 1/2 Stunden bei 20° stehen gelassen und danach eingedampft. Der zurückbleibende Schaum wird in Aethylacetat aufgenommen und eine Lösung von Chlorwasserstoff in Aethylacetat zugegeben. Der ausgefallene Festkörper wird abfiltriert, wobei man 330 mg 9(S) - [1(S) - benzyloxycarbonyl - 3 - phenylpropylamino] - octahydro - 6,10 - dioxo - 6H - pyridazo[1,2-a][1,2]diazepin - 1(S) - carbonsäurehydrochlorid als weissen Festkörper von Schmelzpunkt 198—202° erhält.

Beispiel 13

260 mg 9(S) - [1(S) - benzyloxycarbonyl - 3 - phenylpropylamino] - octahydro - 6,10 - dioxo - 6H - pyridazo[1,2-a][1,2]diazepin - 1(S) - carbonsäure wird in Diäthyläther gelöst, und die Lösung bei 20° mit einer Lösung von Diazomethan in Diäthyläther versetzt. Nach 10 Minuten wird die Lösung mit wässriger Natriumbicarbonatlösung gewaschen, getrocknet und eingedampft. Chromatographie des verbleibenden Gummis an Silicagel unter Verwendung von Aethylacetat/Hexan (1:1) als Eluierungsmittel liefert 180 mg

Methyl 9(S) - [1(S) - benzyloxycarbonyl - 3 - phenylpropylamino] - octahydro - 6,10 - dioxo - 6H - pyridazo[1,2-a][1,2]diazepin - 1(S) - carboxylat als farblosen Gummi.

Beispiel 14

170 mg Methyl 9(S) - [1(S) - benzyloxycarbonyl - 3 - phenylpropylamino] - octahydro - 6,10 - dioxo - 6H - pyridazo[1,2-a][1,2]diazepin - 1(S) - carboxylat werden in 5 ml Essigsäure gelöst und über 10% Palladium auf Kohle bei 20° und Atmosphärendruck während 17 Stunden hydriert. Der Katalysator wird abfiltriert, und das Filtrat eingedampft. Der Rückstand wird in Aethylacetat aufgenommen, und eine Lösung von Chlorwasserstoff in Aethylacetat zugegeben. Der ausgefallene Festkörper wird abfiltriert, wobei man 85 mg Methyl 9(S) - [1(S) - carboxy - 3 - phenylpropylamino] - octahydro - 6,10 - dioxo - 6H - pyridazo[1,2-a][1,2]diazepin - 1(S) - carboxylat als weissen Festkörper mit einem diffusen Schmelzpunkt erhält.

Beispiel 15

715 mg 9(S) - [1(R und S) - äthoxycarbonyl - 3 - phenylpropylamino] - octahydro - 6,10 - dioxo - 6H - pyridazo[1,2 - a][1,2]diazepin - 1(S) - carbonsäure werden in Diäthyläther gelöst, und die Lösung mit einer Lösung von Diazomethan in Diäthyläther versetzt. Die Diastereomeren werden chromatographisch an Silicagel aufgetrennt, wobei man Aethylacetat/n-Hexan (1:1) als Eluierungsmittel verwendet. Auf diese Weise erhält man in form ihrer kristallinen Hydrochloridsalze aus Aethylacetat Methyl 9(S) - [1(R) - äthoxycarbonyl - 3 - phenylpropylamino] - octahydro - 6,10 - dioxo - 6H - pyridazo[1,2 - a][1,2]diazepin - 1(S) - carboxylat - hydrochlorid vom Schmelzpunkt 161—163° (Zers.) und Methyl 9(S) - [1(S) - äthoxycarbonyl - 3 - phenylpropylamino] - octahydro - 6,10 - dioxo - 6H - pyridazo[1,2 - a][1,2]diazepin - 1(S) - carboxylat - hydrochlorid vom Schmelzpunkt 175—178° (Zers.).

Beispiel 16

500 mg 9(S) - [1(S) - äthoxycarbonyl - 3 - phenylpropylamino] - octahydro - 6,10 - dioxo - 6H - pyridazo[1,2 - a][1,2]diazepin - 1(S) - carbonsäure werden in 30 ml Aethanol gelöst, und die Lösung mit trocknem Chlorwasserstoff gesättigt. Nach 17-stündigem Stehen bei 20° wird die Lösung eingedampft, und der Rückstand mit Diäthyläther versetzt. Durch Filtrieren erhält man Aethyl 9(S) - [1(S) - äthoxycarbonyl - 3 - phenylpropylamino] - octahydro - 6,10 - dioxo - 6H - pyridazo[1,2 - a][1,2]diazepin - 1(S) - carboxylat - hydrochlorid als weissen Festkörper vom Schmelzpunkt 187—189° (Zers.).

Beispiel 17

487 mg tert.Butyl 9(S) - [1(S) - äthoxycarbonyl - 3 - phenylpropylamino] - octahydro - 6,10 - dioxo - 6H - pyridazo[1,2 - a][1,2]diazepin - 1(S) - carboxylat werden in Methanol aufgenommen, und die Lösung mit Ammoniak gesättigt. Das Gemisch lässt man 15 Tage bei 0° stehen und dampft danach ein. Chromatographie des Rückstandes an Silicagel unter Verwendung von Aethylacetat/n-Hexan (3:1) als Eluierungsmittel liefert 340 mg tert.Butyl 9(S) - [1(S) - carbamoyl - 3 - phenylpropylamino] - octahydro - 6,10 - dioxo - 6H - pyridazo[1,2 - a][1,2]diazepin - 1(S) - carboxylat als Gummi.

Beispiel 18

In analoger Weise wie in Beispiel 8 beschrieben, erhält man aus 260 mg tert.Butyl 9(S) - [1(S) - carbamoyl - 3 - phenylpropylamino] - octahydro - 6,10 - dioxo - 6H - pyridazo[1,2 - a][1,2]diazepin - 1(S) - carboxylat 250 mg 9(S) - [1(S) - Carbamoyl - 3 - phenylpropylamino] - octahydro - 6,10 - dioxo - 6H - pyridazo[1,2 - a][1,2]diazepin - 1(S) - carbonsäure - hydrobromid als weisse Kristalle vom Schmelzpunkt 170—190° (Zers.); (aus Aethanol/Aethylacetat).

Beispiel 19

In analoger Weise wie in Beispiel 17 beschrieben, erhält man aus 487 mg tert.Butyl 9(S) - [1(S) - äthoxycarbonyl - 3 - phenylpropylamino] - octahydro - 6,10 - dioxo - 6H - pyridazo[1,2 - a][1,2]diazepin - 1(S) - carboxylat unter Verwendung von äthanolischem Aethylamin 260 mg tert.Butyl 9(S) - [1(S) - Aethyl - carbamoyl - 3 - phenylpropylamino] - octahydro - 6,10 - dioxo - 6H - pyridazo[1,2 - a]-[1,2]diazepin - 1(S) - carboxylat als Gummi.

Beispiel 20

In analoger Weise wie in Beispiel 8 beschrieben, erhält man aus 210 mg tert.Butyl 9(S) - [1(S) - äthylcarbamoyl - 3 - phenylpropylamino] - octahydro - 6,10 - dioxo - 6H - pyridazo[1,2 - a][1,2]diazepin - 1(S) - carboxylat 180 mg 9(S) - [1(S) - Aethylcarbamoyl - 3 - phenylpropylamino] - octahydro - 6,10 - dioxo - 6H - pyridazo[1,2 - a][1,2]diazepin - 1(S) - carbonsäure als weissen Festkörper, der sich nach Kristallisation aus Aethanol/Aethylacetat oberhalb 170° zersetzt.

Beispiel 21

In analoger Weise wie in Beispiel 1 beschrieben, erhält man aus 241 mg 9(S) - Amino - octahydro - 6,10 - dioxo - 6H - pyridazo[1,2 - a][1,2]diazepin - 1(S) - carbonsäure und 576 mg 2 - Oxo - 5 - phenylpentansäure 100 mg 9(S) - [1(R und S) - Carboxy - 4 - phenylbutylamino] - octahydro - 6,10 - dioxo - 6H - pyridazo[1,2 - a][1,2]diazepin - 1(S) - carbonsäure als weissen Festkörper.

Beispiel 22

In analoger Weise wie in Beispiel 1 beschrieben, erhält man aus 241 mg 9(S) - Amino - octahydro - 6,10 - dioxo - 6H - pyridazo[1,2 - a][1,2]diazepin - 1(S) - carbonsäure und 612 mg Natriumphenylpyruvatmonohydrat 110 mg 9(S) - [1(R und S) - Carboxy - 4 - phenyläthylamino] - octahydro - 6,10 - dioxo - 6H - pyridazo[1,2 - a][1,2]diazepin - 1(S) - carbonsäure als weissen Festkörper.

Beispiel 23

In analoger Weise wie in Beispiel 1 beschrieben, erhält man aus 241 mg 9(S) - Amino - octahydro - 6,10 - dioxo - 6H - pyridazo[1,2 - a][1,2]diazepin - 1(S) - carbonsäure und 432 mg 2 - Oxo - 5 - methylhexansäure 68 mg 9(S) - [1(R und S) - Carboxy - 4 - methylpentylamino] - octahydro - 6,10 - dioxo - 6H - pyridazo[1,2 - a][1,2]diazepin - 1(S) - carbonsäure als weissen Festkörper.

Beispiel 24

In analoger Weise wie in Beispiel 1 beschrieben, erhält man aus 2,97 g tert.Butyl 9(S) - Amino - octahydro - 6,10 - dioxo - 6H - pyridazo[1,2 - a][1,2]diazepin - 1(S) - carboxylat und 2,6 g Aethyl 2 - brom - 5 - methylhexanoat 1,5 g (33%) tert.Butyl 9(S) - [1(R) - äthoxycarbonyl - 4 - methylpentylamino] - octahydro - 6,10 - dioxo - 6H - pyridazo[1,2 - a][1,2]diazepin - 1(S) - carboxylat und 1,6 g (35%) tert.Butyl 9(S) - [1(S) - äthoxycarbonyl - 4 - methylpentylamino] - octahydro - 6,10 - dioxo - 6H - pyridazo[1,2 - a][1,2]diazepin - 1(S) - carboxylat, beide in Form leicht gelblicher Oele.

Beispiel 25

In analoger Weise wie in Beispiel 8 beschrieben, erhält man aus 1,0 g tert.Butyl 9(S) - [1(R) - äthoxycarbonyl - 4 - methylpentylamino] - octahydro - 6,10 - dioxo - 6H - pyridazo[1,2 - a][1,2]diazepin - 1(S) - carboxylat 0,4 g (38%) 9(S) - [1(R) - Aethoxycarbonyl - 4 - methylpentylamino] - octahydro - 6,10 - dioxo - 6H - pyridazo[1,2 - a][1,2]diazepin - 1(S) - carbonsäure - hydrobromid als weissen Festkörper vom Schmelzpunkt 220—221° (aus Aethanol/Aethylacetat).

Beispiel 26

In analoger Weise wie in Beispiel 8 beschrieben, erhält man aus 1 g tert.Butyl 9(S) - [1(S) - äthoxycarbonyl - 4 - methylpentylamino] - octahydro - 6,10 - dioxo - 6H - pyridazo[1,2 - a][1,2]diazepin - 1(S) - carboxylat 0,74 g (70%) 9(S) - [1(S) - Aethoxycarbonyl - 4 - methylpentylamino] - octahydro - 6,10 - dioxo - 6H - pyridazo[1,2 - a][1,2]diazepin - 1(S) - carbonsäure - hydrobromid als weissen Festkörper vom Schmelzpunkt 209—210° (aus Aethanol/Aethylacetat).

Beispiel 27

In analoger Weise wie in Beispiel 6 beschrieben, erhält man aus 4,45 g tert.Butyl 9(S) - amino - octahydro - 6,10 - dioxo - 6H - pyridazo[1,2 - a][1,2]diazepin - 1(S) - carboxylat und 7,21 g Aethyl 4 - (4 - chlorphenyl) - 2 - oxo - butanoat 2,13 g (27%) tert.Butyl 9(S) - [3 - (4 - chlorphenyl) - 1(R) - äthoxycarbonylpropylamino] - octahydro - 6,10 - dioxo - 6H - pyridazo[1,2 - a][1,2]diazepin - 1(S) - carboxylat in form weisser Kristalle vom Schmelzpunkt 82—83° (aus Diäthyläther/n-Hexan) und 1,49 g (19%) tert.Butyl 9(S) - [3 - (4 - chlorphenyl) - 1(S) - äthoxycarbonylpropylamino] - octahydro - 6,10 - dioxo - 6H - pyridazo[1,2 - a][1,2]diazepin - 1(S) - carboxylat als weisse Kristalle vom Schmelzpunkt 86—88° (aus Diäthyläther/n-Hexan).

Beispiel 28

In analoger Weise wie in Beispiel 8 beschrieben, erhält man aus 1,28 g tert.Butyl 9(S) - [3 - (4 - chlorphenyl) - 1(S) - äthoxycarbonylpropylamino] - octahydro - 6,10 - dioxo - 6H - pyridazo[1,2 - a][1,2]diazepin - 1(S) - carboxylat 0,78 g (57%) 9(S) - [3 - (4 - Chlorphenyl) - 1(S) - äthoxycarbonylpropylamino] - octahydro - 6,10 - dioxo - 6H - pyridazo[1,2 - a][1,2]diazepin - 1(S) - carbonsäure als weisse Kristalle vom Schmelzpunkt 224—225° (aus Aethanol/Aethylacetat).

Beispiel 29

In analoger Weise wie in Beispiel 6 beschrieben, erhält man aus 4,45 g tert.Butyl 9(S) - amino - octahydro - 6,10 - dioxo - 6H - pyridazo[1,2 - a][1,2]diazepin - 1(S) - carboxylat und 5,66 g Aethyl 4 - (4 - Methoxyphenyl) - 2 - oxo - butanoat 1,21 g (16%) tert.Butyl 9(S) - [1(R) - äthoxycarbonyl - 3 - (4 - methoxy - phenyl)propylamino] - octahydro - 6,10 - dioxo - 6H - pyridazo[1,2 - a][1,2]diazepin - 1(S) - carboxylat als weisse Kristalle vom Schmelzpunkt 73—74° (aus Diäthyläther/n-Hexan) und 0,81 g (10%) tert.Butyl 9(S) - [1(S) - äthoxycarbonyl - 3 - (4 - methoxy - phenyl)propylamino] - octahydro - 6,10 - dioxo - 6H - pyridazo[1,2 - a][1,2]diazepin - 1(S) - carboxylat als weisse Nadeln vom Schmelzpunkt 100—101,5° (aus Diäthyläther/n-Hexan).

Beispiel 30

In analoger Weise wie in Beispiel 8 beschrieben, erhält man aus 0,71 g tert.Butyl 9(S) - [1(S) - äthoxycarbonyl - 3 - (4 - methoxyphenyl)propylamino] - octahydro - 6,10 - dioxo - 6H - pyridazo[1,2 - a][1,2]-diazepin - 1(S) - carboxylat 0,49 g (66%) 9(S) - [1(S) - Aethoxycarbonyl - 3 - (4 - methoxyphenyl)propyl-

amino] - octahydro - 6,10 - dioxo - 6H - pyridazo[1,2 - a][1,2]diazepin - 1(S) - carbonsäure - hydrobromid als weissen Festkörper vom Schmelzpunkt 123—127° (aus Aethanol/Aethylacetat).

Beispiel 31

In analoger Weise wie in Beispiel 6 beschrieben, erhält man aus 4,45 g tert.Butyl 9(S) - amino - octahydro - 6,10 - dioxo - 6H - pyridazo[1,2 - a][1,2]diazepin - 1(S) - carboxylat und 8,46 g Aethyl 4 - biphenylyl - 2 - oxo - butanoat 1,83 g (22%) tert.Butyl 9(S) - [3 - (4 - biphenylyl) - (R) - äthoxycarbonyl - propylamino] - octahydro - 6,10 - dioxo - 6H - pyridazo[1,2 - a][1,2]diazepin - 1(S) - carboxylat als weisse Kristalle vom Schmelzpunkt 136—139° (aus Diäthyläther) und 1,56 g (18%) tert.Butyl 9(S) - [3 - (4 - biphenylyl - 1(S) - äthoxycarbonylpropylamino] - octahydro - 6,10 - dioxo - 6H - pyridazo[1,2 - a][1,2]-diazepin - 1(S) - carboxylat als weisse Kristalle vom Schmelzpunkt 101—103° (aus Diäthyläther).

Beispiel 32

In analoger Weise wie in Beispiel 8 beschrieben, erhält man aus 1,36 g tert.Butyl 9(S) - [3 - (4 - biphenylyl) - 1(S) - äthoxycarbonylpropylamino] - octahydro - 6,10 - dioxo - 6H - pyridazo[1,2 - a][1,2]diazepin - 1(S) - carboxylat 0,63 g (45%) 9(S) - [3 - (4 - Biphenylyl - 1(S) - äthoxycarbonylpropylamino] - octahydro - 6,10 - dioxo - 6H - pyridazo[1,2 - a][1,2]diazepin - 1(S) - carbonsäure - hydrobromid als hygroskopischen weissen Festkörper.

Beispiel 33

In analoger Weise wie in Beispiel 5 beschrieben, erhält man aus 1,78 g tert.Butyl 9(S) - amino - octahydro - 6,10 - dioxo - 6H - pyridazo[1,2 - a][1,2]diazepin - 1(S) - carboxylat und 2,62 g Aethyl 2(R,S) - trifluoromethansulfonyloxy - 6 - phthalimidohexanoat 1,62 g (46%) tert.Butyl 9(S) - [1(R) - äthoxycarbonyl - 5 - phthalimidopentylamino] - octahydro - 6,10 - dioxo - 6H - pyridazo[1,2 - a][1,2]diazepin - 1(S) - carboxylat und 1,58 g (45%) tert.Butyl 9(S) - [1(S) - äthoxycarbonyl - 5 - phthalimidopentylamino] - octahydro - 6,10 - dioxo - 6H - pyridazo[1.2 - a][1,2]diazepin - 1(S) - carboxylat, beide in Form leicht gelblicher Oele.

Beispiel 34

In analoger Weise wie in Beispiel 8 beschrieben, erhält man aus 1,9 g tert.Butyl 9(S) - [1(R) - äthoxycarbonyl - 5 - phthalimidopentylamino] - octahydro - 6,10 - dioxo - 6H - pyridazo[1,2 - a][1,2]diazepin - 1(S) - carboxylat 1,36 g (69%) 9(S) - [1(R) - Aethoxycarbonyl - 5 - phthalimidopentylamino] - octahydro - 6,10 - dioxo - 6H - pyridazo[1,2 - a][1,2]diazepin - 1(S) - carbonsäure - hydrobromid als weissen Festkörper vom Schmelzpunkt 207—208° (aus Aethanol/Aethylacetat).

Beispiel 35

In analoger Weise wie in Beispiel 8 beschrieben, erhält man aus 1,79 g tert.Butyl 9(S) - [1(S) - äthoxycarbonyl - 5 - phthalimidopentylamino] - octahydro - 6,10 - dioxo - 6H - pyridazo[1,2 - a][1,2]diazepin - 1(S) - carboxylat 1,36 g (74%) 9(S) - [1(S) - Aethoxycarbonyl - 5 - phthalimidopentylamino] - octahydro - 6,10 - dioxo - 6H - pyridazo[1,2 - a][1,2]diazepin - 1(S) - carbonsäure - hydrobromid als weissen Festkörper vom Schmelzpunkt 227—229° (aus Aethanol/Aethylacetat).

Beispiel 36

Ein Lösung von 1,15 g tert.Butyl 8(S) - amino - 2,3,6,7,8,9 - hexahydro - 5,9 - dioxo - 1H,5H - pyrazolo [1,2 - a][1,2]diazepin - 1(S) - carboxylat und 3,10 g Aethyl 2 - oxo - 4 - phenylbutanoat in 50 ml Aethanol wird über 1,0 g 10% Palladium auf Kohle in Gegenwart von Molekularsieb bei 4 Atmosphären während 48 Stunden hydriert. Der Katalysator wird abfiltriert, und das Filtrat eingedampft. Der Rückstand wird an Silicagel chromatophiert, wobei man zunächst 0,18 g tert.Butyl 8(S) - [1(R) - äthoxycarbonyl - 3 - phenylpropylamino] - 2,3,6,7,8,9 - hexahydro - 5,9 - dioxo - 1H,5H - pyrazolo [1,2 - a][1,2]diazepin - 1(S) - carboxylat als leicht gelbliches Oel und danach 0,19 g tert.Butyl 8(S) - [1(S) - äthoxycarbonyl - 3 - phenylpropylamino] - 2,3,6,7,8,9 - hexahydro - 5,9 - dioxo - 1H,5H - pyrazolo [1,2 - a][1,2]diazepin - 1(S) - carboxylat als leicht gelbliches Oel erhält.

Das als Ausgangsmaterial verwendete tert.Butyl 8(S) - amino - 2,3,6,7,8,9 - hexahydro - 5,9 - dioxo - 1H,5H - pyrazolo [1,2 - a][1,2]diazepin - 1(S) - carboxylat kann wie folgt hergestellt werden:

2,48 g tert.Butyl 2,3,6,7,8,9 - hexahydro - 5,9 - dioxo - 8(S) - phthalamido - 1H,5H - pyrazolo [1,2 - a][1,2]diazepin - 1(S) - carboxylat [hergestellt wie in Beispiel 3D) beschrieben] werden bei Raumtemperatur während einer halben Stunde in 80 ml Aethanol mit 0,3 g Hydrazinhydrat gerührt. Danach dampft man das Reaktionsgemisch ein, gibt 90 ml 2N Essigsäure zu, rührt das Gemisch während einer Stunde bei Raumtemperatur und filtriert. Das Filtrat wird mit festem Natriumcarbonat basisch gestellt und danach zweimal mit Methylenchlorid extrahiert. Die vereinigten Methylenchloridextrakte werden über Magnesiumsulfat getrocknet und eingedampft, wobei man 1,58 g (93%) tert.Butyl 8(S) - amino - 2,3,6,7,8,9 - hexahydro - 5,9 - dioxo - 1H,5H - pyrazolo [1,2 - a][1,2]diazepin - 1(S) - carboxylat als Gummi erhält.

Beispiel 37

Eine Lösung von 0,15 g tert.Butyl 8(S) - [1(S] - äthoxycarbonyl - 3 - phenylpropylamino] - 2,3,6,7,8,9 - hexahydro - 5,9 - dioxo - 1H,5H - pyrazolo [1,2 - a][1,2]diazepin - 1(S) - carboxylat in 0.3 ml

Essisgsäure wird bei Raumtemperatur während einer halben Stunde mit 1,3 ml einer 45%-igen Lösung von Bromwasserstoff in Essigsäure versetzt. Das Gemisch wird danach eingedampft, und der zurückbleibende ölige Festkörper mit Diäthyläther angerieben, wobei man 0,11 g (65%) 8(S) - [1(S] - Aethoxycarbonyl - 3 - phenylpropylamino] - 2,3,6,7,8,9 - hexahydro - 5,9 - dioxo - 1H,5H - pyrazolo [1,2 - a][1,2]diazepin - 1(S) - carbonsäure - hydrobromid als leicht gelbbraunen gefärbten Festkörper vom Schmelzpunkt 207—210° erhält.

Beispiel 38

1,27 g Benzyl 9(S) - amino - octahydro - 10 - oxo - 6H - pyridazo[1,2 - a][1,2]diazepin - 1(S) - carboxylat, 1,36 g Aethyl 2(R) - trifluoromethansulfonyloxy - 4 - phenylbutanoat und 0,4 g Triäthylamin in 10 ml Acetonitril werden während 16 Stunden bei Raumtemperatur gerührt. Das Lösungsmittel wird abgedampft, und der Rückstand zwischen Wasser und Aethylacetat verteilt. Die organische Lösung wird über Magnesiumsulfat getrocknet und eingedampft. Nach Reinigung durch Chromatographie an Silicagel unter Verwendung von Aethylacetat/n-Hexan (1:1) als Eluierungsmittel erhält man 1,55 g Benzyl 9(S) - [1(S) - äthoxycarbonyl - 3 - phenylpropylamino] - octahydro - 10 - oxo - 6H - pyridazo[1,2 - a][1,2]-diazepin - 1(S) - carboxylat als farbloses Oel.

Das als Ausgangsmaterial eingesetzte Benzyl 9(S) - amino - octahydro - 10 - oxo - 6H - pyridazo-[1,2 - a][1,2]diazepin - 1(S) - carboxylat kann wie folgt hergestellt werden:

A) 2 g α(S) - (3 - Brompropyl) - 1,3 - dioxo - isoindolinessigsäure in 15 ml trockenem Diäthyläther werden bei Raumtemperatur während 1½ Stunden mit 1,34 g Phosphorpentachlorid gerührt. Das Lösungsmittel wird abgedampft und der Rückstand zweimal in Toluol aufgenommen und wieder eingedampft und danach in 25 ml Methylenchlorid gelöst. Zu dieser Lösung werden unter Rühren 1,64 g tert.-Butyl 1-benzyloxycarbonylhexahydro - 3 - pyridazincarboxylat in 10 ml Methylenchlorid und danach 25 ml gesättigte wässrige Natriumbicarbonatlösung gegeben. Das Reaktionsgemisch wird 3 Stunden bei Raumtemperatur gerührt, und danach werden die Phasen getrennt. Die organische Phase wird mit Natriumchloridlösung gewaschen, über Magnesiumsulfat getrocknet und eingedampft. Nach Reinigung durch Chromatographie an Silicagel unter Verwendung von Toluol/Acetonitril (4:1) als Eluierungsmittel erhält man 2,4 g (75%) 1-Benzyl 3-tert.butyl 2 - (5 - brom - 2 - phthalimidovaleryl) - 1,3 - pyridazindicarboxylat (2 Diastereomere) als Oel.

B) 2,37 g 1 - Benzyl 3 - tert.Butyl 2 - (5 - brom - 2 - phthalimidovaleryl) - 1,3 - pyridazindicarboxylat (2 Diastereomere) werden in 25 ml Aethanol gelöst, und die Lösung wird bei Atmosphärendruck über 50 mg 10% Palladium auf Kohle solange hydriert, bis die Wasserstoffaufnahme beendet ist. Der Katalysator wird abfiltriert, und das Filtrat eingedampft, wobei man 1,79 g (96%) tert.Butyl 2 - (5 - brom - 2 - phthalimidovaleryl) - 3 - pyridazincarboxylat (2 Diastereomere) als Oel erhält.

C) 15 g tert.Butyl 2 - (5 - brom - 2 - phthalimidovaleryl) - 3 - pyridazincarboxylat (2 Diastereomere) werden in 200 ml trockenem Dimethylformamid gelöst, und die Lösung bei 80° während 60 Stunden gerührt. Das Lösungsmittel wird danach abgedampft, und der Rückstand zwischen Wasser und Methylenchlorid verteilt. Die oranische Phase wird über Magnesiumsulfat getrocknet und eingedampft. Nach Reinigung des Rückstands durch Chromatographie an Silicagel unter Verwendung von Toluol/Essigsäure (4:1) als Eluierungsmittel erhält man 3,6 g (33%) Octahydro - 10 - oxo - 9(S) - phthalimido - 6H - pyridazo[1,2 - a][1,2]diazepin - 1(S) - carbonsäure als weisse Kristalle vom Schmelzpunkt 256—258° (aus Aethylacetat/n-Hexan) und 1,4 g (13%) Octahydro - 10 - oxo - 9(S) - phthalimido - 6H - pyridazo-[1,2 - a][1,2]diazepin - 1(R) - carbonsäure als weisse Kristalle vom Schmelzpunkt 241—244° (aus Aethylacetat/n-Hexan).

D) 3,57 g Octahydro - 10 - oxo - 9(S) - phthalimido - 6H - pyridazo[1,2 - a][1,2]diazepin - 1(S) - carbonsäure werden in 25 ml 0,4M äthanolischer Natriumhydroxidlösung gelöst und 0,5 g Hydrazinhydrat in 25 ml Aethanol zugebenen. Das Gemisch wird bei Raumtemperatur während 4 Stunden gerührt und danach eingedampft. 80 ml 2N Essigsäure werden zugegeben, das Gemisch bei Raumtemperatur 3 Stunden gerührt und danach filtriert. Das Filtrat wird eingedampft und der Rückstand auf eine Kolonne von 40 g Duolit C225 Ionenaustauscherharz ($H^+$-Form) gegeben. Die Kolonne wird mit 2% Pyridin enthaltendem Wasser eluiert, und die Eluate eingedampft, wobei man 1,7 g (75%) 9(S) - amino - octahydro - 10 - oxo - 6H - pyridazo[1,2 - a][1,2]diazepin - 1(S) - carbonsäure als weissen Festkörper erhält. Nach Kristallisation aus Wasser/Acetonitril schmilzt die Säure bei 247—249° (Zers.); $[\alpha]_D^{20} = -121,9°$ (c = 0,675% in Wasser).

E) 1,6 g 9(S) - amino - octahydro - 10 - oxo - 6H - pyridazo[1,2 - a][1,2]diazepin - 1(S) - carbonsäure in 5 ml Methanol werden mit einer Lösung von Phenyldiazomethan in Diäthyläther bis zur vollständigen Umsetzung versetzt. Die Lösungsmittel werden abgedampft, und der Rückstand zwischen Methylenchlorid und 2N Salzsäure verteilt. Die wässrige Lösung wird mit Kaliumcarbonat basisch gestellt und mit Methylenchlorid extrahiert. Die organische Lösung wird mit Natriumchloridlösung gewaschen, über Magnesiumsulfat getrocknet und eingedampft, wobei man 1,36 g rohes Benzyl 9(S) - amino - octahydro - 10 - oxo - 6H - pyridazo[1,2 - a][1,2]diazepin - 1(S) - carboxylat als Oel erhält, welches ohne weitere Reinigung verwendet wird.

Beispiel 39

1,35 g Benzyl 9(S) - [1(S) - Aethoxycarbonyl - 3 - phenylpropylamino] - octahydro - 10 - oxo - 6H - pyridazo[1,2 - a][1,2]diazepin - 1(S) - carboxylat werden in 20 ml Aethanol gelöst, und die Lösung bei

Atmosphärendruck über 50 mg 10% Palladium auf Kohle bis zur beendeten Wasserstoffaufnahme hydriert. Der Katalysator wird abfiltriert, und das Filtrat eingedampft, wobei man 1,02 g 9(S) - [1(S) - Aethoxycarbonyl - 3 - phenylpropylamino] - octahydro - 10 - oxo - 6H - pyridazo[1,2 - a][1,2]diazepin - 1(S) - carbonsäure als farbloses Oel erhält. Dieses Oel wird in 10 ml Aethylacetat gelöst und 2 ml 2,5N Chlorwasserstoff in Aethylacetat zugebenen. 100 ml Diäthyläther werden danach zugegeben, und das gemisch für eine Stunde gerührt. Der erhaltene Festkörper wird abfiltriert, wobei man 0,72 g (60%) 9(S) - [1(S) - Aethoxycarbonyl - 3 - phenylpropylamino] - octahydro - 10 - oxo - 6H - pyridazo[1,2 - a][1,2]-diazepin - 1(S) - carbonsäure - hydrochlorid als hygroskopischen Festkörper erhält; $[\alpha]_D^{20} = -69,1°$ (c = 1% in Wasser).

Beispiel 40

In analoger Weise wie in Beispiel 4 beschrieben, erhält man aus 1,10 g tert.-Butyl 9(S) - amino - octahydro - 6,10 - dioxo - 6H - pyridazo[1,2 - a][1,2]diazepin - 1(S) - carboxylat, 1,40 g Aethyl 2 - brom - 6 - benzyloxyformamidohexanoat und 0,37 g Triäthylamin 0,4 g tert.Butyl 9(S) - [5 - benzyloxyformamido - 1(R) - äthoxycarbonylpentylamino] - octahydro - 6,10 - dioxo - 6H - pyridazo[1,2 - a][1,2]diazepin - 1(S) - carboxylat als leicht gelbliches Oel und 0,33 g tert.Butyl 9(S) - [5 - benzyloxyformamido - 1(S) - äthoxycarbonylpentylamino] - octahydro - 6,10 - dioxo - 6H - pyridazo[1,2 - a][1,2]diazepin - 1(S) - carboxylat als gelbliches Oel.

Beispiel 41

Durch Umsetzen von tert.Butyl 9(S) - [5 - benzyloxyformamido - 1(S) - äthoxycarbonylpentylamino] - octahydro - 6,10 - dioxo - 6H - pyridazo[1,2 - a][1,2]diazepin - 1(S) - carboxylat mit Trifluoressigsäure erhält man 9(S) - [5 - Benzyloxyformamido - 1(S) - äthoxycarbonylpentylamino] - octahydro - 6,10 - dioxo - 6H - pyridazo[1,2 - a][1,2]diazepin - 1(S) - carbonsäure.

Die folgenden Beispiele veranschaulichen pharmazeutische Kompositionen, enthaltend eine erfindungsgemässe Verbindung:

Beispiel A

Tabletten enthaltend die folgenden Bestandteile können in herklömmlicher Weise hergestellt werden:

| Bestandteile | pro Tablette |
|---|---|
| Verbindung der Formel I | 10,0 mg |
| Lactose | 125,0 mg |
| Maisstärke | 75,0 mg |
| Talk | 4,0 mg |
| Magnesiumstearat | 1,0 mg |
| Totalgewicht | 125,0 mg |

Beispiel B

Kapseln enthaltend die folgenden Bestandteile können in herklömmlicher Weise hergestellt werden:

| Bestandteile | pro Kapsel |
|---|---|
| Verbindung der Formel I | 25,0 mg |
| Lactose | 150,0 mg |
| Maisstärke | 20,0 mg |
| Talk | 5,0 mg |
| Totalgewicht | 200,0 mg |

**Patentansprüche für die Vertragsstaaten: BE CH DE FR GB IT LI LU NL SE**

1. Verbindungen der allgemeinen Formel

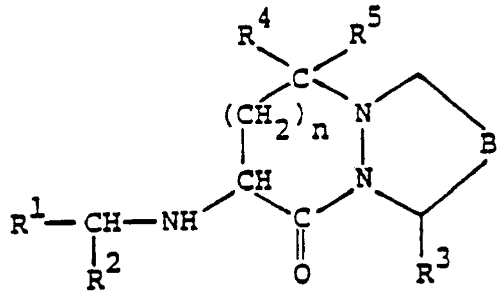

I

worin B Methylen ($-CH_2-$), Aethylen ($-CH_2-CH_2-$) oder Vinylen ($-CH=CH-$), $R^1$ Wasserstoff, Alkyl, Aralkyl, Aminoalkyl, Monoalkylaminoalkyl, Dialkylaminoalkyl, Aralkylcarbonylaminoalkyl, Phthalimidoalkyl, Alkoxycarbonylaminoalkyl, Aryloxycarbonylaminoalkyl, Aralkoxycarbonylaminoalkyl, Alkylaminocarbonylaminoalkyl, Arylaminocarbonylaminoalkyl, Aralkylaminocarbonylaminoalkyl, Alkylsulfonylaminoalkyl oder Arylsulfonylaminoalkyl, $R^2$ Carboxyl, Alkoxycarbonyl oder Aralkoxycarbonyl oder eine Gruppe der Formel

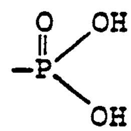

(i)

oder

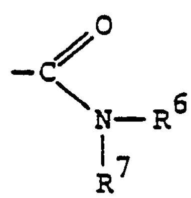

(ii)

$R^3$ Carboxyl, Alkoxycarbonyl oder Aralkoxycarbonyl, $R^4$ und $R^5$ je Wasserstoff oder zusammen eine Oxogruppe, $R^6$ und $R^7$ je Wasserstoff, Alkyl oder Aralkyl und n die Zahl 2 bedeuten, wobei "Aryl" gegebenenfalls durch Halogen, Alkyl, Alkoxy, Trifluormethyl oder Phenyl ein- oder mehrfach substituiertes Phenyl und "Alkyl" und "Alkoxy" — allein oder in Kombination — Reste mit 1—8 Kohlenstoffatomen bedeuten, und pharmazeutisch verwendbare Salze davon.

2. Verbindung gemäss Anspruch 1, worin $R^1$ Wasserstoff, Alkyl, Aralkyl, Aminoalkyl, Monoalkylaminoalkyl, Dialkylaminoalkyl, Aralkylcarbonylaminoalkyl, Alkoxycarbonylaminoalkyl, Aryloxycarbonylaminoalkyl, Aralkoxycarbonylaminoalkyl, Alkylaminocarbonylaminoalkyl, Arylaminocarbonylaminoalkyl, Aralkylaminocarbonylaminoalkyl, Alkylsulfonylaminoalkyl oder Arylsulfonylaminoalkyl, $R^2$ Carboxyl oder Alkoxycarbonyl oder eine Gruppe der Formel (i) und $R^3$ Carboxyl oder Alkoxycarbonyl bedeuten.

3. Verbindungen gemäss Anspruch 1, worin B Methyl oder Aethylen bedeutet.

4. Verbindungen gemäss Anspruch 1, oder 3, worin $R^1$ Alkyl, Aralkyl, Aralkylcarbylaminoalkyl, Phthalimidoalkyl, Aralkoxycarbonylaminoalkyl oder Arylaminocarbonylaminoalkyl bedeutet.

5. Verbindungen gemäss Anspruch 1, 3 oder 4, worin $R^2$ Carboxyl, Alkoxycarbonyl, Aralkoxycarbonyl oder die Gruppe der Formel (ii) bedeutet.

6. Verbindungen gemäss Anspruch 1 oder einem der Ansprüche 3—5, worin $R^3$ Carboxyl bedeutet.

7. Verbindungen gemäss Anspruch 1 oder einem der Ansprüche 3—6, worin B Methylen oder Aethylen, $R^1$ Alkyl, Aralkyl, Aralkylcarbonylaminoalkyl, Phthalimidoalkyl, Aralkoxycarbonylaminoalkyl oder Arylaminocarbonylaminoalkyl, $R^2$ Carboxyl, Alkoxycarbonyl oder Aralkoxycarbonyl oder eine Gruppe der Formel (ii), $R^3$ Carboxyl und n die Zahl 2 bedeuten.

8. 9-(1-Carboxy-3-phenylpropylamino)-octahydro-6,10-dioxo-6H-pyridazo[1,2-a][1,2]diazepin-1-carbonsäure.

9. 9-(1-Aethoxycarbonyl-3-phenylpropylamino)-octahydro-6,10-dioxo-6H-pyridazo[1,2-a][1,2]diazepin-1-carbonsäure.

10. 8-(1-Carboxy-3-phenylpropylamino)-2,3,6,7,8,9-hexahydro-5,9-dioxo-1H,5H-pyrazolo[1,2-a][1,2]diazepin-1-carbonsäure.

11. 9-(1-Benzyloxycarbonyl-3-phenylpropylamino)-octahydro-6,10-dioxo-6H-pyridazo[1,2-a][1,2]diazepin-1-carbonsäure.

12. 9-(1-Carbamoyl-3-phenylpropylamino)-octahydro-6,10-dioxo-6H-pyridazo[1,2-a][1,2]diazepin-1-carbonsäure.

13. 9-(1-Aethylcarbamoyl-3-phenylpropylamino)octahydro-6,10-dioxo-6H-pyridazo[1,2-a][1,2]diazepin-1-carbonsäure.

14. 9-(1-Carboxy-4-phenylbutylamino)-octahydro-6,10-dioxo-6H-pyridazo[1,2-a][1,2]diazepin-1-carbonsäure.

15. 9-(1-Carboxy-2-phenyläthylamino)-octahydro-6,10-dioxo-6H-pyridazo[1,2-a][1,2]diazepin-1-carbonsäure.

16. 9-(1-Carboxy-4-methylpentylamino)-octahydro-6,10-dioxo-6H-pyridazo[1,2-a][1,2]diazepin-1-carbonsäure.

17. 9-(1-Aethoxycarbonyl-4-methylpentylamino)-octahydro-6,10-dioxo-6H-pyridazo[1,2-a][1,2]diazepin-1-carbonsäure.

18. 9-[3-(4-Chlorphenyl)-1-äthoxycarbonylpropylamino]-octahydro-6,10-dioxo-6H-pyridazo[1,2-a][1,2]diazepin-1-carbonsäure.

19. 9-[1-Aethoxycarbonyl-3-(4-methoxyphenyl)propylamino]-octahydro-6,10-dioxo-6H-pyridazo[1,2-a][1,2]diazepin-1-carbonsäure.

20. 9-[3-(4-Biphenylyl)-1-äthoxycarbonylpropylamino]-octahydro-6,10-dioxo-6H-pyridazo[1,2-a][1,2]diazepin-1-carbonsäure.

21. 9-(1-Aethoxycarbonyl-5-phthalimidopentylamino)-octahydro-6,10-dioxo-6H-pyridazo[1,2-a][1,2]diazepin-1-carbonsäure.

22. 8-(1-Aethoxycarbonyl-3-phenylpropylamino)-2,3,6,7,8,9-hexahydro-5,9-dioxo-1H,5H-pyrazolo[1,2-a][1,2]diazepin-1-carbonsäure.

23. 9-(1-Aethoxycarbonyl-3-phenylpropylamino)-octahydro-10-oxo-6H-pyridazo[1,2-a][1,2]diazepin-1-carbonsäure.

24. 9-(5-Benzyloxyformamido-1-äthoxycarbonylpentylamino)-octahydro-6,10-dioxo-6H-pyridazo[1,2-a][1,2]diazepin-1-carbonsäure.

25. tert.Butyl 9-(1-äthoxycarbonyl-3-phenylpropylamino)-octahydro-6,10-dioxo-6H-pyridazo[1,2-a][1,2]diazepin-1-carboxylat,

tert.Butyl 9-(1-benzyloxycarbonyl-3-phenylpropylamino)-octahydro-6,10-dioxo-6H-pyridazo[1,2-a][1,2]diazepin-1-carboxylat,

Methyl 9-(1-benzyloxycarbonyl-3-phenylpropylamino)-octahydro-6,10-dioxo-6H-pyridazo[1,2-a][1,2]diazepin-1-carboxylat,

Methyl 9-(1-carboxy-3-phenylpropylamino)-octahydro-6,10-dioxo-6H-pyridazo[1,2-a][1,2]diazepin-1-carboxylat,

Methyl 9-(1-äthoxycarbonyl-3-phenylpropylamino)-octahydro-6,10-dioxo-6H-pyridazo[1,2-a][1,2]diazepin-1-carboxylat,

Aethyl 9-(1-äthoxycarbonyl-3-phenylpropylamino)-octahydro-6,10-dioxo-6H-pyridazo[1,2-a][1,2]diazepin-1-carboxylat,

tert.Butyl 9-(1-carbamoyl-3-phenylpropylamino)-octahydro-6,10-dioxo-6H-pyridazo[1,2-a][1,2]diazepin-1-carboxylat,

tert.Butyl 9-(1-äthylcarbamoyl-3-phenylpropylamino)-octahydro-6,10-dioxo-6H-pyridazo[1,2-a][1,2]diazepin-1-carboxylat,

tert.Butyl 9-(1-äthoxycarbonyl-4-methylpentylamino)-octahydro-6,10-dioxo-6H-pyridazo[1,2-a][1,2]diazepin-1-carboxylat,

tert.Butyl 9-[3-(4-chlorphenyl)-1-äthoxycarbonylpropylamino]-octahydro-6,10-dioxo-6H-pyridazo[1,2-a][1,2]diazepin-1-carboxylat,

tert.Butyl 9-[1-äthoxycarbonyl-3-(4-methoxyphenyl)propylamino]-octahydro-6,10-dioxo-6H-pyridazo[1,2-a][1,2]diazepin-1-carboxylat,

tert.Butyl 9-[3-(4-biphenylyl)-1-äthoxycarbonylpropylamino]-octahydro-6,10-dioxo-6H-pyridazo[1,2-a][1,2]diazepin-1-carboxylat,

tert.Butyl 9-(1-äthoxycarbonyl-5-phthalimidopentylamino)-octahydro-6,10-dioxo-6H-pyridazo[1,2-a][1,2]diazepin-1-carboxylat,

tert.Butyl 8-(1-äthoxycarbonyl-3-phenylpropylamino)-2,3,6,7,8,9-hexahydro-5,9-dioxo-1H,5H-pyrazolo[1,2-a][1,2]diazepin-1-carboxylat,

Benzyl 9-(1-äthoxycarbonyl-3-phenylpropylamino)-octahydro-10-oxo-6H-pyridazo[1,2-a][1,2]diazepin-1-carboxylat und

tert.Butyl 9-(5-benzyloxyformamido-1-äthoxycarbonylpentylamino)-octahydro-6,10-dioxo-6H-pyridazo[1,2-a][1,2]diazepin-1-carboxylat.

26. Eine Verbindung gemäss einem der Ansprüche 1—25, worin die Konfiguration an jedem asymmetrischen Kohlenstoffatom die S-Konfiguration ist.

27. Eine Verbindung der allgemeinen Formel

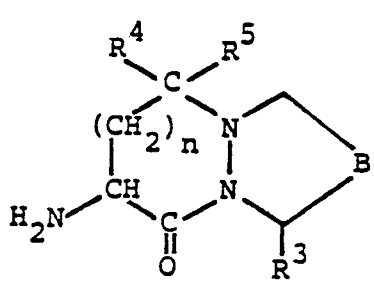

IV

worin B Methylen, Aethylen oder Vinylen, $R^3$ Carboxyl, Alkoxycarbonyl oder Aralkoxycarbonyl, $R^4$ und $R^5$ je Wasserstoff oder $R^4$ und $R^5$ zusammen eine Oxogruppe und n die Zahl Z bedeuten, wobei "Aryl" gegebenenfalls durch Halogen, Alkyl, Alkoxy, Trifluormethyl oder Phenyl ein- oder mehrfach substituiertes Phenyl bedeutet und "Alkyl" und "Akoxy" 1—8 Kohlenstoffatome aufweisen.

28. Eine Verbindung der allgemeinen Formel

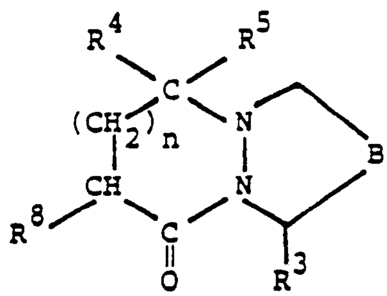

XVIIa

worin B Methylen, Aethylen oder Vinylen, $R^3$ Carboxyl, Alkoxycarbonyl oder Aralkoxycarbonyl, $R^4$ und $R^5$ je Wasserstoff oder $R^4$ und $R^5$ zusammen eine Oxogruppe, $R^8$ Phthaloylamino und n die Zahl 2 bedeuten, wobei "Aryl" gegebenenfalls durch Halogen, Alkyl, Alkoxy, Trifluormethyl oder Phenyl ein- oder mehrfach substituiertes Phenyl bedeutet und "Alkyl" und "Alkoxy" 1—8 Kohlenstoffatome aufweisen.

29. Eine Verbindung gemäss einem der Ansprüche 1—26 oder ein pharmazeutisch verwendbares Salz davon als pharmazeutischer Wirkstoff.

30. Eine Verbindung gemäss einem der Ansprüche 1—26 oder ein pharmazeutisch verwendbares Salz davon als Antihypertensivum.

31. Verfahren zur Herstellung von Verbindungen der in Anspruch 1 angegebenen Formel I und von pharmazeutisch verwendbaren Salzen davon, dadurch gekennzeichnet, dass man

a) eine Verbindung der allgemeinen Formel

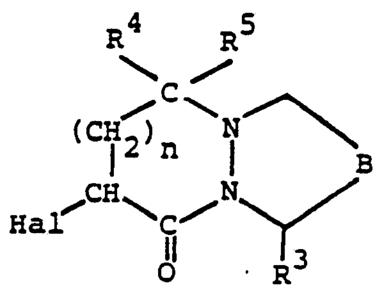

II

worin B, $R^3$, $R^4$, $R^5$ und n die in Anspruch 1 angegebene Bedeutung besitzen und Hal Halogen bedeutet, mit einer Verbindung der allgemeinen Formel

$$R^1—CH—NH_2$$
$$|$$
$$R^2$$

III

worin $R^1$ und $R^2$ die in Anspruch 1 angegebene Bedeutung besitzen, umsetzt, oder

b) eine Verbindung der allgemeinen Formel

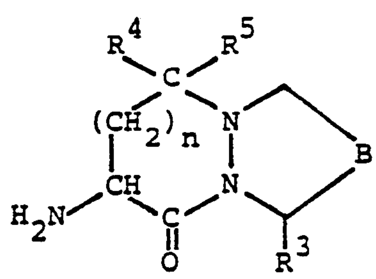

IV

worin B, $R^3$, $R^4$, $R^5$ und n die in Anspruch 1 angegebene Bedeutung besitzen, mit einer Verbindung der allgemeinen Formel

$$R^1—C=O \quad (\text{mit } R^2 \text{ an C}) \qquad \text{V}$$

worin $R^1$ und $R^2$ die in Anspruch 1 angegebene Bedeutung besitzen, reduktive alkyliert, oder

c) zur Herstellung von Verbindungen der Formel I, worin $R^2$ Alkoxycarbonyl oder Aralkoxycarbonyl oder eine Gruppe der Formel (ii) und $R^3$ Alkoxycarbonyl oder Aralkoxycarbonyl bedeuten, eine Verbindung der obigen Formel IV, worin $R^3$ Alkoxycarbonyl oder Aralkoxycarbonyl bedeutet, mit einer Verbindung der allgemeinen Formel

$$R^1—CH—Q \quad (\text{mit } R^{20} \text{ an CH}) \qquad \text{VI}$$

worin $R^1$ die in Anspruch 1 angegebene Bedeutung besitzt, $R^{20}$ Alkoxycarbonyl oder Aralkoxycarbonyl oder eine Gruppe der Formel (ii) und Q eine Abgangsgruppe bedeutet, umsetzt, oder

d) zur Herstellung von Verbindungen der Formel I, worin $R^2$ eine Gruppe der Formel (ii) und $R^3$ Carboxyl oder tert. Butoxycarbonyl bedeuten, eine Verbindung der Formel I, worin $R^2$ Alkoxycarbonyl und $R^3$ Carboxyl oder tert. Butoxycarbonyl bedeuten, mit einer Verbindung der allgemeinen Formel

$$HN(R^6)(R^7) \qquad \text{VII}$$

worin $R^6$ und $R^7$ die in Anspruch Bedeutung besitzen, umsetzt, oder

e) zur Herstellung von Verbindungen der Formel I, worin B Aethylen bedeutet, eine Verbindung der Formel I, worin B Vinylen bedeutet, katalytisch hydriert, oder

f) zur Herstellung von Verbindungen der Formel I, worin $R^2$ und/oder $R^3$ Alkoxycarbonyl oder Aralkoxycarbonyl bedeuten, eine Verbindung der Formel I, worin $R^2$ und/oder $R^3$ Carboxyl bedeuten, verestert, oder

g) zur Herstellung von Verbindungen der Formel I, worin $R^2$ und/oder $R^3$ Carboxyl bedeuten, eine Verbindung der Formel I, worin $R^2$ und/oder $R^3$ Alkoxycarbonyl bedeuten, mit einer Säure oder einer Base behandelt, oder

h) zur Herstellung von Verbindungen der Formel I, worin B Methylen oder Aethylen und $R^2$ und/oder $R^3$ Carboxyl bedeuten, eine Verbindung der Formel I, worin $R^2$ und/oder $R^3$ Aralkoxycarbonyl bedeuten, hydriert, oder

i) zur Herstellung von Verbindungen der Formel I, worin $R^1$ Aminoalkyl bedeutet, in einer Verbindung der Formel I, worin $R^1$ Alkoxycarbonylaminoalkyl, Aryloxycarbonylaminoalkyl oder Aralkoxycarbonylaminoalkyl bedeutet, die Alkoxycarbonyl-, Aryloxycarbonyl- oder Aralkoxycarbonylgruppe abspaltet und

j) erwünschtenfalls ein erhaltenes Gemisch von Diastereomeren in die entsprechenden diastereomeren Racemate oder optisch reinen Diastereomeren auftrennt und/oder

k) erwünschtenfalls ein erhaltenes Racemat in die beiden Antipoden auftrennt und

l) erwünschtenfalls eine erhaltene Verbindung der Formel I in ein pharmazeutisch verwendbares Salz überführt.

32. Verfahren gemäss Anspruch 31, dadurch gekennzeichnet, dass man eine Verbindung der Formel I, worin $R^1$ Wasserstoff, Alkyl, Aralkyl, Aminoalkyl, Monoalkylaminoalkyl, Dialkylaminoalkyl, Aralkylcarbonylaminoalkyl, Alkoxycarbonylaminoalkyl, Aryloxycarbonylaminoalkyl, Aralkoxycarbonylaminoalkyl, Alkylaminocarbonylaminoalkyl, Arylaminocarbonylaminoalkyl, Aralkylaminocarbonylaminoalkyl, Alkylsulfonylaminoalkyl oder Arylsulfonylaminoalkyl, $R^2$ Carboxyl oder Alkoxycarbonyl oder eine Gruppe der Formel (i) und $R^3$ Carboxyl oder Alkoxycarbonyl bedeuten, oder ein pharmazeutisch verwendbares Salz davon nach Verfahrensvarianten a), b), e), f), g), i), j), k) und/oder l) herstellt.

33. Arzneimittel, enthaltend eine Verbindung gemäss einem der Ansprüche 1—26 oder ein pharmazeutisch verwendbares Salz davon.

34. Antihypertensivum, enthaltend eine Verbindung gemäss einem der Ansprüche 1—26 oder ein pharmazeutisch verwendbares Salz davon.

35. Verwendung einer Verbindung gemäß einem der Ansprüche 1—26 oder eines pharmazeutisch verwendbaren Salzes davon zur Herstellung eines Antihypertensivums.

36. Verwendung von 9(S) - [1(S) - Äthoxycarbonyl - 3 - phenylpropylamino] - octahydro - 10 - oxo - 6H - pyridazo[1,2 - a][1,2]diazepin - 1(S) - carbonsäure zur Herstellung eines Antihypertensivums.

**Patentansprüche für den Vertragsstaat: AT**

1. Verfahren zur Herstellung von Verbindungen der Allgemeinen Formel

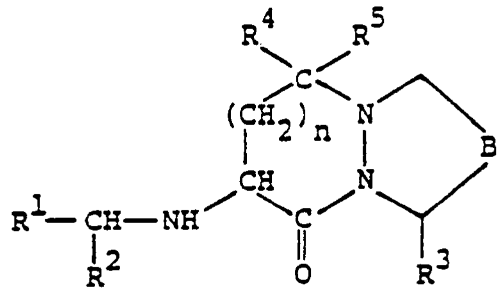

I

worin B Methylen ($—CH_2—$), Aethylen ($—CH_2—CH_2—$) oder Vinylen ($—CH=CH—$), $R^1$ Wasserstoff, Alkyl, Aralkyl, Aminoalkyl, Monoalkylaminoalkyl, Dialkylaminoalkyl, Aralkylcarbonylaminoalkyl, Phthalimidoalkyl, Alkoxycarbonylaminoalkyl, Aryloxycarbonylaminoalkyl, Aralkoxycarbonylaminoalkyl, Alkylaminocarbonylaminoalkyl, Arylaminocarbonylaminoalkyl, Aralkylaminocarbonylaminoalkyl, Alkylsulfonylaminoalkyl oder Arylsulfonylaminoalkyl, $R^2$ Carboxyl, Alkoxycarbonyl oder Aralkoxycarbonyl oder eine Gruppe der Formel

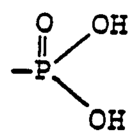

(i)

oder

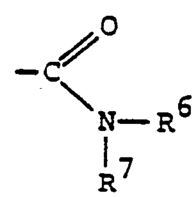

(ii)

$R^3$ Carboxyl, Alkoxycarbonyl oder Aralkoxycarbonyl, $R^4$ und $R^5$ je Wasserstoff oder zusammen eine Oxogruppe, $R^6$ und $R^7$ je Wasserstoff, Alkyl oder Aralkyl und n die Zahl 2 bedeuten, wobei "Aryl" gegebenenfalls durch Halogen, Alkyl, Alkoxy, Trifluormethyl oder Phenyl ein- oder mehrfach substituiertes Phenyl und "Alkyl" und "Alkoxy" — allein oder in Kombination — Reste mit 1—8 Kohlenstoffatomen bedeuten, und pharmazeutisch verwendbaren Salzen davon, dadurch gekennzeichnet, dass man

a) eine Verbindung der allgemeinen Formel

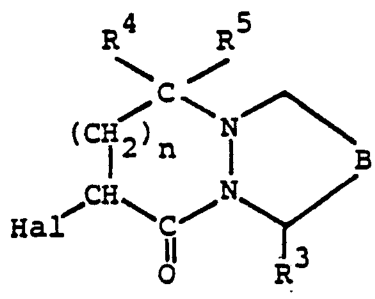

II

worin B, $R^3$, $R^4$, $R^5$ und n die obige Bedeutung besitzen und Hal Halogen bedeutet, mit einer Verbindung der allgemeinen Formel

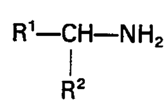

III

worin $R^1$ und $R^2$ die obige Bedeutung besitzen, umsetzt, oder

b) eine Verbindung der allgemeinen Formel

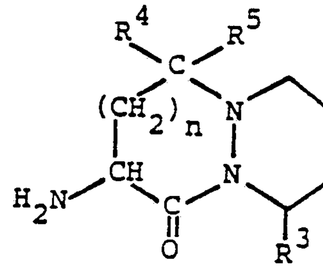

IV

worin B, $R^3$, $R^4$, $R^5$ und n die obige Bedeutung besitzen, mit einer Verbindung der allgemeinen Formel

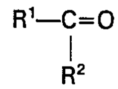

V

worin $R^1$ und $R^2$ die in obige Bedeutung besitzen, reduktive alkyliert, oder

c) zur Herstellung von Verbindungen der Formel I, worin $R^2$ Alkoxycarbonyl oder Aralkoxycarbonyl oder eine Gruppe der Formel (ii) und $R^3$ Alkoxycarbonyl oder Aralkoxycarbonyl bedeuten, eine Verbindung der obigen Formel IV, worin $R^3$ Alkoxycarbonyl oder Aralkoxycarbonyl bedeutet, mit einer Verbindung der allgemeinen Formel

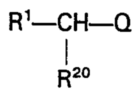

VI

worin $R^1$ die in obige Bedeutung besitzt, $R^{20}$ Alkoxycarbonyl oder Aralkoxycarbonyl oder eine Gruppe der Formel (ii) und Q eine Abgangsgruppe bedeutet, umsetzt, oder

d) zur Herstellung von Verbindungen der Formel I, worin $R^2$ eine Gruppe der Formel (ii) und $R^3$ Carboxyl oder tert. Butoxycarbonyl bedeuten, eine Verbindung der Formel I, worin $R^2$ Alkoxycarbonyl und $R^3$ Carboxyl oder tert. Butoxycarbonyl bedeuten, mit einer Verbindung der allgemeinen Formel

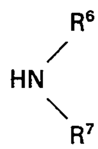

VII

worin $R^6$ und $R^7$ die obige Bedeutung besitzen, umsetzt, oder

e) zur Herstellung von Verbindungen der Formel I, worin B Aethylen bedeutet, eine Verbindung der Formel I, worin B Vinylen bedeutet, katalytisch hydriert, oder

f) zur Herstellung von Verbindungen der Formel I, worin $R^2$ und/oder $R^3$ Alkoxycarbonyl oder Aralkoxycarbonyl bedeuten, eine Verbindung der Formel I, worin $R^2$ und/oder $R^3$ Carboxyl bedeuten, verestert, oder

g) zur Herstellung von Verbindungen der Formel I, worin $R^2$ und/oder $R^3$ Carboxyl bedeuten, eine Verbindung der Formel I, worin $R^2$ und/oder $R^3$ Alkoxycarbonyl bedeuten, mit einer Säure oder einer Base behandelt, oder

h) zur Herstellung von Verbindungen der Formel I, worin B Methylen oder Aethylen und $R^2$ und/oder $R^3$ Carboxyl bedeuten, eine Verbindung der Formel I, worin $R^2$ und/oder $R^3$ Aralkoxycarbonyl bedeuten, hydriert, oder

i) zur Herstellung von Verbindungen der Formel I, worin $R^1$ Aminoalkyl bedeutet, in einer Verbindung der Formel I, worin $R^1$ Alkoxycarbonylaminoalkyl, Aryloxycarbonylaminoalkyl oder Aralkoxycarbonyl-aminoalkyl bedeutet, die Alkoxycarbonyl-, Aryloxycarbonyl- oder Aralkoxycarbonylgruppe abspaltet und

j) erwünschtenfalls ein erhaltenes Gemisch von Diastereomeren in die entsprechenden diastereomeren Racemate oder optisch reinen Diastereomeren auftrennt und/oder

k) erwünschtenfalls ein erhaltenes Racemat in die beiden Antipoden auftrennt und

l) erwünschtenfalls eine erhaltene Verbindung der Formel I in ein pharmazeutisch verwendbares Salz überführt.

2. Verfahren nach Anspruch 1, dadurch gekennzeichnet, dass man eine Verbindung der Formel I, worin $R^1$ Wasserstoff, Alkyl, Aralkyl, Aminoalkyl, Monoalkylaminoalkyl, Dialkylaminoalkyl, Aralkylcarbonylaminoalkyl, Alkoxycarbonylaminoalkyl, Aryloxycarbonylaminoalkyl, Aralkoxycarbonylaminoalkyl, Alkylaminocarbonylaminoalkyl, Arylaminocarbonylaminoalkyl, Aralkylaminocarbonylaminoalkyl, Alkylsulfonylaminoalkyl oder Arylsulfonylaminoalkyl, $R^2$ Carboxyl oder Alkoxycarbonyl oder eine Gruppe der Formel (i) and $R^3$ Carboxyl oder Alkoxycarbonyl bedeuten, oder ein pharmazeutisch annehmbares Salz davon gemäss Verfahrensvarianten a), b), e), f), g), i), j), k), und/oder l) herstellt.

3. Verfahren nach Anspruch 1, worin B Methylen oder Aethylen bedeutet.

4. Verfahren nach Anspruch 1 oder 3, worin $R^1$ Alkyl, Aralkyl, Aralkylcarbonylaminoalkyl, Phthalimidoalkyl, Aralkoxycarbonylaminoalkyl oder Arylaminocarbonylaminoalkyl bedeutet.

5. Verfahren nach Anspruch 1, 3 oder 4, worin $R^2$ Carboxyl, Alkoxycarbonyl oder Aralkoxycarbonyl oder eine Gruppe der Formel (ii) bedeutet.

6. Verfahren nach Anspruch 1 oder einem der Ansprüche 3—5, worin $R^3$ Carboxyl bedeutet.

7. Verfahren nach Anspruch 1 oder einem der Ansprüche 3—6, worin B Methylen oder Aethylen, $R^1$ Alkyl, Aralkyl, Aralkylcarbonylaminoalkyl, Phthalimidoalkyl, Aralkoxycarbonylaminoalkyl oder Arylaminocarbonylaminoalkyl, $R^2$ Carboxyl, Alkoxycarbonyl oder Aralkoxycarbonyl oder eine Gruppe der Formel (ii), $R^3$ Carboxyl und n die Zahl 2 bedeuten.

8. Verfahren nach Anspruch 2, dadurch gekennzeichnet, dass man 9-(1-Carboxy-3-phenylpropylamino)-octahydro-6,10-dioxo-6H-pyridazo[1,2-a][1,2]diazepin-1-carbonsäure herstellt.

9. Verfahren nach Anspruch 2, dadurch gekennzeichnet, dass man 9-(1-Aethoxycarbonyl-3-phenylpropylamino)-octahydro-6,10-dioxo-6H-pyridazo[1,2-a][1,2]diazepin-1-carbonsäure herstellt.

10. Verfahren nach Anspruch 2, dadurch gekennzeichnet, dass man 8-(1-Carboxy-3-phenylpropylamino)-2,3,6,7,8,9-hexahydro-5,9-dioxo-1H,5H-pyrazolo[1,2-a][1,2]diazepin-1-carbonsäure herstellt.

11. Verfahren nach Anspruch 1, dadurch gekennzeichnet, dass man 9-(1-Benzyloxycarbonyl-3-phenylpropylamino)-octahydro-6,10-dioxo-6H-pyridazo[1,2-a][1,2]diazepin-1-carbonsäure herstellt.

12. Verfahren nach Anspruch 1, dadurch gekennzeichnet, dass man 9-(1-Carbamoyl-3-phenylpropylamino)-octahydro-6,10-dioxo-6H-pyridazo[1,2-a][1,2]diazepin-1-carbonsäure herstellt.

13. Verfahren nach Anspruch 1, dadurch gekennzeichnet, dass man 9-(1-Aethylcarbamoyl-3-phenylpropylamino)octahydro-6,10-dioxo-6H-pyridazo[1,2-a][1,2]diazepin-1-carbonsäure herstellt.

14. Verfahren nach Anspruch 1, dadurch gekennzeichnet, dass man 9-(1-Carboxy-4-phenylbutylamino)-octahydro-6,10-dioxo-6H-pyridazo[1,2-a][1,2]diazepin-1-carbonsäure herstellt.

15. Verfahren nach Anspruch 1, dadurch gekennzeichnet, dass man 9-(1-Carboxy-2-phenyläthylamino)-octahydro-6,10-dioxo-6H-pyridazo[1,2-a][1,2]diazepin-1-carbonsäure herstellt.

16. Verfahren nach Anspruch 1, dadurch gekennzeichnet, dass man 9-(1-Carboxy-4-methylpentylamino)-octahydro-6,10-dioxo-6H-pyridazo[1,2-a][1,2]diazepin-1-carbonsäure herstellt.

17. Verfahren nach Anspruch 1, dadurch gekennzeichnet, dass man 9-(1-Aethoxycarbonyl-4-methylpentylamino)-octahydro-6,10-dioxo-6H-pyridazo[1,2-a][1,2]diazepin-1-carbonsäure herstellt.

18. Verfahren nach Anspruch 1, dadurch gekennzeichnet, dass man 9-[3-(4-Chlorphenyl)-1-äthoxycarbonylpropylamino]-octahydro-6,10-dioxo-6H-pyridazo[1,2-a][1,2]diazepin-1-carbonsäure herstellt.

19. Verfahren nach Anspruch 1, dadurch gekennzeichnet, dass man 9-[1-Aethoxycarbonyl-3-(4-methoxyphenyl)propylamino]-octahydro-6,10-dioxo-6H-pyridazo[1,2-a][1,2]diazepin-1-carbonsäure herstellt.

20. Verfahren nach Anspruch 1, dadurch gekennzeichnet, dass man 9-[3-(4-Biphenylyl)-1-äthoxycarbonylpropylamino]-octahydro-6,10-dioxo-6H-pyridazo[1,2-a][1,2]diazepin-1-carbonsäure herstellt.

21. Verfahren nach Anspruch 1, dadurch gekennzeichnet, dass man 9-(1-Aethoxycarbonyl-5-phthalimidopentylamino)-octahydro-6,10-dioxo-6H-pyridazo[1,2-a][1,2]diazepin-1-carbonsäure herstellt.

22. Verfahren nach Anspruch 1, dadurch gekennzeichnet, dass man 8-(1-Aethoxycarbonyl-3-phenylpropylamino)-2,3,6,7,8,9-hexahydro-5,9-dioxo-1H,5H-pyrazolo[1,2-a][1,2]diazepin-1-carbonsäure herstellt.

23. Verfahren nach Anspruch 1, dadurch gekennzeichnet, dass man 9-(1-Aethoxycarbonyl-3-phenylpropylamino)-octahydro-10-oxo-6H-pyridazo[1,2-a][1,2]diazepin-1-carbonsäure herstellt.

24. Verfahren nach Anspruch 1, dadurch gekennzeichnet, dass man 9-(5-Benzyloxyformamido-1-äthoxycarbonylpentylamino)-octahydro-6,10-dioxo-6H-pyridazo[1,2-a][1,2]diazepin-1-carbonsäure herstellt.

25. Verfahren nach einem der Ansprüche 1—24, dadurch gekennzeichnet, dass die Konfiguration an jedem asymmetrischen Kohlenstoffatom die S-Konfiguration ist.

26. Verwendung einer Verbindung erhalten gemäß einem der Ansprüche 1—25 zur Herstellung eines Antihypertensivums.

27. Verwendung von 9(S)-[1(S)-Äthoxycarbonyl-3-phenylpropylamino]-octahydro-10-oxo-6H-pyridazo-[1,2-a][1,2]diazepin-1(S)-carbonsäure zur Herstellung eines Antihypertensivums.

**Revendications pour les Etats contractants: BE CH DE FR GB IT LI LU NL SE**

1. Composés de formule générale

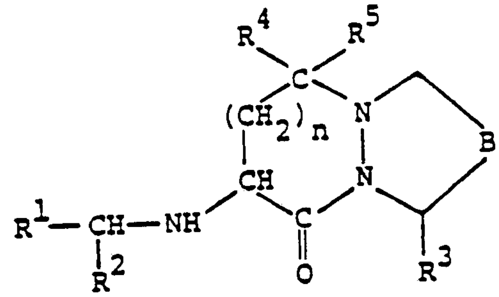

I

où B représente un méthylène ($-CH_2-$), éthylène ($-CH_2-CH_2-$) ou vinylène ($-CH=CH-$), $R^1$ un hydrogène, alcoyle, aralcoyle, aminoalcoyle, monoalcoylaminoalcoyle, dialcoylaminoalcoyle, aralcoylcarbonylaminoalcoyle, phtalimidoalcoyle, alcoxycarbonylaminoalcoyle, aryloxycarbonylaminoalcoyle, aralcoxycarbonylaminoalcoyle, alcoylaminocarbonylaminoalcoyle, arylaminocarbonylaminoalcoyle, aralcoylaminocarbonylaminoalcoyle, alcoylsulfonylaminoalcoyle ou arylsulfonylaminoalcoyle, $R^2$ un carboxyle, alcoxycarbonyle ou aralcoxycarbonyle ou un groupe de formule

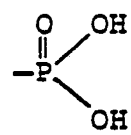

ou

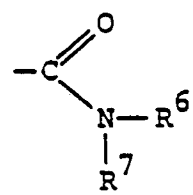

(i) (ii)

$R^3$ représente un carboxyle, alcoxycarbonyle ou aralcoxycarbonyle, $R^4$ et $R^5$ représentent chacun un hydrogène ou ensemble un groupe oxo, $R^6$ et $R^7$ chacun un hydrogène, alcoyle ou aralcoyle et n le nombre 2, où "aryle" représente un phényle éventuellement mono- ou polysubstitué par un halogène, alcoyle, alcoxy, trifluorométhyle ou phényle, et "alcoyle" et "alcoxy" — seuls ou en combinaison — représentent des radicaux en $C_{1-8}$, et leurs sels pharmaceutiquement acceptables.

2. Composés selon la revendication 1, où $R^1$ représente un hydrogène, alcoyle, aralcoyle, aminoalcoyle, monoalcoylaminoalcoyle, dialcoylaminoalcoyle, aralcoylcarbonylaminoalcoyle, alcoxycarbonylaminoalcoyle, aryloxycarbonylaminoalcoyle, aralcoxycarbonylaminoalcoyle, alcoylaminocarbonylaminoalcoyle, arylaminocarbonylaminoalcoyle, aralcoylaminocarbonylaminoalcoyle, alcoylesulfonylaminoalcoyle, ou arylsulfonylaminoalcoyle, $R^2$ représente un carboxyle ou un alcoxycarbonyle ou un groupe de formule (i) et $R^3$ représente un carboxyle ou un alcoxycarbonyle.

3. Composés selon la revendication 1, où B représente un méthylène ou un éthylène.

4. Composés selon la revendication 1 ou 3, où $R^1$ représente un alcoyle, aralcoyle, aralcoylcarbonylaminoalcoyle, phtalimidoalcoyle, aralcoxycarbonylaminoalcoyle ou arylaminocarbonylaminoalcoyle.

5. Composés selon la revendication 1, 3 ou 4 où $R^2$ représente un carboxyle, alcoxycarbonyle, aralcoxycarbonyle ou le groupe de formule (ii).

6. Composés selon la revendication 1, ou l'une des revendications 3—5, où $R^3$ représente un carboxyle.

7. Composés selon la revendication 1, ou l'une des revendications 3—6, où B représente un méthylène ou éthylène, $R^1$ représente un alcoyle, aralcoyle, aralcoylcarbonylaminoalcoyle, phtalimidoalcoyle, aralcoxycarbonylaminoalcoyle ou arylaminocarbonylaminoalcoyle, $R^2$ représente un carboxyle, alcoxycarbonyle ou aralcoxycarbonyle ou un groupe de formule (ii), $R^3$ représente un carboxyle, et n représente le nombre 2.

8. Acide 9-(1-carboxy-3-phénylpropylamino)-octahydro-6,10-dioxo-6H-pyridazo[1,2-a][1,2]diazépine-1-carboxylique.

9. Acide 9-(1-éthoxycarbonyl-3-phénylpropylamino)-octahydro-6,10-dioxo-6H-pyridazo[1,2-a][1,2]diazépine-1-carboxylique.

10. Acide 8-(1-carboxy-3-phénylpropylamino)-2,3,6,7,8,9-hexahydro-5,9-dioxo-1H,5H-pyrazolo[1,2-a][1,2]diazépine-1-carboxylique.

11. Acide 9-(1-benzyloxycarbonyl-3-phénylpropylamino)-octahydro-6,10-dioxo-6H-pyridazo[1,2-a][1,2]diazépine-1-carboxylique.

12. Acide 9-(1-carbamoyl-3-phénylpropylamino)-octahydro-6,10-dioxo-6H-pyridazo[1,2-a][1,2]diazépine-1-carboxylique.

13. Acide 9-(1-éthylcarbamoyl-3-phénylpropylamino)-octahydro-6,10-dioxo-6H-pyridazo[1,2-a][1,2]diazépine-1-carboxylique.

14. Acide 9-(1-carboxy-4-phénylbutylamino)-octahydro-6,10-dioxo-6H-pyridazo[1,2-a][1,2]diazépine-1-carboxylique.

15. Acide 9-(1-carboxy-2-phényléthylamino)-octahydro-6,10-dioxo-6H-pyridazo[1,2-a][1,2]diazépine-1-carboxylique.

16. Acide 9-(1-carboxy-4-méthylpentylamino)-octahydro-6,10-dioxo-6H-pyridazo[1,2-a][1,2]diazépine-1-carboxylique.

17. Acide 9-(1-éthoxycarbonyl-4-méthylpentylamino)-octahydro-6,10-dioxo-6H-pyridazo[1,2-a][1,2]diazépine-1-carboxylique.

18. Acide 9-[3-(4-chlorophényl)-1-éthoxycarbonylpropylamino]-octahydro-6,10-dioxo-6H-pyridazo[1,2-a][1,2]diazépine-1-carboxylique.

19. Acide 9-[1-éthoxycarbonyl-3-(4-méthoxyphényl)propylamino]-octahydro-6,10-dioxo-6H-pyridazo[1,2-a][1,2]diazépine-1-carboxylique.

20. Acide 9-[3-(4-biphénylyl)-1-éthoxycarbonylpropylamino]-octahydro-6,10-dioxo-6H-pyridazo[1,2-a][1,2]diazépine-1-carboxylique.

21. Acide 9-(1-éthoxycarbonyl-5-phtalimidopentylamino)-octahydro-6,10-dioxo-6H-pyridazo[1,2-a][1,2]diazépine-1-carboxylique.

22. Acide 8-(1-éthoxycarbonyl-3-phénylpropylamino)-2,3,6,7,8,9-hexahydro-5,9-dioxo-1H,5H-pyrazolo[1,2-a][1,2]diazépine-1-carboxylique.

23. Acide 9-(1-éthoxycarbonyl-3-phénylpropylamino)-octahydro-10-oxo-6H-pyridazo[1,2-a][1,2]diazépine-1-carboxylique.

24. Acide 9-(5-benzyloxyformamido-1-éthoxycarbonylpentylamino)-octahydro-6,10-dioxo-6H-pyridazo[1,2-a][1,2]diazépine-1-carboxylique.

25. Tert.butyl 9-(1-éthoxycarbonyl-3-phénylpropylamino)-octahydro-6,10-dioxo-6H-pyridazo[1,2-a][1,2]diazépine-1-carboxylate,

tert.butyl 9-(1-benzyloxycarbonyl-3-phénylpropylamino)-octahydro-6,10-dioxo-6H-pyridazo[1,2-a][1,2]diazépine-1-carboxylate,

méthyl-9-(1-benzyloxycarbonyl-3-phénylpropylamino)-octahydro-6,10-dioxo-6H-pyridazo[1,2-a][1,2]diazépine-1-carboxylate,

méthyl-9-(1-carboxy-3-phénylpropylamino)-octahydro-6,10-dioxo-6H-pyridazo[1,2-a][1,2]diazépine-1-carboxylate,

méthyl-9-(1-éthoxycarbonyl-3-phénylpropylamino)-octahydro-6,10-dioxo-6H-pyridazo[1,2-a][1,2]diazépine-1-carboxylate,

éthyl-9-(1-éthoxycarbonyl-3-phénylpropylamino)-octahydro-6,10-dioxo-6H-pyridazo[1,2-a][1,2]diazépine-1-carboxylate,

tert.butyl-9-(1-carbamoyl-3-phénylpropylamino)octahydro-6,10-dioxo-6H-pyridazo[1,2-a][1,2]diazépine-1-carboxylate,

tert.butyl-9-(1-éthylcarbamoyl-3-phénylpropylamino)-octahydro-6,10-dioxo-6H-pyridazo[1,2-a][1,2]diazépine-1-carboxylate,

tert.butyl-9-(1-éthoxycarbonyl-4-méthylpentylamino)-octahydro-6,10-dioxo-6H-pyridazo[1,2-a][1,2]diazépine-1-carboxylate,

tert.butyl-9-[3-(4-chlorophényl)-1-éthoxycarbonyl-propylamino]-octahydro-6,10-dioxo-6H-pyridazo[1,2-a][1,2]diazépine-1-carboxylate,

tert.butyl-9-[1-éthoxycarbonyl-3-(4-méthoxyphényl)-propylamino]-octahydro-6,10-dioxo-6H-pyridazo[1,2-a][1,2]diazépine-1-carboxylate,

tert.butyl-9-[3-(4-biphénylyl)-1-éthoxycarbonylpropylamino]-octahydro-6,10-dioxo-6H-pyridazo[1,2-a][1,2]diazépine-1-carboxylate,

tert.butyl-9-(1-éthoxycarbonyl-5-phtalimidopentylamino)-octahydro-6,10-dioxo-6H-pyridazo[1,2-a][1,2]diazépine-1-carboxylate,

tert.butyl-8-(1-éthoxycarbonyl)-3-phénylpropylamino)-2,3,6,7,8,9-hexahydro-5,9-dioxo-1H,5H-pyrazolo[1,2-a][1,2]diazépine-1-carboxylate,

benzyl-9-(1-éthoxycarbonyl-3-phénylpropylamino)-octahydro-10-oxo-6H-pyridazo[1,2-a][1,2]diazépine-1-carboxylate et

tert.butyl-9-(5-benzyloxyformamido-1-éthoxycarbonylpentylamino)-octahydro-6,10-dioxo-6H-pyridazo[1,2-a][1,2]diazépine-1-carboxylate.

26. Composé selon l'une des revendications 1—25 où la configuration sur chaque atome de carbone asymétrique est la configuration S.

27. Composé de formule générale

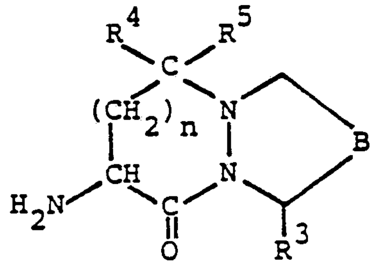

IV

où B représente un méthylène, éthylène ou vinylène, $R^3$ représente un carboxyle, alcoxycarbonyle ou aralcoxycarbonyle, $R^4$ et $R^5$ représentent chacun un hydrogène ou $R^4$ et $R^5$ ensemble un groupe oxo et n le nombre 2, où "aryle" représente un phényle éventuellement mono- ou polysubstitué par un halogène, alcoyle, alcoxy, trifluorométhyle ou phényle et "alcoyle" et "alcoxy" présentent de 1 à 8 atomes de carbone.

28. Composé de formule générale

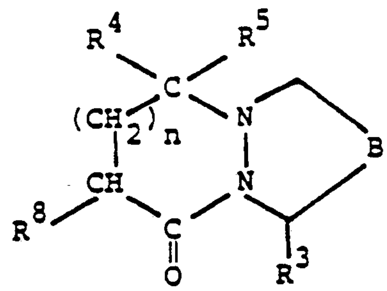

XVIIa

où B représente un méthylène, éthylène ou vinylène, $R^3$ représente un carboxyle, alcoxycarbonyle ou aralcoxycarbonyle, $R^4$ et $R^5$ représentent chacun un hydrogène ou $R^4$ et $R^5$ représentent ensemble un groupe oxo, $R^8$ un phtaloylamino et n le nombre 2, où "aryle" représente un phényle éventuellement mono- ou polysubstitué par un halogène, alcoyle, alcoxy, trifluorométhyle ou phényle et "alcoyle" et "alcoxy" présentent de 1 à 8 atomes de carbone.

29. Composé selon l'une des revendications 1—26 ou un de ses sels pharmaceutiquement acceptables comme substance active pharmaceutique.

30. Composé selon l'une des revendications 1—26 ou un de ses sels pharmaceutiquement acceptables comme agent antihypertenseur.

31. Procédé de préparation de composés de formule I données dans la revendication 1 et de leurs sels pharmaceutiquement acceptables, caractérisé en ce que

a) on fait réagir un composé de formule générale

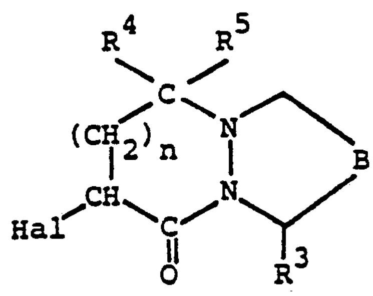

II

où B, $R^3$, $R^4$, $R^5$ et n ont la signification donnée dans la revendication 1 et Hal représente un halogène, avec un composé de formule générale

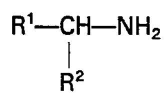

III

où $R^1$ et $R^2$ ont la signification donnée dans la revendication 1, ou

b) on alcoyle de façon réductrice un composé de formule générale

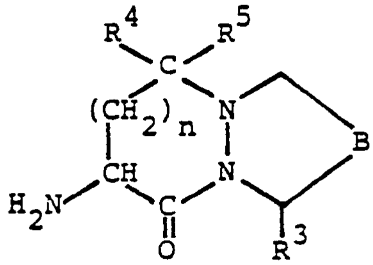

IV

où B, $R^3$, $R^4$, $R^5$ et n ont la signification donnée dans la revendication 1, avec un composé de formule générale

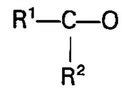

V

où $R^1$ et $R^2$ ont la signification donnée dans la revendication 1, ou

c) pour préparer les composés de formule I, où $R^2$ représente un alcoxycarbonyle ou un aralcoxycarbonyle ou un groupe de formule (ii) et $R^3$ représente un alcoxycarbonyle ou un aralcoxycarbonyle, on fait réagir un composé de formule IV ci-dessus, où $R^3$ représente un alcoxycarbonyle ou un aralcoxycarbonyle, avec un composé de formule générale

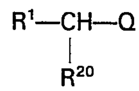

VI

où $R^1$ a la signification donnée dans la revendication 1, $R^{20}$ représente un alcoxycarbonyle ou aralcoxycarbonyle ou un groupe de formule (ii) et Q un groupe sortant, ou

d) pour préparer des composés de formule I où $R^2$ représente un groupe de formule (ii) et $R^3$ un carboxyle ou un tert.butoxycarbonyle, on fait réagir un composé de formule I, où $R^2$ représente un alcoxycarbonyle et $R^3$ un carboxyle ou un tert.butoxycarbonyle, avec un composé de formule générale

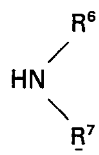

VII

où $R^6$ et $R^7$ ont la signification donnée dans la revendication 1, ou

e) pour préparer des composés de formule I où B représente un éthylène, on hydrogène de façon catalytique un composé de formule I où B représente un vinylène, ou

f) pour préparer les composés de formule I où $R^2$ et/ou $R^3$ représentent un alcoxycarbonyle, ou un aralcoxycarbonyle, on estérifie un composé de formule I où $R^2$ et/ou $R^3$ représentent un carboxyle, ou

g) pour préparer les composés de formule I où $R^2$ et/ou $R^3$ représentent un carboxyle, on traite un composé de formule I où $R^2$ et/ou $R^3$ représentent un alcoxycarbonyle, avec un acide ou une base, ou

h) pour préparer les composés de formule I où B représente un méthylène ou un éthylène et $R^2$ et/ou $R^3$ représentent un carboxyle, on hydrogène un composé de formule I où $R^2$ et/ou $R^3$ représentent un aralcoxycarbonyle, ou

i) pour préparer des composés de formule I où $R^1$ représente un aminoalcoyle, on sépare dans un composé de formule I où $R^1$ représente un alcoxycarbonylaminoalcoyle, aryloxycarbonylaminoalcoyle, ou aralcoxycarbonylaminoalcoyle, le groupe alcoxycarbonyle, aryloxycarbonyle ou aralcoxycarbonyle et

j) si on le désire, on dédouble un mélange de diastéréomères obtenu en les racémates diastéréomères correspondants ou un diastéréomères optiquement purs, et/ou

k) si on le désire, on sépare un racémate obtenu en les deux antipodes et

l) si on le désire, on transforme un composé de formule I obtenu en un sel pharmaceutiquement acceptable.

32. Procédé selon la revendication 31, caractérisé en ce qu'on prépare un composé de formule I où $R^1$ représente un hydrogène, alcoyle, aralcoyle, aminoalcoyle, monoalcoylaminoalcoyle, dialcoylaminoalcoyle, aralcoylcarbonylaminoalcoyle, alcoxycarbonylaminoalcoyle, aryloxycarbonylaminoalcoyle, aralcoxycarbonylaminoalcoyle, alcoylaminocarbonylaminoalcoyle, arylaminocarbonylaminoalcoyle, aralcoylaminocarbonylaminoalcoyle, alcoylsulfonylaminoalcoyle ou arylsulfonylaminoalcoyle $R^2$ représente un carboxyle ou un alcoxycarbonyle ou un groupe de formule (i) et $R^3$ représente un carboxyle ou un alcoxycarbonyle, ou un de ses sels pharmaceutiquement acceptable selon les variantes a), b), e), f), g), i), j), k), et/ou l) du procédé.

33. Médicament contenant un composé selon l'une des revendications 1—26 ou un de ses sels pharmaceutiquement acceptable.

34. Agent antihypertenseur contenant un composé selon l'une des revendications 1—26 ou un de ses sels pharmaceutiquement acceptable.

35. Application d'un composé selon l'une des revendications 1—26 ou d'un de ses sels pharmaceutiquement acceptables à la préparation d'un agent antihypertenseur.

36. Application de l'acide 9-(S)-[1(S)-éthoxycarbonyl-3-phénylpropylamino]-octahydro-10-oxo-6H-pyridazo[1,2-a][1,2]diazépine-1(S)-carboxylique à la préparation d'un agent antihypertenseur.

**Revendications pour l'Etat contractant: AT**

1. Procédé de préparation de composés de formule générale

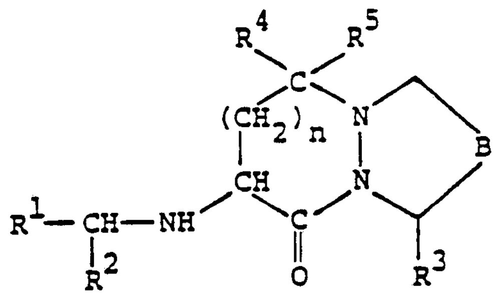

I

où B représente un méthylène (—$CH_2$—), éthylène (—$CH_2$—$CH_2$—) ou vinylène (—CH=CH—), $R^1$ un hydrogène, alcoyle, aralcoyle, aminoalcoyle, monoalcoylaminoalcoyle, dialcoylaminoalcoyle, aralcoylcarbonylaminoalcoyle, phtalimidoalcoyle, alcoxycarbonylaminoalcoyle, aryloxycarbonylaminoalcoyle, aralcoxycarbonylaminoalcoyle, alcoylaminocarbonylaminoalcoyle, arylaminocarbonylaminoalcoyle, aralcoylaminocarbonylaminoalcoyle, alcoylsulfonylaminoalcoyle ou arylsulfonylaminoalcoyle, $R^2$ un carboxyle, alcoxycarbonyle ou aralcoxycarbonyle ou un groupe de formule

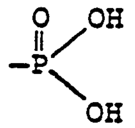

ou

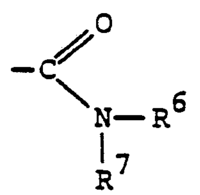

(i) (ii)

$R^3$ représente un carboxyle, alcoxycarbonyle ou aralcoxycarbonyle, $R^4$ et $R^5$ représentent chacun un hydrogène ou ensemble un groupe oxo, $R^6$ et $R^7$ chacun un hydrogène, alcoyle ou aralcoyle et n le nombre 2, où "aryle" représente un phényle éventuellement mono- ou polysubstitué par un halogène, alcoyle, alcoxy, trifluorométhyle ou phényle, et "alcoyle" et "alcoxy" — seuls ou en combinaison — représentent des radicaux en $C_{1-8}$, et leurs sels pharmaceutiquement acceptables, caractérisé en ce que

a) on fait réagir un composé de formule générale

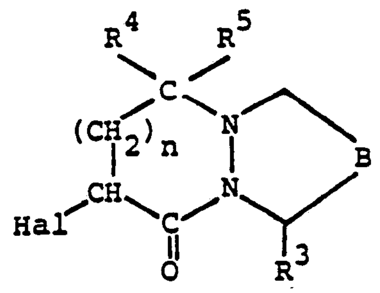

II

où B, $R^3$, $R^4$, $R^5$ et n ont la signification donnée ci-dessus, et Hal représente un halogène, avec un composé de formule générale

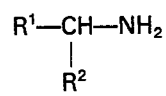

III

où $R^1$ et $R^2$ ont la signification donnée ci-dessus, ou

b) on alcoyle de façon réductrice un composé de formule générale

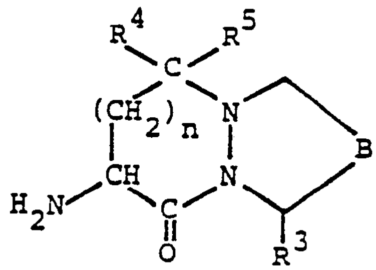

IV

où B, $R^3$, $R^4$, $R^5$ et n ont la signification donnée ci-dessus, avec un composé de formule générale

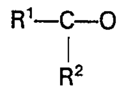

V

où $R^1$ et $R^2$ ont la signification donnée ci-dessus, ou

c) pour préparer les composés de formule I, où $R^2$ représente un alcoxycarbonyle ou un aralcoxycarbonyle ou un groupe de formule (ii) et $R^3$ représente un alcoxycarbonyle ou un aralcoxycarbonyle, on fait réagir un composé de formule IV ci-dessus, où $R^3$ représente un alcoxycarbonyle ou un aralcoxycarbonyle, avec un composé de formule générale

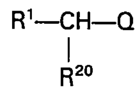

VI

où $R^1$ a la signification donnée ci-dessus, $R^{20}$ représente un alcoxycarbonyle ou aralcoxycarbonyle ou un groupe de formule (ii) et Q un groupe sortant, ou

d) pour préparer des composés de formule I où $R^2$ représente un groupe de formule (ii) et $R^3$ un carboxyle ou un tert.butoxycarbonyle, on fait réagir un composé de formule I, où $R^2$ représente un alcoxycarbonyle et $R^3$ un carboxyle ou un tert.butoxycarbonyle, avec un composé de formule générale

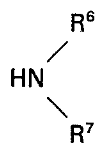

où $R^6$ et $R^7$ ont la signification donnée ci-dessus, ou

e) pour préparer des composés de formule I où B représente un éthylène, on hydrogène de façon catalytique un composé de formule I où B représente un vinylène, ou

f) pour préparer les composés de formule I où $R^2$ et/ou $R^3$ représentent un alcoxycarbonyle, ou un aralcoxycarbonyle, on estérifie un composé de formule I où $R^2$ et/ou $R^3$ représentent un carboxyle, ou

g) pour préparer les composés de formule I où $R^2$ et/ou $R^3$ représentent un carboxyle, on traite un composé de formule I où $R^2$ et/ou $R^3$ représentent un alcoxycarbonyle, avec un acide ou une base, ou

h) pour préparer les composés de formule I où B représente un méthylène ou un éthylène et $R^2$ et/ou $R^3$ représentent un carboxyle, on hydrogène un composé de formule I où $R^2$ et/ou $R^3$ représentent un aralcoxycarbonyle, ou

i) pour préparer des composés de formule I où $R^1$ représente un aminoalcoyle, on sépare dans un composé de formule I où $R^1$ représente un alcoxycarbonylaminoalcoyle, aryloxycarbonylaminoalcoyle, ou aralcoxycarbonylaminoalcoyle, le groupe alcoxycarbonyle, aryloxycarbonyle ou aralcoxycarbonyle et

j) si on le désire, on dédouble un mélange de diastéréomères obtenu en les racémates diastéréomères correspondants ou un diastéréomères optiquement purs, et/ou

k) si on le désire, on sépare un racémate obtenu en les deux antipodes et

l) si on le désire, on transforme un composé de formule I obtenu en un sel pharmaceutiquement acceptable.

2. Procédé selon la revendication 1, caractérisé en ce qu'on prépare un composé de formule I où $R^1$ représente un hydrogène, alcoyle, aralcoyle, aminoalcoyle, monoalcoylaminoalcoyle, dialcoylaminoalcoyle, aralcoylcarbonylaminoalcoyle, alcoxycarbonylaminoalcoyle, aryloxycarbonylaminoalcoyle, aralcoxycarbonylaminoalcoyle, alcoylaminocarbonylaminoalcoyle, arylaminocarbonylaminoalcoyle, aralcoylaminocarbonylaminoalcoyle, alcoylsulfonylaminoalcoyle, ou arylsulfonylaminoalcoyle, $R^2$ représente un carboxyle ou un alcoxycarbonyle ou un groupe de formule (i) et $R^3$ représente un carboxyle ou un alcoxycarbonyle, ou un de ses sels pharmaceutiquement acceptables selon les variantes a), b), e), f), g), i), j), k), et/ou l) du procédé.

3. Procédé selon la revendication 1, où B représente un méthylène ou un éthylène.

4. Procédé selon la revendication 1 ou 3, où $R^1$ représente un alcoyle, aralcoyle, aralcoylcarbonylaminoalcoyle, phtalimidoalcoyle, aralcoxycarbonylaminoalcoyle ou arylaminocarbonylaminoalcoyle.

5. Procédé selon la revendication 1, 3 ou 4 où $R^2$ représente un carboxyle, alcoxycarbonyle, aralcoxycarbonyle ou un groupe de formule (ii).

6. Procédé selon la revendication 1, ou l'une des revendications 3—5, où $R^3$ représente un carboxyle.

7. Procédé selon la revendication 1 ou l'une des revendications 3—6, où B représente un méthylène ou un éthylène, $R^1$ représente un alcoyle, aralcoyle, aralcoylcarbonylaminoalcoyle, phtalimidoalcoyle, aralcoxycarbonylaminoalcoyle ou arylaminocarbonylaminoalcoyle, $R^2$ représente un carboxyle, alcoxycarbonyle ou aralcoxycarbonyle ou un groupe de formule (ii), $R^3$ représente un carboxyle, et n représente le nombre 2.

8. Procédé selon la revendication 2, caractérisé en ce qu'on prépare l'acide 9-(1-carboxy-3-phénylpropylamino)-octahydro-6,10-dioxo-6H-pyridazo[1,2-a][1,2]diazépine-1-carboxylique.

9. Procédé selon la revendication 2, caractérisé en ce qu'on prépare l'acide 9-(1-éthoxycarbonyl-3-phénylpropylamino)-octahydro-6,10-dioxo-6H-pyridazo[1,2-a][1,2]diazépine-1-carboxylique.

10. Procédé selon la revendication 2, caractérisé en ce qu'on prépare l'acide 8-(1-carboxy-3-phénylpropylamino)-2,3,6,7,8,9-hexahydro-5,9-dioxo-1H,5H-pyrazolo[1,2-a][1,2]diazépine-1-carboxylique.

11. Procédé selon la revendication 1, caractérisé en ce qu'on prépare l'acide 9-(1-benzyloxycarbonyl-3-phénylpropylamino)-octahydro-6,10-dioxo-6H-pyridazo[1,2-a][1,2]diazépine-1-carboxylique.

12. Procédé selon la revendication 1, caractérisé en ce qu'on prépare l'acide 9-(1-carbamoyl-3-phénylpropylamino)-octahydro-6,10-dioxo-6H-pyridazo[1,2-a][1,2]diazépine-1-carboxylique.

13. Procédé selon la revendication 1, caractérisé en ce qu'on prépare l'acide 9-(1-éthylcarbamoyl-3-phénylpropylamino)-octahydro-6,10-dioxo-6H-pyridazo[1,2-a][1,2]diazépine-1-carboxylique.

14. Procédé selon la revendication 1, caractérisé en ce qu'on prépare l'acide 9-(1-carboxy-4-phénylbutylamino)-octahydro-6,10-dioxo-6H-pyridazo[1,2-a][1,2]diazépine-1-carboxylique.

15. Procédé selon la revendication 1, caractérisé en ce qu'on prépare l'acide 9-(1-carboxy-2-phényléthylamino)-octahydro-6,10-dioxo-6H-pyridazo[1,2-a][1,2]diazépine-1-carboxylique.

16. Procédé selon la revendication 1, caractérisé en ce qu'on prépare l'acide 9-(1-carboxy-4-méthylpentylamino)-octahydro-6,10-dioxo-6H-pyridazo[1,2-a][1,2]diazépine-1-carboxylique.

17. Procédé selon la revendication 1, caractérisé en ce qu'on prépare l'acide 9-(1-éthoxycarbonyl-4-méthylpentylamino)-octahydro-6,10-dioxo-6H-pyridazo[1,2-a][1,2]diazépine-1-carboxylique.

18. Procédé selon la revendication 1, caractérisé en ce qu'on prépare l'acide 9-[3-(4-chlorophényl)-1-éthoxycarbonylpropylamino]-octahydro-6,10-dioxo-6H-pyridazo[1,2-a][1,2]diazépine-1-carboxylique.

19. Procédé selon la revendication 1, caractérisé en ce qu'on prépare l'acide 9-[1-éthoxycarbonyl-3-(4-méthoxyphényl)propylamino]-octahydro-6,10-dioxo-6H-pyridazo[1,2-a][1,2]diazépine-1-carboxylique.

20. Procédé selon la revendication 1, caractérisé en ce qu'on prépare l'acide 9-[3-(4-biphénylyl)-1-éthoxycarbonylpropylamino]-octahydro-6,10-dioxo-6H-pyridazo[1,2-a][1,2]diazépine-1-carboxylique.

21. Procédé selon la revendication 1, caractérisé en ce qu'on prépare l'acide 9-(1-éthoxycarbonyl-5-phtalimidopentylamino)-octahydro-6,10-dioxo-6H-pyridazo[1,2-a][1,2]diazépine-1-carboxylique.

22. Procédé selon la revendication 1, caractérisé en ce qu'on prépare l'acide 8-(1-éthoxycarbonyl-3-phénylpropylamino)-2,3,6,7,8,9-hexahydro-5,9-dioxo-1H,5H-pyrazolo[1,2-a][1,2]diazépine-1-carboxylique.

23. Procédé selon la revendication 1, caractérisé en ce qu'on prépare l'acide 9-(1-éthoxycarbonyl-3-phénylpropylamino)-octahydro-10-oxo-6H-pyridazo[1,2-a][1,2]diazépine-1-carboxylique.

24. Procédé selon la revendication 1, caractérisé en ce qu'on prépare l'acide 9-(5-benzyloxyformamido-1-éthoxycarbonylpentylamino)-octahydro-6,10-dioxo-6H-pyridazo[1,2-a][1,2]diazépine-1-carboxylique.

25. Procédé selon l'une des revendications 1—24, caractérisé en ce que la configuration sur chaque atome de carbone asymétrique est la configuration S.

26. Application d'un composé obtenu selon l'une des revendications 1—25 à la préparation d'un agent antihypertenseur.

27. Application de l'acide 9-(S)-[1(S)-éthoxycarbonyl-3-phénylpropylamino]-octahydro-10-oxo-6H-pyridazo[1,2-a][1,2]diazépine-1(S)-carboxylique à la préparation d'un agent antihypertenseur.

**Claims for the Contracting States: BE CH DE FR GB IT LI LU NL SE**

1. Compounds of the general formula

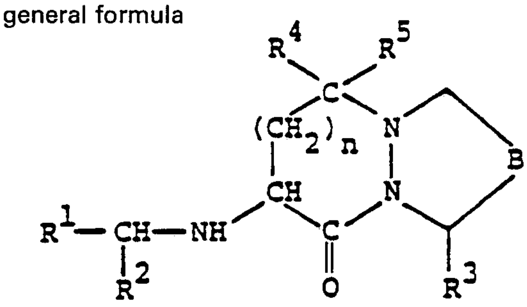

I

wherein B signifies methylene ($-CH_2-$), ethylene ($-CH_2-CH_2-$) or vinylene ($-CH=CH-$), $R^1$ signifies hydrogen, alkyl, aralkyl, aminoalkyl, monoalkylaminoalkyl, dialkylaminoalkyl, aralkylcarbonylaminoalkyl, phthalimidoalkyl, alkoxycarbonylaminoalkyl, aryloxycarbonylaminoalkyl, aralkoxycarbonylaminoalkyl, alkylaminocarbonylaminoalkyl, arylaminocarbonylaminoalkyl, aralkylaminocarbonylaminoalkyl, alkylsulphonylaminoalkyl or arylsulphonylaminoalkyl, $R^2$ signifies carboxyl, alkoxycarbonyl or aralkoxycarbonyl or a group of the formula

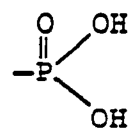

(i)

or

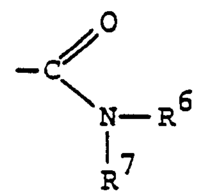

(ii)

$R^3$ signifies carboxyl, alkoxycarbonyl or aralkoxycarbonyl, $R^4$ and $R^5$ each signify hydrogen or together signify an oxo group, $R^6$ and $R^7$ each signify hydrogen, alkyl or aralkyl and n signifies the number 2, whereby "aryl" signifies phenyl which is optionally mono- or multiply-substituted by halogen, alkyl, alkoxy, trifluoromethyl or phenyl and "alkyl" and "alkoxy" — alone or in combination — signify residues with 1—8 carbon atoms, and pharmaceutically usable salts thereof.

2. Compounds according to claim 1, wherein $R^1$ signifies hydrogen, alkyl, aralkyl, aminoalkyl, monoalkylaminoalkyl, dialkylaminoalkyl, aralkylcarbonylaminoalkyl, alkoxycarbonylaminoalkyl, aryloxycarbonylaminoalkyl, aralkoxycarbonylaminoalkyl, alkylaminocarbonylaminoalkyl, arylaminocarbonylaminoalkyl, aralkylaminocarbonylaminoalkyl, alkylsulphonylaminoalkyl or arylsulphonylaminoalkyl, $R^2$ signifies carboxyl or alkoxycarbonyl or a group of formula (i) and $R^3$ signifies carboxyl or alkoxycarbonyl.

3. Compounds according to claim 1, wherein B signifies methylene or ethylene.

4. Compounds according to claim 1 or 3, wherein $R^1$ signifies alkyl, aralkyl, aralkylcarbonylaminoalkyl, phthalimidoalkyl, aralkoxycarbonylaminoalkyl or arylaminocarbonylaminoalkyl.

5. Compounds according to claim 1, 3 or 4, wherein $R^2$ signifies carboxyl, alkoxycarbonyl, aralkoxycarbonyl or the group of formula (ii).

6. Compounds according to claim 1 or any one of claims 3—5, wherein $R^3$ signifies carboxyl.

7. Compounds according to claim 1 or any one of claims 3—6, wherein B signifies methylene or ethylene, $R^1$ signifies alkyl, aralkyl, aralkylcarbonylaminoalkyl, phthalimidoalkyl, aralkoxycarbonylaminoalkyl or arylaminocarbonylaminoalkyl, $R^2$ signifies carboxyl, alkoxycarbonyl or aralkoxycarbonyl or a group of formula (ii), $R^3$ signifies carboxyl and n signifies the number 2.

8. 9-(1-Carboxy-3-phenylpropylamino)-octahydro-6,10-dioxo-6H-pyridazo[1,2-a][1,2]diazepine-1-carboxylic acid.

9. 9-(1-Ethoxycarbonyl-3-phenylpropylamino)-octahydro-6,10-dioxo-6H-pyridazo[1,2-a][1,2]diazepine-1-carboxylic acid.

10. 8-(1-Carboxy-3-phenylpropylamino)-2,3,6,7,8,9-hexahydro-5,9-dioxo-1H,5H-pyrazolo[1,2-a][1,2]diazepine-1-carboxylic acid.

11. 9-(1-Benzyloxycarbonyl-3-phenylpropylamino)-octahydro-6,10-dioxo-6H-pyridazo[1,2-a][1,2]diazepine-1-carboxylic acid.

12. 9-(1-Carbamoyl-3-phenylpropylamino)-octahydro-6,10-dioxo-6H-pyridazo[1,2-a][1,2]diazepine-1-carboxylic acid.

13. 9-(1-Ethylcarbamoyl-3-phenylpropylamino)-octahydro-6,10-dioxo-6H-pyridazo[1,2-a][1,2]diazepine-1-carboxylic acid.

14. 9-(1-Carboxy-4-phenylbutylamino)-octahydro-6,10-dioxo-6H-pyridazo[1,2-a][1,2]diazepine-1-carboxylic acid.

15. 9-(1-Carboxy-2-phenylethylamino)-octahydro-6,10-dioxo-6H-pyridazo[1,2-a][1,2]diazepine-1-carboxylic acid.

16. 9-(1-Carboxy-4-methylpentylamino)-octahydro-6,10-dioxo-6H-pyridazo[1,2-a][1,2]diazepine-1-carboxylic acid.

17. 9-(1-Ethoxycarbonyl-4-methylpentylamino)-octahydro-6,10-dioxo-6H-pyridazo[1,2-a][1,2]diazepine-1-carboxylic acid.

18. 9-[3-(4-Chlorophenyl)-1-ethoxycarbonylpropylamino]-octahydro-6,10-dioxo-6H-pyridazo[1,2-a][1,2]diazepine-1-carboxylic acid.

19. 9-[1-Ethoxycarbonyl-3-(4-methoxyphenyl)propylamino]-octahydro-6,10-dioxo-6H-pyridazo[1,2-a][1,2]diazepine-1-carboxylic acid.

20. 9-[3-(4-Biphenylyl)-1-ethoxycarbonylpropylamino]-octahydro-6,10-dioxo-6H-pyridazo[1,2-a][1,2]diazepine-1-carboxylic acid.

21. 9-(1-Ethoxycarbonyl-5-phthalimidopentylamino)-octahydro-6,10-dioxo-6H-pyridazo[1,2-a][1,2]diazepine-1-carboxylic acid.

22. 8-(1-Ethoxycarbonyl-3-phenylpropylamino)-2,3,6,7,8,9-hexahydro-5,9-dioxo-1H,5H-pyrazolo[1,2-a][1,2]diazepine-1-carboxylic acid.

23. 9-(1-Ethoxycarbonyl-3-phenylpropylamino)-octahydro-10-dioxo-6H-pyridazo[1,2-a][1,2]diazepine-1-carboxylic acid.

24. 9-(5-Benzyloxyformamido-1-ethoxycarbonylpentylamino)-octahydro-6,10-dioxo-6H-pyridazo[1,2-a][1,2]diazepine-1-carboxylic acid.

25. tert.Butyl 9-(1-ethoxycarbonyl-3-phenylpropylamino)-octahydro-6,10-dioxo-6H-pyridazo[1,2-a][1,2]diazepine-1-carboxylate,

tert.butyl 9-(1-benzyloxycarbonyl-3-phenylpropylamino)-octahydro-6,10-dioxo-6H-pyridazo[1,2-a][1,2]diazepine-1-carboxylate,

methyl 9-(1-benzyloxycarbonyl-3-phenylpropylamino)-octahydro-6,10-dioxo-6H-pyridazo[1,2-a][1,2]diazepine-1-carboxylate,

methyl 9-(1-carboxy-3-phenylpropylamino)-octahydro-6,10-dioxo-6H-pyridazo[1,2-a][1,2]diazepine-1-carboxylate,

methyl 9-(1-ethoxycarbonyl-3-phenylpropylamino)-octahydro-6,10-dioxo-6H-pyridazo[1,2-a][1,2]diazepine-1-carboxylate,

ethyl 9-(1-ethoxycarbonyl-3-phenylpropylamino)-octahydro-6,10-dioxo-6H-pyridazo[1,2-a][1,2]diazepine-1-carboxylate,

tert.butyl 9-(1-carbamoyl-3-phenylpropylamino)-octahydro-6,10-dioxo-6H-pyridazo[1,2-a][1,2]diazepine-1-carboxylate,

tert.butyl 9-(1-ethylcarbamoyl-3-phenylpropylamino)-octahydro-6,10-dioxo-6H-pyridazo[1,2-a][1,2]diazepine-1-carboxylate,

tert.butyl 9-(1-ethoxycarbonyl-4-methylpentylamino)-octahydro-6,10-dioxo-6H-pyridazo[1,2-a][1,2]diazepine-1-carboxylate,

tert.butyl 9-[3-(4-chlorophenyl)-1-ethoxycarbonylpropylamino]-octahydro-6,10-dioxo-6H-pyridazo[1,2-a][1,2]diazepine-1-carboxylate,

tert.butyl 9-[1-ethoxycarbonyl-3-(4-methoxyphenyl)propylamino]-octahydro-10-oxo-6H-pyridazo[1,2-a][1,2]diazepine-1-carboxylate,

tert.butyl 9-(3-(4-biphenylyl)-1-ethoxycarbonylpropylamino]-octahydro-6,10-dioxo-6H-pyridazo[1,2-a][1,2]diazepine-1-carboxylate,

tert.butyl 9-(1-ethoxycarbonyl-5-phthalimidopentylamino)-octahydro-6,10-dioxo-6H-pyridazo[1,2-a][1,2]diazepine-1-carboxylate,

tert.butyl 8-(1-ethoxycarbonyl-3-phenylpropylamino)-2,3,6,7,8,9-hexahydro-5,9-dioxo-1H,5H-pyrazolo[1,2-a][1,2]diazepine-1-carboxylate,

benzyl 9-(1-ethoxycarbonyl-3-phenylpropylamino)-octahydro-10-oxo-6H-pyridazo[1,2-a][1,2]-diazepine-1-carboxylate and

tert.butyl 9-(5-benzyloxyformamido-1-ethoxycarbonylpentylamino)-octahydro-6,10-dioxo-6H-pyridazo[1,2-a][1,2]diazepine-1-carboxylate.

26. A compound according to any one of claims 1—25, wherein the configuration at each asymmetric carbon atom is the S-configuration.

27. A compound of the general formula

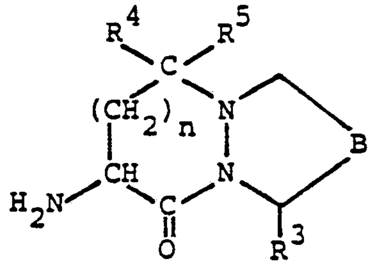

IV

wherein B signifies methylene, ethylene or vinylene, $R^3$ signifies carboxyl, alkoxycarbonyl or aralkoxycarbonyl, $R^4$ and $R^5$ each signify hydrogen or $R^4$ and $R^5$ together signify an oxo group and n signifies the number 2, whereby "aryl" signifies phenyl which is optionally mono- or multiply-substituted by halogen, alkyl, alkoxy, trifluoromethyl or phenyl and "alkyl" and "alkoxy" have 1—8 carbon atoms.

28. A compound of the general formula

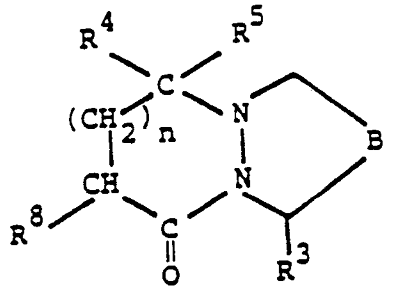

XVIIa

wherein B signifies methylene, ethylene or vinylene, $R^3$ signifies carboxyl, alkoxycarbonyl or aralkoxycarbonyl, $R^4$ and $R^5$ each signify hydrogen or $R^4$ and $R^5$ together signify an oxo group, $R^8$ signifies phthaloylamino and n signifies the number 2, whereby "aryl" signifies phenyl which is optionally mono- or multiply-substituted by halogen, alkyl, alkoxy, trifluoromethyl or phenyl and "alkyl" and "alkoxy" have 1—8 carbon atoms.

29. A compound according to any one of claims 1—26 or a pharmaceutically usable salt thereof as a pharmaceutically active substance.

30. A compound according to any one of claims 1—26 or a pharmaceutically usable salt thereof as an anti-hypertensive.

31. A process for the manufacture of compounds of formula I given in claim 1 and of pharmaceutically usable salts thereof, characterized by

a) reacting a compound of the general formula

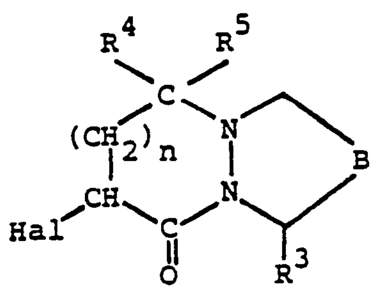

II

wherein B, $R^3$, $R^4$, $R^5$ and n have the significance given in claim 1 and Hal signifies halogen, with a compound of the general formula

$$R^1—CH—NH_2$$
$$|$$
$$R^2$$

III

wherein $R^1$ and $R^2$ have the significance given in claim 1, or

b) reductively alkylating a compound of the general formula

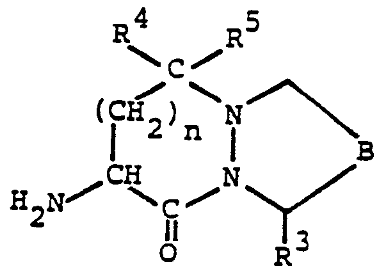

IV

wherein B, $R^3$, $R^4$, $R^5$ and n have the significance given in claim 1, with a compound of the general formula

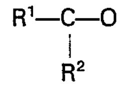

V

wherein $R^1$ and $R^2$ have the significance given in claim 1, or

c) for the manufacture of compounds of formula I in which $R^2$ signifies alkoxycarbonyl or aralkoxycarbonyl or a group of formula (ii) and $R^3$ signifies alkoxycarbonyl or aralkoxycarbonyl, reacting a compound of formula IV above in which $R^3$ signifies alkoxycarbonyl or aralkoxycarbonyl with a compound of the general formula

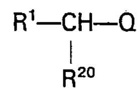

VI

wherein $R^1$ has the significance given in claim 1, $R^{20}$ signifies alkoxycarbonyl or aralkoxycarbonyl or a group of formula (ii) and Q signifies a leaving group, or

d) for the manufacture of compounds of formula I in which $R^2$ signifies a group of formula (ii) and $R^3$ signifies carboxyl or tert.butoxycarbonyl, reacting a compound of formula I in which $R^2$ signifies alkoxycarbonyl and $R^3$ signifies carboxyl or tert.butoxycarbonyl with a compound of the general formula

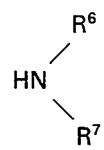

VII

wherein $R^6$ and $R^7$ have the significance given in claim 1, or

e) for the manufacture of compounds of formula I in which B signifies ethylene, catalytically hydrogenating a compound of formula I in which B signifies vinylene, or

f) for the manufacture of compounds of formula I in which $R^2$ and/or $R^3$ signifies alkoxycarbonyl or aralkoxycarbonyl, esterifying a compound of formula I in which $R^2$ and/or $R^3$ signifies carboxyl, or

g) for the manufacture of compounds of formula I in which $R^2$ and/or $R^3$ signifies carboxyl, treating a compound of formula I in which $R^2$ and/or $R^3$ signifies alkoxycarbonyl with an acid or a base, or

h) for the manufacture of compounds of formula I in which B signifies methylene or ethylene and $R^2$ and/or $R^3$ signifies carboxyl, hydrogenating a compound of formula I in which $R^2$ and/or $R^3$ signifies aralkoxycarbonyl, or

i) for the manufacture of compounds of formula I in which $R^1$ signifies aminoalkyl, cleaving off the alkoxycarbonyl, aryloxycarbonyl or aralkoxycarbonyl group in a compound of formula I in which $R^1$ signifies alkoxycarbonylaminoalkyl, aryloxycarbonylaminoalkyl or aralkoxycarbonylaminoalkyl, and

j) if desired, separating a mixture of diastereomers obtained into the corresponding diastereomeric racemates or optically pure diastereomers and/or

k) if desired, resolving a racemate obtained into the two antipodes and

l) if desired, converting a compound of formula I obtained into a pharmaceutically usable salt.

32. A process according to claim 31, characterized in that a compound of formula I in which $R^1$ signifies hydrogen, alkyl, aralkyl, aminoalkyl, monoalkylaminoalkyl, dialkylaminoalkyl, aralkylcarbonylaminoalkyl, alkoxycarbonylaminoalkyl, aryloxycarbonylaminoalkyl, aralkoxycarbonylaminoalkyl, alkylaminocarbonylaminoalkyl, arylaminocarbonylaminoalkyl, aralkylaminocarbonylaminoalkyl, alkylsulphonylaminoalkyl or arylsulphonylaminoalkyl, $R^2$ signifies carboxyl or alkoxycarbonyl or a group of formula (i) and $R^3$ signifies carboxyl or alkoxycarbonyl or a pharmaceutically usable salt thereof is manufactured according to process variants a), b), e), f), g), i), j), k) and/or l).

33. A medicament containing a compound according to any one of claims 1—26 or a pharmaceutically usable salt thereof.

34. An antihypertensive containing a compound according to any one of claims 1—26 or a pharmaceutically usable salt thereof.

35. The use of a compound according to any one of claims 1—26 or of a pharmaceutically usable salt thereof for the manufacture of an antihypertensive.

36. The use of 9(S)-[1(S)-ethoxycarbonyl-3-phenylpropylamino]-octahydro-10-oxo-6H-pyridazo[1,2-a][1,2]-diazepine-1(S)-carboxylic acid for the manufacture of an antihypertensive.

**Claims for the Contracting State: AT**

1. A process for the manufacture of compounds of the general formula

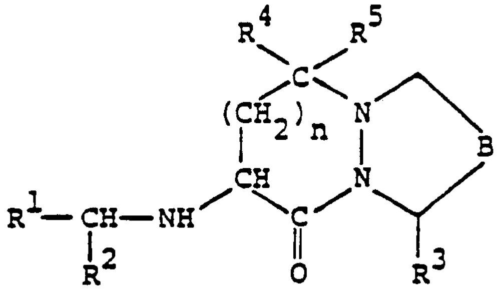

I

wherein B signifies methylene (—$CH_2$—), ethylene (—$CH_2$—$CH_2$—) or vinylene (—CH=CH—), $R^1$ signifies hydrogen, alkyl, aralkyl, aminoalkyl, monoalkylaminoalkyl, dialkylaminoalkyl, aralkylcarbonylaminoalkyl, phthalimidoalkyl, alkoxycarbonylaminoalkyl, aryloxycarbonylaminoalkyl, aralkoxycarbonylaminoalkyl, alkylaminocarbonylaminoalkyl, arylaminocarbonylaminoalkyl, aralkylaminocarbonylaminoalkyl, alkylsulphonylaminoalkyl or arylsulphonylaminoalkyl, $R^2$ signifies carboxyl, alkoxycarbonyl or aralkoxycarbonyl or a group of the formula

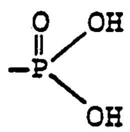

or

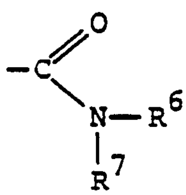

(i) (ii)

$R^3$ signifies carboxyl, alkoxycarbonyl or aralkoxycarbonyl, $R^4$ and $R^5$ each signify hydrogen or together signify an oxo group, $R^6$ and $R^7$ each signify hydrogen, alkyl or aralkyl and n signifies the number 2, whereby "aryl" signifies phenyl which is optionally mono- or multiply-substituted by halogen, alkyl, alkoxy, trifluoromethyl or phenyl and "alkyl" and "alkoxy" — alone or in combination — signify residues with 1—8 carbon atoms, and pharmaceutically usable salts thereof, characterized by

a) reacting a compound of the general formula

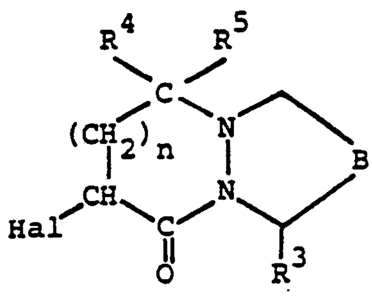

II

wherein B, $R^3$, $R^4$, $R^5$ and n have the above significance and Hal signifies halogen, with a compound of the general formula

$R^1$—CH—$NH_2$
|
$R^2$

III

wherein $R^1$ and $R^2$ have the above significance , or

b) reductively alkylating a compound of the general formula

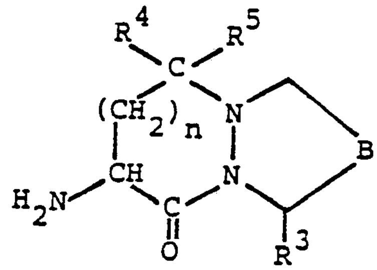

IV

wherein B, $R^3$, $R^4$, $R^5$ and n have the above significance, with a compound of the general formula

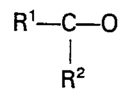

V

wherein $R^1$ and $R^2$ have the above significance, or

c) for the manufacture of compounds of formula I in which $R^2$ signifies alkoxycarbonyl or aralkoxycarbonyl or a group of formula (ii) and $R^3$ signifies alkoxycarbonyl or aralkoxycarbonyl, reacting a compound of formula IV above in which $R^3$ signifies alkoxycarbonyl or aralkoxycarbonyl with a compound of the general formula

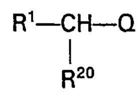

VI

wherein $R^1$ has the above significance, $R^{20}$ signifies alkoxycarbonyl or aralkoxycarbonyl or a group of formula (ii) and Q signifies a leaving group, or

d) for the manufacture of compounds of formula I in which $R^2$ signifies a group of formula (ii) and $R^3$ signifies carboxyl or tert.butoxycarbonyl, reacting a compound of formula I in which $R^2$ signifies alkoxycarbonyl and $R^3$ signifies carboxyl or tert.butoxycarbonyl with a compound of the general formula

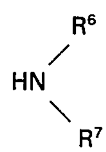

VII

wherein $R^6$ and $R^7$ have the above significance, or

e) for the manufacture of compounds of formula I in which B signifies ethylene, catalytically hydrogenating a compound of formula I in which B signifies vinylene, or

f) for the manufacture of compounds of formula I in which $R^2$ and/or $R^3$ signifies alkoxycarbonyl or aralkoxycarbonyl, esterifying a compound of formula I in which $R^2$ and/or $R^3$ signifies carboxyl, or

g) for the manufacture of compounds of formula I in which $R^2$ and/or $R^3$ signifies carboxyl, treating a compound of formula I in which $R^2$ and/or $R^3$ signifies alkoxycarbonyl with an acid or a base, or

h) for the manufacture of compounds of formula I in which B signifies methylene or ethylene and $R^2$ and/or $R^3$ signifies carboxyl, hydrogenating a compound of formula I in which $R^2$ and/or $R^3$ signifies aralkoxycarbonyl, or

i) for the manufacture of compounds of formula I in which $R^1$ signifies aminoalkyl, cleaving off the alkoxycarbonyl, aryloxycarbonyl or aralkoxycarbonyl group in a compound of formula I in which $R^1$ signifies alkoxycarbonylaminoalkyl, aryloxycarbonylaminoalkyl or aralkoxycarbonylaminoalkyl, and

j) if desired, separating a mixture of diastereomers obtained into the corresponding diastereomeric racemates or optically pure diastereomers and/or

k) if desired, resolving a racemate obtained into the two antipodes and

l) if desired, converting a compound of formula I obtained into a pharmaceutically usable salt.

2. A process according to claim 1, characterized in that a compound of formula I in which $R^1$ signifies hydrogen, alkyl, aralkyl, aminoalkyl, monoalkylaminoalkyl, dialkylaminoalkyl, aralkylcarbonylaminoalkyl, alkoxycarbonylaminoalkyl, aryloxycarbonylaminoalkyl, aralkoxycarbonylaminoalkyl, alkylaminocarbonylaminoalkyl, arylaminocarbonylaminoalkyl, aralkylaminocarbonylaminoalkyl, alkylsulphonylaminoalkyl or arylsulphonylaminoalkyl, $R^2$ signifies carboxyl or alkoxycarbonyl or a group of formula (i) and $R^3$ signifies carboxyl or alkoxycarbonyl or a pharmaceutically usable salt thereof is manufactured according to process variants a), b), e), f), g), i), j), k) and/or l).

3. A process according to claim 1, wherein B signifies methylene or ethylene.

4. A process according to claim 1 or 3, wherein $R^1$ signifies alkyl, aralkyl, aralkylcarbonylaminoalkyl, phthalimidoalkyl, aralkoxycarbonylaminoalkyl or arylaminocarbonylaminoalkyl.

5. A process according to claim 1, 3 or 4, wherein $R^2$ signifies carboxyl, alkoxycarbonyl or aralkoxycarbonyl or the group of formula (ii).

6. A process according to claim 1 or any one of claims 3—5, wherein $R^3$ signifies carboxyl.

7. A process according to claim 1 or any one of claims 3—6, wherein B signifies methylene or ethylene, $R^1$ signifies alkyl, aralkyl, aralkylcarbonylaminoalkyl, phthalimidoalkyl, aralkoxycarbonylaminoalkyl or arylaminocarbonylaminoalkyl, $R^2$ signifies carboxyl, alkoxycarbonyl or aralkoxycarbonyl or a group of formula (ii), $R^3$ signifies carboxyl and n signifies the number 2.

8. A process according to claim 2, characterized in that 9-(1-carboxy-3-phenylpropylamino)-octahydro-6,10-dioxo-6H-pyridazo[1,2-a][1,2]diazepine-1-carboxylic acid is manufactured.

9. A process according to claim 2, characterized in that 9-(1-ethoxycarbonyl-3-phenylpropylamino)-octahydro-6,10-dioxo-6H-pyridazo[1,2-a][1,2]diazepine-1-carboxylic acid is manufactured.

10. A process according to claim 2, characterized in that 8-(1-carboxy-3-phenylpropylamino)-2,3,6,7,8,9-hexahydro-5,9-dioxo-1H,5H-pyrazolo[1,2-a][1,2]diazepine-1-carboxylic acid is manufactured.

11. A process according to claim 1, characterized in that 9-(1-benzyloxycarbonyl-3-phenylpropylamino)-octahydro-6,10-dioxo-6H-pyridazo[1,2-a][1,2]diazepine-1-carboxylic acid is manufactured.

12. A process according to claim 1, characterized in that 9-(1-carbamoyl-3-phenylpropylamino)-octahydro-6,10-dioxo-6H-pyridazo[1,2-a][1,2]diazepine-1-carboxylic acid is manufactured.

13. A process according to claim 1, characterized in that 9-(1-ethylcarbamoyl-3-phenylpropylamino)-octahydro-6,10-dioxo-6H-pyridazo[1,2-a][1,2]diazepine-1-carboxylic acid is manufactured.

14. A process according to claim 1, characterized in that 9-(1-carboxy-4-phenylbutylamino)-octahydro-6,10-dioxo-6H-pyridazo[1,2-a][1,2]diazepine-1-carboxylic acid is manufactured.

15. A process according to claim 1, characterized in that 9-(1-carboxy-2-phenylethylamino)-octahydro-6,10-dioxo-6H-pyridazo[1,2-a][1,2]diazepine-1-carboxylic acid is manufactured.

16. A process according to claim 1, characterized in that 9-(1-carboxy-4-methylpentylamino)-octahydro-6,10-dioxo-6H-pyridazo[1,2-a][1,2]diazepine-1-carboxylic acid is manufactured.

17. A process according to claim 1, characterized in that 9-(1-ethoxycarbonyl-4-methylpentylamino)-octahydro-6,10-dioxo-6H-pyridazo[1,2-a][1,2]diazepine-1-carboxylic acid is manufactured.

18. A process according to claim 1, characterized in that 9-[3-(4-chlorophenyl)-1-ethoxycarbonylpropylamino]-octahydro-6,10-dioxo-6H-pyridazo[1,2-a][1,2]diazepine-1-carboxylic acid is manufactured.

19. A process according to claim 1, characterized in that 9-[1-ethoxycarbonyl-3-(4-methoxyphenyl)propylamino]-octahydro-6,10-dioxo-6H-pyridazo[1,2-a][1,2]diazepine-1-carboxylic acid is manufactured.

20. A process according to claim 1, characterized in that 9-[3-(4-biphenylyl)-1-ethoxycarbonylpropylamino]-octahydro-6,10-dioxo-6H-pyridazo[1,2-a][1,2]diazepine-1-carboxylic acid is manufactured.

21. A process according to claim 1, characterized in that 9-(1-ethoxycarbonyl-5-phthalimidopentylamino)-octahydro-6,10-dioxo-6H-pyridazo[1,2-a][1,2]diazepine-1-carboxylic acid is manufactured.

22. A process according to claim 1, characterized in that 8-(1-ethoxycarbonyl-3-phenylpropylamino)-2,3,6,7,8,9-hexahydro-5,9-dioxo-1H,5H-pyrazolo[1,2-a][1,2]diazepine-1-carboxylic acid is manufactured.

23. A process according to claim 1, characterized in that 9-(1-ethoxycarbonyl-3-phenylpropylamino)-octahydro-10-oxo-6H-pyridazo[1,2-a][1,2]diazepine-1-carboxylic acid is manufactured.

24. A process according to claim 1, characterized in that 9-(5-benzyloxyformamido-1-ethoxycarbonylpentylamino)-octahydro-6,10-dioxo-6H-pyridazo[1,2-a][1,2]diazepine-1-carboxylic acid is manufactured.

25. A process according to any one of claims 1—24, characterized in that the configuration at each asymmetric carbon atom is the S-configuration.

26. The use of a compound obtained according to any one of claims 1—25 for the manufacture of an antihypertensive.

27. The use of 9(S)-[1(S)-ethoxycarbonyl-3-phenylpropylamino]-octahydro-10-oxo-6H-pyridazo[1,2-a][1,2]-diazepine-1(S)-carboxylic acid for the manufacture of an antihypertensive.